# EUROPEAN PATENT APPLICATION

(11) **EP 4 831 160 A1**
(43) Date of publication of application: **28.10.2026**
(21) Application number: 25751526.2
(22) Date of filing: 05.02.2025
(51) Int. Cl.: C07D 239/94, A61K 31/517, A61P 35/04

(54) **TRISUBSTITUTED QUINAZOLINE DERIVATIVE, ACID ADDITION SALT THEREOF, AND PHARMACEUTICAL COMPOSITION AND USE THEREOF**

(30) Priority: 05.02.2024 CN 202410164123
(71) Applicant: TELIGENE LTD, Suzhou, Jiangsu 215123 (CN)
(72) Inventor: ZHANG, Dawei, Suzhou, Jiangsu 215123 (CN)
(74) Representative: Meissner Bolte Nürnberg
(86) International application number: PCT/CN2025/075840
(87) International publication number: WO 2025/167927

(57) **Abstract**

Disclosed in the present application are a trisubstituted quinazoline derivative, an acid addition salt thereof, and a pharmaceutical composition and a use thereof. Specifically, the trisubstituted quinazoline derivative is (E)-4-(dimethylamino)-N-(4-((4-(4-fluorophenoxy)phenyl)amino)-7-ethoxyquinazolin-6-yl)but-2-enamide. Further disclosed in the present application is an acid addition salt, a solvate or a hydrate of (E)-4-(dimethylamino)-N-(4-((4-(4-fluorophenoxy)phenyl)amino)-7-ethoxyquinazolin-6-yl)but-2-enamide, or a pharmaceutical composition thereof. The substances disclosed in the present application can be used for the prevention, inhibition, or treatment of cancer.

## Description

This application claims priority to the Chinese patent application No. 202410164123.2, filed on February 5, 2024, entitled "Trisubstituted quinazoline derivative, acid addition salt thereof, and pharmaceutical composition and use thereof", the contents of which is incorporated by reference in its entirety.

### Technical Field

The present invention relates to, but is not limited to, the field of medical technology, in particular, trisubstituted quinazoline derivative, acid addition salt thereof, and pharmaceutical composition and use thereof. The trisubstituted quinazoline derivative is (E)-4-(dimethylamino)-N-(4-((4-(4-fluoro-phenoxy)phenyl)amino)-7-ethoxyquinazolin-6-yl)but-2-enamide.

### Background of the Invention

Protein kinases represent a large family of enzymes that catalyze the phosphorylation of target protein substrates. Phosphorylation generally involves the transfer of a phosphate group from adenosine triphosphate (ATP) to a protein substrate. Common attachment site for the phosphate group to the protein substrate include, for example, tyrosine, serine, or threonine residues. Due to their activity in numerous cellular processes, protein kinases have become important therapeutic targets.

The human epidermal growth factor receptor (HER) kinase family includes HER1 (erythroblastic leukemia viral oncogene homolog 1 (ErbB1), epidermal growth factor receptor (EGFR)), HER2 (ErbB2, NEU), HER3 (ErbB3), and HER4 (ErbB4). The HER kinase family plays an important regulatory role in cellular physiological processes.

EGFR is also known as HER1 or ErbB1. EGFR mutants are found in many tumors, and numerous EGFR mutations have been identified. The effects of an EGFR mutant may include: sustained activation of cells with ligand-independent receptors; disruption of receptor downregulation mechanisms due to the deletion of certain EGFR domains; activation of aberrant signaling pathways; and inhibition of apoptosis. Deletions, mutations, and rearrangements of the EGFR gene cause EGFR mutants.

HER3 overexpression is common in breast cancer, detected in 50-70% of breast cancer patients. HER3 also contributes to resistance to targeted therapies in breast cancer, activating downstream signaling pathways such as PI3K/AKT through bypass mechanisms, promoting cancer cell survival and proliferation, thereby counteracting the killing effects of therapeutic drugs on cancer cells.

Interaction of HER4 (ErbB4) with its ligand promotes receptor dimerization, kinase activation, and initiation of phosphorylation-dependent signaling cascades, such as the mitogenactivated protein kinase (MAPK) and PI3K/Akt cascades. ErbB4 is a fully functional receptor tyrosine kinase (RTK) that can function as both a homodimer and a heterodimer. It is activated by at least seven EGF-related peptide growth factors: neuregulins (NRG-1, NRG-2, NRG-3, and NRG-4), heparin-biding EGF-like growth factor (HB-EGF), betacellulin (BTC), and epiregulin (ER). Furthermore, unlike other ErbB members, ErbB4 has been shown to undergo regulated intramembrane proteolysis (RIP), resulting in the generation of a soluble intracellular domain (ICD). The released ErbB4 ICD is able to translocate to the nucleus and regulate transcription. ErbB4 and its ligands may play important roles in normal cardiovascular and neural development, mammary gland differentiation, and pathological conditions such as heart disease and cancer.

HER2, a member of the human epidermal growth factor receptor (EGFR) protein family, is overexpressed in a variety of tumor cells. Its main mechanisms of action include gene amplification, protein overexpression, and alterations in the tyrosine kinase domain. Studies have shown that HER2 gene amplification and/or HER2 protein overexpression are present in a variety of malignant tumor tissues, including 30% of breast cancer, 25%-30% of ovarian cancer, 35%-45% of pancreatic cancer, 30%-80% of esophageal adenocarcinoma and squamous cell carcinoma, and 2%-5% of colorectal cancer. Most studies have indicated that HER2 protein expression is associated with a poor outcome in breast and ovarian cancer. HER2 mutations occur in approximately 1-3% of non-small cell lung cancers, 90% of which are exon 20 insertion mutations.

Therefore, compounds that can inhibit the activity of HER2 protein kinase are highly desirable. The present invention aims to invent therapeutic drugs for HER2-related cancers.

### Summary of the Invention

In a first aspect, the present disclosure provides trisubstituted quinazoline derivative, acid addition salt thereof, or solvate or hydrate thereof, wherein the trisubstituted quinazoline derivative is (E)-4-(dimethylamino)-N-(4-((4-(4-fluoro-phenoxy)phenyl)amino)-7-ethoxyquinazoline-6-yl)but-2-enamide, having a chemical structure shown in Formula (I) below:

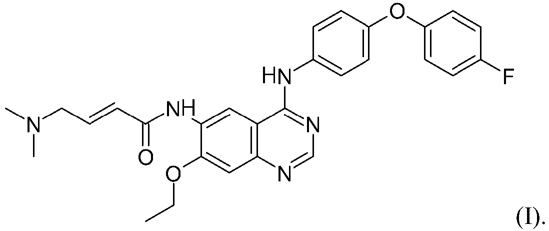

In a second aspect, the present disclosure provides a method for preparing (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof.

In a third aspect, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof.

In a fourth aspect, the present disclosure provides the use of (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, or pharmaceutical composition thereof, in the preparation of a medicament for preventing, inhibiting or treating cancer.

In a fifth aspect, the present disclosure provides the use of (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, or pharmaceutical composition thereof, as a medicament.

In a sixth aspect, the present disclosure provides the use of (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate hereof, or pharmaceutical composition thereof, in preventing, inhibiting or treating cancer.

In a seventh aspect, the present disclosure provides a method for preventing, inhibiting or treating cancer, the method comprising: administering a therapeutically effective amount of (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, or pharmaceutical composition thereof, to a subject in need thereof.

### Brief Description of the Drawings

The accompanying drawings are used to provide an understanding of the technical solution of the present disclosure and constitute a part of the specification. Together with the embodiments of the present disclosure, the drawings are used to explain the technical solution of the present disclosure, but do not constitute a limitation to the technical solution of the present disclosure.
FIG. 1 is a powder X-ray diffraction (PXRD) diagram of crystal form A of the maleate salt of the present disclosure;
FIG. 2 is a thermogravimetric analysis (TGA) diagram of crystal form A of the maleate salt of the present disclosure;
FIG. 3 is a nuclear magnetic resonance (NMR) spectrum of crystal form A of the maleate salt of the present disclosure;
FIG. 4 is a PXRD pattern of crystal form B of the maleate salt of the present disclosure;
FIG. 5 is a TGA diagram of crystal form B of the maleate salt of the present disclosure;
FIG. 6 is a PXRD pattern of crystal form C of the maleate salt of the present disclosure;
FIG. 7 is an NMR spectrum of crystal form C of the maleate salt of the present disclosure;
FIG. 8 is a PXRD pattern of crystal form S-1 of the hydrochloride salt of the present disclosure;
FIG. 9 is a PXRD pattern of crystal form S-2 of the hydrochloride salt of the present disclosure;
FIG. 10 is a PXRD pattern of crystal form S-3 of the sulfate salt of the present disclosure;
FIG. 11 is a PXRD pattern of crystal form S-4 of the sulfate salt of the present disclosure;
FIG. 12 is a PXRD pattern of crystal form S-5 of the sulfate salt of the present disclosure;
FIG. 13 is a PXRD pattern of crystal form S-6 the tartrate salt of the present disclosure;
FIG. 14 is a PXRD pattern of crystal form S-7 of the fumarate salt of the present disclosure;
FIG. 15 is a PXRD pattern of crystal form S-8 of the p-toluenesulfonate salt of the present disclosure;
FIG. 16 is a PXRD pattern of crystal form F-1 of the free base of the present disclosure;
FIG. 17 is a differential scanning calorimetry (DSC) diagram of crystal form F-1 of the free base of the present disclosure;
FIG. 18 is a TGA diagram of crystal form F-1 of the free base of the present disclosure;
FIG. 19 is a PXRD pattern of crystal form F-2 of the free base of the present disclosure;
FIG. 20 is a DSC diagram of crystal form F-2 of the free base of the present disclosure;
FIG. 21 is a TGA diagram of crystal form F-2 of the free base of the present disclosure;
FIG. 22 is a PXRD pattern of crystal form F-3 of the free base of the present disclosure;
FIG. 23 is a DSC diagram of crystal form F-3 of the free base of the present disclosure;
FIG. 24 is a TGA diagram of crystal form F-3 of the free base of the present disclosure;
FIG. 25 is a PXRD pattern of crystal form F-4 of the free base of the present disclosure;
FIG. 26 is a PXRD pattern of crystal form F-5 of the free base of the present disclosure;
FIG. 27 is a PXRD pattern of crystal form F-6 of the free base of the present disclosure;
FIG. 28 is a PXRD pattern of crystal form A of another maleate salt of the present disclosure;
FIG. 29 is a comparison of the in vivo efficacy of the maleate salt of the present disclosure vs that of neratinib.

### Detailed Description of the Invention

The present disclosure provides (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, or pharmaceutical composition thereof. The compound or the pharmaceutical composition thereof exhibits good stability, ease to handle, suitability as a drug, utility in preventing, inhibiting or treating cancer, especially having a preventive or therapeutic effect on HER2-positive advanced cancer in patients.

In an embodiment of the first aspect, the present disclosure provides a trisubstituted quinazoline derivative, acid addition salt thereof, or solvate or hydrate thereof (hereinafter referred to as (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof), wherein the chemical structure of the (E)-4-(dimethylamino)-N-(4-((4-(4-fluoro-phenoxy)phenyl)amino)-7-ethoxyquinazolin-6-yl)but-2-enamide is shown in Formula (I) below:

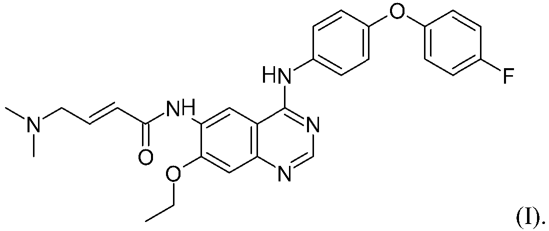

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I).

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I), which is in a solid form.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I), which is in a crystalline solid form.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I), which is in an amorphous solid form.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I) in the form of a hydrate. In one embodiment, the water content of the hydrate is from 2.1 wt% to 10.0 wt%, or from 2.2 wt% to 9.2 wt%, or from 2.3 wt% to 9.1 wt%, or from 2.3 wt% to 8.0 wt%, or from 2.1 wt% to 4.1 wt%, or from 2.2 wt% to 4.0 wt%, or from 2.8wt% to 4.0wt%, or 2.9wt%, or 3.0wt%, or 3.1wt%, or 3.2wt%, or 3.3wt%, or 3.3wt%, or 3.4wt%, or 3.5wt%, or 3.6wt%, or 3.7wt%, or 3.8wt%, or 3.9wt%, or 4.0wt%. In one embodiment, the hydrate is a monohydrate, such as 0.25 hydrate (1/4 hydrate), hemihydrate (1/2 hydrate), monohydrate, 5/4 hydrate, sesquihydrate, dihydrate, or trihydrate, etc.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide as shown in Formula (I) as a hydrate, which is in a crystalline solid form.

In one embodiment of the first aspect, the present disclosure provides a crystal form F-1 of (E)-4-(dimethylamino)-N-(4-((4-(4-fluoro-phenoxy)phenyl)amino)-7-ethoxyquinazolin-6-yl)but-2-enamide, wherein the crystal form F-1 has a purity greater than 90%. In some embodiments, the crystal form F-1 is a hydrate. In some embodiments, the powder X-ray diffraction (PXRD) pattern of the crystal form F-1 comprises at least one of characteristic peaks at 2θ values of about 5.9±0.2 degrees, 9.8±0.2 degrees, 21.6±0.2 degrees, and 25.8±0.2 degrees. In some embodiments, the PXRD pattern of the crystal form F-1 comprises the characteristic peaks at 2θ values of about 5.9±0.2 degrees, 9.8±0.2 degrees, 21.6±0.2 degrees, and 25.8±0.2 degrees. In some embodiments, the PXRD pattern of the crystal form F-1 further comprises characteristic peaks at 2θ values of about 12.9±0.2 degrees, 17.6±0.2 degrees, 20.7±0.2 degrees, 21.1±0.2 degrees, 25.3±0.2 degrees, and 30.6±0.2 degrees. In some embodiments, the PXRD pattern thereof comprises characteristic peaks at 2θ values of about 5.9±0.2 degrees, 9.8±0.2 degrees, 12.9±0.2 degrees, 17.6±0.2 degrees, 20.7±0.2 degrees, 21.1±0.2 degrees, 21.6±0.2 degrees, 25.3±0.2 degrees, 25.8±0.2 degrees, and 30.6±0.2 degrees. In one embodiment, the PXRD pattern thereof comprises the following characteristic peaks:

| **2θ** | **d-value** | **I/I₀** |
|---|---|---|
| 5.9199(17) | 14.917(4) | 100.00 |
| 7.674(5) | 11.511(8) | 10.29 |
| 9.753(2) | 9.062(2) | 58.35 |
| 11.474(11) | 7.706(7) | 4.07 |
| 11.979(11) | 7.382(7) | 7.70 |
| 12.901(4) | 6.8565(19) | 41.46 |
| 14.203(18) | 6.231(8) | 1.10 |
| 15.527(6) | 5.702(2) | 16.11 |
| 15.99(2) | 5.539(7) | 0.90 |
| 17.004(4) | 5.2103(14) | 10.31 |
| 17.554(3) | 5.0481(8) | 33.49 |
| 18.112(6) | 4.8939(15) | 12.41 |
| 18.9(2) | 4.69(5) | 9.62 |
| 19.140(5) | 4.6332(11) | 15.93 |
| 19.741(2) | 4.4936(5) | 25.38 |
| 20.743(10) | 4.279(2) | 43.12 |
| 21.120(3) | 4.2031(5) | 36.99 |
| 21.596(3) | 4.1116(5) | 62.47 |
| 22.397(4) | 3.9663(7) | 17.33 |
| 23.428(3) | 3.7941(5) | 26.38 |
| 24.316(16) | 3.657(2) | 2.61 |
| 24.834(9) | 3.5823(13) | 8.25 |
| 25.314(2) | 3.5154(3) | 30.88 |
| 25.8258(16) | 3.4470(2) | 60.03 |
| 26.985(12) | 3.3015(14) | 23.40 |
| 27.934(6) | 3.1914(7) | 6.71 |
| 29.49(4) | 3.026(4) | 12.97 |
| 30.418(14) | 2.9362(14) | 4.70 |
| 30.620(17) | 2.9173(16) | 30.44 |
| 31.82(3) | 2.810(3) | 0.89 |
| 32.45(4) | 2.757(3) | 3.52 |
| 33.11(3) | 2.704(3) | 1.98 |
| 35.091(7) | 2.5552(5) | 6.38 |
| 36.29(2) | 2.4737(14) | 3.23 |
| 38.27(3) | 2.3501(17) | 2.83 |
| 39.068(19) | 2.3037(11) | 1.87 |
| 39.89(3) | 2.2580(15) | 1.57 |

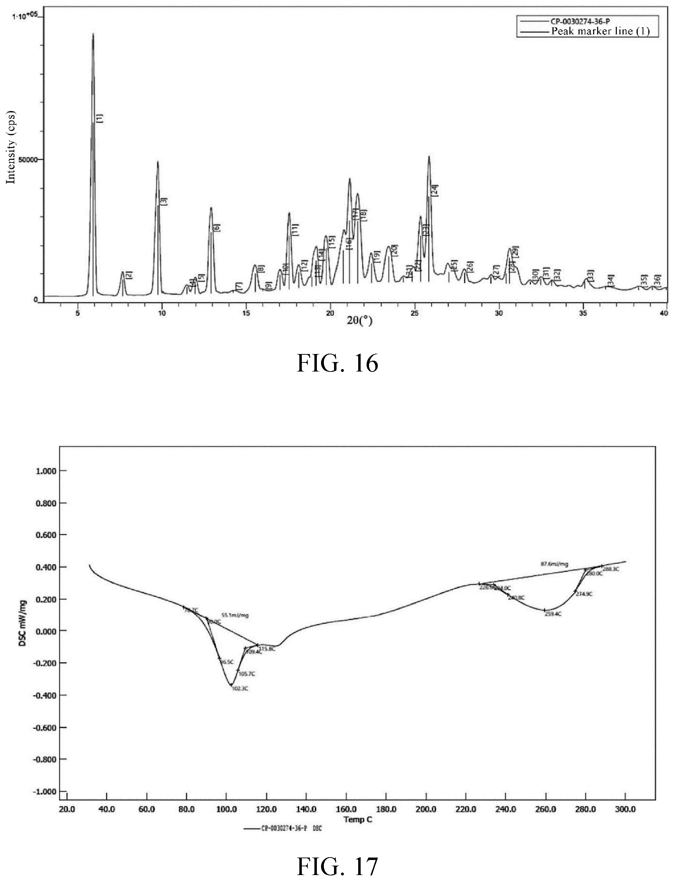

In one embodiment of the first aspect, the crystal form F-1 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 16.

In one embodiment of the first aspect, the crystal form F-1 of (E)-4-(dimethylamino)-N-(4-((4-(4-fluoro-phenoxy)phenyl)amino)-7-ethoxyquinazolin-6-yl)but-2-enamide provided herein has a differential scanning calorimetry (DSC) thermogram substantially the same as that shown in FIG. 17.

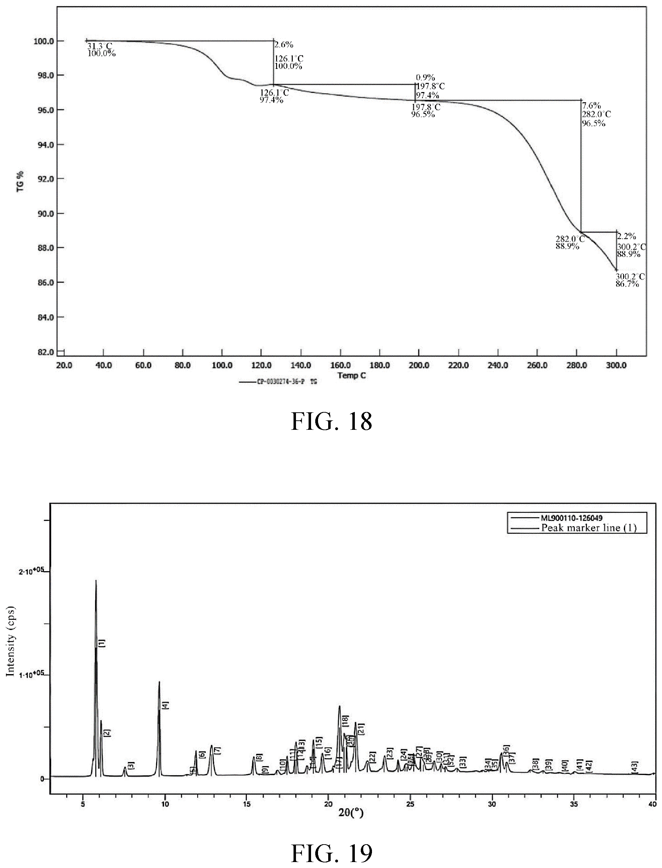

In one embodiment of the first aspect, the crystal form F-1 of (E)-4-(dimethylamino)-N-(4-((4-(4-fluoro-phenoxy)phenyl)amino)-7-ethoxyquinazolin-6-yl)but-2-enamide provided herein has a thermogravimetric analysis (TGA) diagram substantially the same as that shown in FIG. 18.

In one embodiment of the first aspect, the present disclosure provides a crystal form F-2 of (E)-4-(dimethylamino)-N-(4-((4-(4-fluoro-phenoxy)phenyl)amino)-7-ethoxyquinazolin-6-yl)but-2-enamide, wherein the crystal form F-2 has a purity greater than 90%. In some embodiments, the crystal form F-2 is a hydrate. In some embodiments, the PXRD pattern of the crystal form F-2 comprises at least one of the characteristic peaks at 2θ values of about 5.8±0.2 degrees, 9.6±0.2 degrees, 20.7±0.2 degrees, and 21.6±0.2 degrees. In some embodiments, the PXRD pattern of the crystal form F-2 comprises characteristic peaks at 2θ values of about 5.8±0.2 degrees, 9.6±0.2 degrees, 20.7±0.2 degrees, and 21.6±0.2 degrees. In some embodiments, the PXRD pattern of the crystal form F-2 further comprises characteristic peaks at 2θ values of about 6.1±0.2 degrees, 12.8±0.2 degrees, 19.1±0.2 degrees, and 20.9±0.2 degrees. In some embodiments, the PXRD pattern thereof comprises characteristic peaks at 2θ values of about 5.8±0.2 degrees, 6.1±0.2 degrees, 9.6±0.2 degrees, 12.8±0.2 degrees, 19.1±0.2 degrees, 20.7±0.2 degrees, 20.9±0.2 degrees, and 21.6±0.2 degrees. In one embodiment, the PXRD pattern thereof comprises the following characteristic peaks:

| **2θ** | **d-value** | **I/I₀** |
|---|---|---|
| 5.780(4) | 15.279(10) | 100.00 |
| 6.092(4) | 14.495(9) | 24.43 |
| 7.54858 | 11.70199 | 6.21 |
| 9.64871 | 9.15913 | 47.19 |
| 11.35649 | 7.78534 | 0.57 |
| 11.90967 | 7.42494 | 13.57 |
| 12.82850 | 6.89512 | 25.48 |
| 15.40875 | 5.74583 | 11.97 |
| 15.76820 | 5.61565 | 0.68 |
| 16.85818 | 5.25494 | 3.22 |
| 17.46472 | 5.07378 | 9.13 |
| 17.94327 | 4.93952 | 6.05 |
| 18.02760 | 4.91661 | 11.96 |
| 18.69056 | 4.74368 | 3.86 |
| 19.07566 | 4.64878 | 20.48 |
| 19.61250 | 4.52272 | 12.09 |
| 20.27436 | 4.37655 | 1.24 |
| 20.65610 | 4.29652 | 53.20 |
| 20.93881 | 4.23914 | 21.57 |
| 21.06347 | 4.21433 | 11.10 |
| 21.62491 | 4.10617 | 39.87 |
| 22.33265 | 3.97762 | 11.26 |
| 23.39352 | 3.79958 | 12.31 |
| 24.23688 | 3.66924 | 6.18 |
| 24.67637 | 3.60488 | 4.65 |
| 24.78825 | 3.58886 | 2.31 |
| 25.18490 | 3.53323 | 12.87 |
| 25.63768 | 3.47185 | 5.54 |
| 25.77871 | 3.45318 | 6.82 |
| 26.42938 | 3.36962 | 7.33 |
| 26.85995 | 3.31657 | 4.89 |
| 27.12498 | 3.28477 | 3.02 |
| 27.85367 | 3.20047 | 2.25 |
| 29.39670 | 3.03589 | 0.54 |
| 29.81641 | 2.99410 | 2.86 |
| 30.51388 | 2.92724 | 14.58 |
| 30.86484 | 2.89475 | 8.22 |
| 32.30962 | 2.76852 | 2.54 |
| 33.10894 | 2.70349 | 1.46 |
| 34.07558 | 2.62898 | 0.51 |
| 34.99409 | 2.56205 | 1.45 |
| 35.55966 | 2.52258 | 0.51 |
| 38.34600 | 2.34535 | 0.58 |
| 39.85098 | 2.26027 | 1.17 |

In one embodiment of the first aspect, the crystal form F-2 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 19.

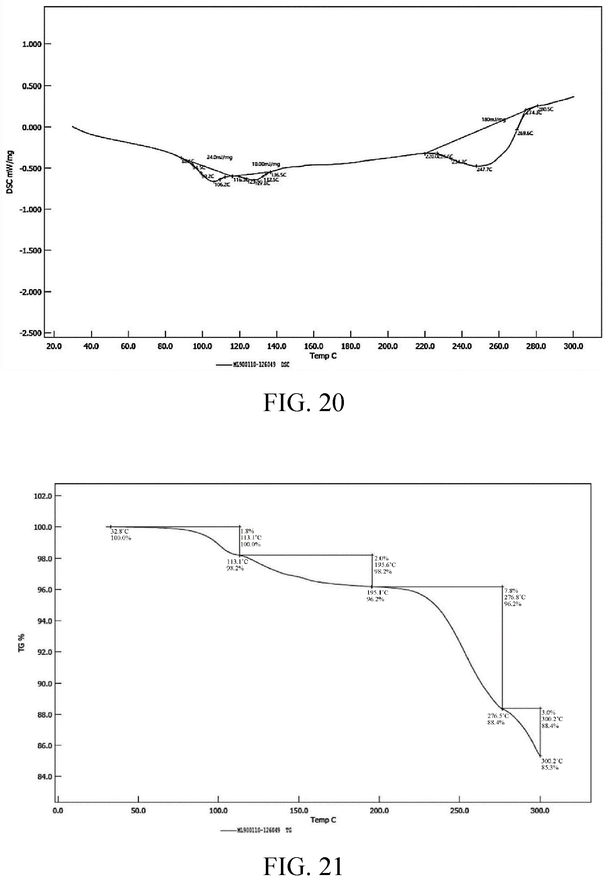

In one embodiment of the first aspect, the crystal form F-2 of (E)-4-(dimethylamino)-N-(4-((4-(4-fluoro-phenoxy)phenyl)amino)-7-ethoxyquinazolin-6-yl)but-2-enamide provided herein has a DSC thermogram substantially the same as that shown in FIG. 20.

In one embodiment of the first aspect, the crystal form F-2 of (E)-4-(dimethylamino)-N-(4-((4-(4-fluoro-phenoxy)phenyl)amino)-7-ethoxyquinazolin-6-yl)but-2-enamide provided herein has a TGA diagram substantially the same as that shown in FIG. 21.

In one embodiment of the first aspect, the present disclosure provides a crystal form F-3 of (E)-4-(dimethylamino)-N-(4-((4-(4-fluoro-phenoxy)phenyl)amino)-7-ethoxyquinazolin-6-yl)but-2-enamide, wherein the crystal form F-3 has a purity greater than 90%. In some embodiments, the crystal form F-3 is a hydrate. In some embodiments, the PXRD pattern of the crystal form F-3 comprises at least one of the characteristic peaks at 2θ values of about 20.2±0.2 degrees, 21.0±0.2 degrees, 24.1±0.2 degrees, and 25.3±0.2 degrees. In some embodiments, the PXRD pattern of the crystal form F-3 comprises the characteristic peaks at 2θ values of about 20.2±0.2 degrees, 21.0±0.2 degrees, 24.1±0.2 degrees, and 25.3±0.2 degrees. In some embodiments, the PXRD pattern of the crystal form F-3 further comprises characteristic peaks at 2θ values of about 8.2±0.2 degrees, 11.8±0.2 degrees, 17.8±0.2 degrees, 25.5±0.2 degrees, and 26.7±0.2 degrees. In some embodiments, the PXRD pattern thereof comprises characteristic peaks at 2θ values of about 8.2±0.2 degrees, 11.8±0.2 degrees, 17.8±0.2 degrees, 20.2±0.2 degrees, 21.0±0.2 degrees, 24.1±0.2 degrees, 25.3±0.2 degrees, 25.5±0.2 degrees, and 26.7±0.2 degrees. In one embodiment, the PXRD pattern thereof comprises the following characteristic peaks:

| **2θ** | **d-value** | **I/I₀** |
|---|---|---|
| 6.247(3) | 14.137(6) | 4.11 |
| 7.153(8) | 12.348(14) | 1.23 |
| 8.164(4) | 10.821(5) | 6.24 |
| 10.24(3) | 8.63(3) | 1.26 |
| 11.236(5) | 7.868(3) | 1.81 |
| 11.755(3) | 7.522(2) | 7.59 |
| 12.566(17) | 7.039(9) | 0.29 |
| 13.291(4) | 6.6563(19) | 2.96 |
| 14.273(11) | 6.201(5) | 0.84 |
| 14.873(9) | 5.952(3) | 4.80 |
| 15.406(8) | 5.747(3) | 3.61 |
| 16.678(8) | 5.311(3) | 2.24 |
| 17.183(5) | 5.1562(14) | 4.05 |
| 17.765(17) | 4.989(5) | 9.13 |
| 18.027(16) | 4.917(4) | 2.96 |
| 18.624(3) | 4.7604(9) | 5.82 |
| 19.284(11) | 4.599(2) | 2.42 |
| 19.685(4) | 4.5061(9) | 5.88 |
| 20.203(3) | 4.3919(6) | 22.13 |
| 21.015(3) | 4.2239(5) | 25.57 |
| 21.395(6) | 4.1498(11) | 2.09 |
| 21.76(3) | 4.081(6) | 4.06 |
| 23.154(9) | 3.8384(14) | 1.24 |
| 24.112(4) | 3.6880(6) | 16.64 |
| 25.29(8) | 3.519(11) | 100.00 |
| 25.506(10) | 3.4895(13) | 7.25 |
| 26.364(14) | 3.3778(17) | 2.69 |
| 26.721(9) | 3.3335(11) | 12.70 |
| 27.464(17) | 3.245(2) | 1.80 |
| 28.106(14) | 3.1723(16) | 1.20 |
| 30.09(2) | 2.967(2) | 2.71 |
| 30.885(16) | 2.8929(14) | 3.51 |
| 31.804(11) | 2.8114(10) | 1.32 |
| 33.02(4) | 2.711(3) | 2.93 |
| 34.807(18) | 2.5754(13) | 0.77 |
| 36.37(2) | 2.4683(13) | 1.31 |
| 36.947(10) | 2.4310(6) | 0.47 |
| 39.84(2) | 2.2610(13) | 2.41 |

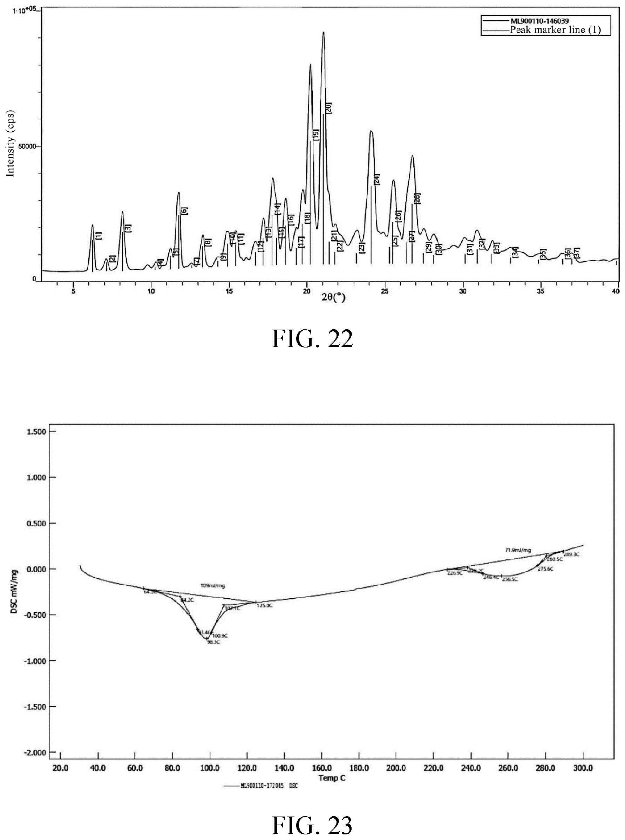

In one embodiment of the first aspect, the crystal form F-3 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 22.

In one embodiment of the first aspect, the crystal form F-3 of (E)-4-(dimethylamino)-N-(4-((4-(4-fluoro-phenoxy)phenyl)amino)-7-ethoxyquinazolin-6-yl)but-2-enamide provided herein has a DSC thermogram substantially the same as that shown in FIG. 23.

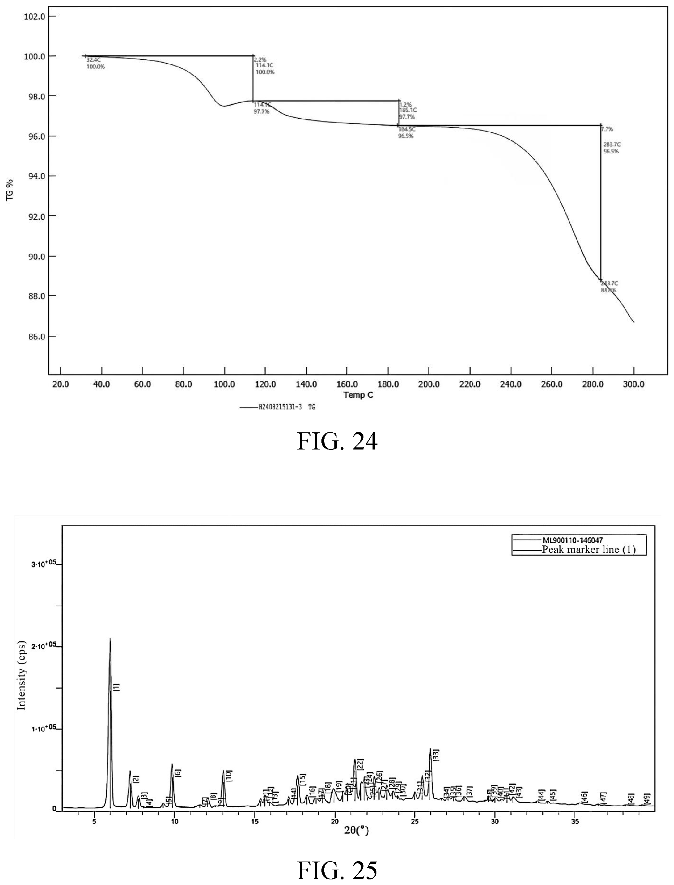

In one embodiment of the first aspect, the crystal form F-3 of (E)-4-(dimethylamino)-N-(4-((4-(4-fluoro-phenoxy)phenyl)amino)-7-ethoxyquinazolin-6-yl)but-2-enamide provided herein has a TGA diagram substantially the same as that shown in FIG. 24.

In one embodiment of the first aspect, the present disclosure provides a crystal form F-4 of (E)-4-(dimethylamino)-N-(4-((4-(4-fluoro-phenoxy)phenyl)amino)-7-ethoxyquinazolin-6-yl)but-2-enamide, wherein the crystal form F-4 has a purity greater than 90%. In some embodiments, the PXRD pattern of the crystal form F-4 comprises at least one of the characteristic peaks at 2θ values of about 6.0±0.2 degrees, 9.8±0.2 degrees, 21.2±0.2 degrees, and 25.9±0.2 degrees. In some embodiments, the PXRD pattern of the crystal form F-4 comprises characteristic peaks at 2θ values of about 6.0±0.2 degrees, 9.8±0.2 degrees, 21.2±0.2 degrees, and 25.9±0.2 degrees. In some embodiments, the PXRD pattern of the crystal form F-4 further comprises characteristic peaks at 2θ values of about 7.2±0.2 degrees, 13.0±0.2 degrees, 17.6±0.2 degrees, and 20.8±0.2 degrees. In some embodiments, the PXRD pattern thereof comprises characteristic peaks at 2θ values of about 6.0±0.2 degrees, 7.2±0.2 degrees, 9.8±0.2 degrees, 13.0±0.2 degrees, 17.6±0.2 degrees, 20.8±0.2 degrees, 21.2±0.2 degrees, and 25.9±0.2 degrees. In one embodiment, the PXRD pattern thereof comprises the following characteristic peaks:

| **2θ** | **d-value** | **I/I₀** |
|---|---|---|
| 6.0036(13) | 14.709(3) | 100.00 |
| 7.231(2) | 12.215(3) | 21.07 |
| 7.743(3) | 11.408(4) | 7.20 |
| 8.06(5) | 10.96(6) | 1.07 |
| 9.293(3) | 9.509(3) | 2.41 |
| 9.8366(15) | 8.9846(14) | 25.70 |
| 11.558(5) | 7.650(3) | 2.03 |
| 12.068(2) | 7.3277(14) | 5.01 |
| 12.559(10) | 7.043(5) | 0.57 |
| 13.0275(19) | 6.7902(10) | 20.11 |
| 15.348(7) | 5.768(3) | 3.79 |
| 15.633(5) | 5.6640(17) | 5.75 |
| 15.907(9) | 5.567(3) | 2.71 |
| 17.099(4) | 5.1815(13) | 3.93 |
| 17.634(4) | 5.0255(10) | 19.28 |
| 18.233(5) | 4.8617(13) | 5.66 |
| 18.801(8) | 4.7160(9) | 3.80 |
| 19.217(4) | 4.6149(8) | 7.42 |
| 19.911(5) | 4.4555(12) | 11.55 |
| 20.500(4) | 4.3290(8) | 3.38 |
| 20.818(6) | 4.2635(12) | 17.17 |
| 21.237(2) | 4.1804(4) | 22.13 |
| 21.638(6) | 4.1038(12) | 8.90 |
| 21.826(5) | 4.0689(9) | 10.66 |
| 22.03(2) | 4.031(4) | 1.48 |
| 22.447(2) | 3.9576(4) | 12.98 |
| 22.745(4) | 3.9064(7) | 7.05 |
| 23.234(3) | 3.8253(5) | 9.59 |
| 23.612(5) | 3.7650(9) | 5.57 |
| 23.898(8) | 3.7205(12) | 1.13 |
| 24.990(6) | 3.5604(8) | 3.74 |
| 25.455(6) | 3.4963(8) | 13.40 |
| 25.941(2) | 3.4319(3) | 27.58 |
| 26.630(11) | 3.3447(13) | 0.69 |
| 27.059(13) | 3.2926(16) | 2.30 |
| 27.369(9) | 3.2561(11) | 2.11 |
| 28.051(15) | 3.1784(16) | 2.74 |
| 29.36(4) | 3.040(4) | 0.89 |
| 29.598(6) | 3.0157(6) | 3.35 |
| 30.014(8) | 2.9748(7) | 2.69 |
| 30.372(8) | 2.9406(7) | 1.55 |
| 30.732(8) | 2.9070(8) | 7.54 |
| 31.136(7) | 2.8701(7) | 3.44 |
| 32.55(3) | 2.749(2) | 1.17 |
| 33.262(15) | 2.6914(12) | 1.18 |
| 35.22(3) | 2.546(2) | 2.32 |
| 36.41(2) | 2.4657(15) | 0.55 |
| 38.15(3) | 2.357(2) | 1.30 |
| 39.143(13) | 2.2995(7) | 0.67 |

In one embodiment of the first aspect, the crystal form F-4 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 25.

In one embodiment of the first aspect, the present disclosure provides a crystal form F-5 of (E)-4-(dimethylamino)-N-(4-((4-(4-fluoro-phenoxy)phenyl)amino)-7-ethoxyquinazolin-6-yl)but-2-enamide, wherein the crystal form F-5 has a purity greater than 90%. In some embodiments, the PXRD pattern of the crystal form F-5 comprises at least one of the characteristic peaks at 2θ values of about 6.0±0.2 degrees, 11.8±0.2 degrees, 21.1±0.2 degrees, and 22.3±0.2 degrees. In some embodiments, the PXRD pattern of the crystal form F-5 comprises characteristic peaks at 2θ values of about 6.0±0.2 degrees, 11.8±0.2 degrees, 21.1±0.2 degrees, and 22.3±0.2 degrees. 2 degrees, 21.1±0.2 degrees, and 22.3±0.2. In some embodiments, the PXRD pattern of the crystal form F-5 further comprises characteristic peaks at 2θ values of about 6.1±0.2 degrees, 8.2±0.2 degrees, 20.3±0.2 degrees, 24.3±0.2 degrees, and 26.8±0.2 degrees. In some embodiments, the PXRD pattern thereof comprises characteristic peaks at 2θ values of about 6.0±0.2 degrees, 6.1±0.2 degrees, 8.2±0.2 degrees, 11.8±0.2 degrees, 20.3±0.2 degrees, 21.1±0.2 degrees, 22.3±0.2 degrees, 24.3±0.2 degrees, and 26.8±0.2 degrees. In one embodiment, the PXRD pattern thereof comprises the following characteristic peaks:

| **2θ** | **d-value** | **I/I₀** |
|---|---|---|
| 5.894(3) | 14.982(8) | 1.26 |
| 5.9824(12) | 14.762(3) | 7.34 |
| 6.0432(12) | 14.613(3) | 14.74 |
| 6.1391(15) | 14.385(4) | 10.76 |
| 6.342(2) | 13.925(5) | 9.13 |
| 6.4332(17) | 13.728(4) | 1.62 |
| 7.16(3) | 12.34(4) | 1.75 |
| 8.189(5) | 10.789(7) | 11.89 |
| 9.829(13) | 8.992(12) | 0.97 |
| 9.922(5) | 8.907(5) | 0.26 |
| 10.363(9) | 8.529(7) | 0.89 |
| 10.891(18) | 8.117(14) | 0.32 |
| 11.309(7) | 7.818(5) | 1.47 |
| 11.8463(12) | 7.4645(7) | 26.73 |
| 11.984(4) | 7.379(2) | 0.71 |
| 12.109(5) | 7.303(3) | 1.46 |
| 12.18(9) | 7.26(5) | 1.69 |
| 12.721(8) | 6.953(4) | 1.19 |
| 13.054(7) | 6.777(4) | 0.30 |
| 13.355(4) | 6.6247(18) | 2.99 |
| 14.302(6) | 6.188(2) | 0.84 |
| 14.853(3) | 5.9596(14) | 5.59 |
| 15.454(3) | 5.7293(12) | 2.79 |
| 16.087(16) | 5.505(5) | 0.15 |
| 16.544(16) | 5.354(5) | 0.64 |
| 16.663(13) | 5.316(4) | 0.64 |
| 16.810(14) | 5.270(4) | 1.26 |
| 17.249(7) | 5.1368(19) | 2.77 |
| 17.754(4) | 4.9917(10) | 5.02 |
| 18.036(6) | 4.9142(16) | 5.52 |
| 18.701(5) | 4.7412(13) | 5.14 |
| 19.413(9) | 4.569(2) | 5.19 |
| 19.802(7) | 4.4798(15) | 4.91 |
| 20.266(7) | 4.3783(15) | 13.41 |
| 21.111(5) | 4.2050(10) | 29.23 |
| 21.423(13) | 4.144(2) | 4.23 |
| 21.864(13) | 4.062(2) | 1.18 |
| 22.20(4) | 4.001(7) | 0.45 |
| 22.28(10) | 3.987(17) | 100.00 |
| 23.227(11) | 3.8265(18) | 2.80 |
| 23.979(4) | 3.7081(6) | 6.49 |
| 24.291(5) | 3.6612(8) | 12.64 |
| 24.887(8) | 3.5749(12) | 1.20 |
| 25.652(5) | 3.4699(6) | 10.61 |
| 25.961(9) | 3.4293(12) | 1.67 |
| 26.490(2) | 3.3621(3) | 4.28 |
| 26.839(5) | 3.3191(6) | 11.38 |
| 27.551(6) | 3.2349(7) | 2.45 |
| 28.069(11) | 3.1764(12) | 0.67 |
| 28.293(7) | 3.1518(7) | 1.50 |
| 29.544(10) | 3.0210(10) | 0.53 |
| 30.09(2) | 2.968(2) | 1.03 |
| 30.922(11) | 2.8895(10) | 3.80 |
| 31.212(17) | 2.8634(15) | 1.56 |
| 31.485(19) | 2.8391(17) | 0.44 |
| 31.948(3) | 2.7990(3) | 1.76 |
| 32.59(2) | 2.7453(17) | 0.15 |
| 33.18(2) | 2.6978(17) | 2.74 |
| 34.902(6) | 2.5686(4) | 1.51 |
| 35.214(12) | 2.5466(9) | 0.56 |
| 36.258(6) | 2.4756(4) | 1.02 |
| 36.57(2) | 2.4549(13) | 0.61 |
| 37.025(8) | 2.4261(5) | 0.35 |
| 37.153(10) | 2.4180(6) | 0.39 |
| 38.35(2) | 2.3450(14) | 0.07 |
| 38.912(10) | 2.3126(6) | 0.41 |
| 39.165(14) | 2.2982(8) | 0.12 |

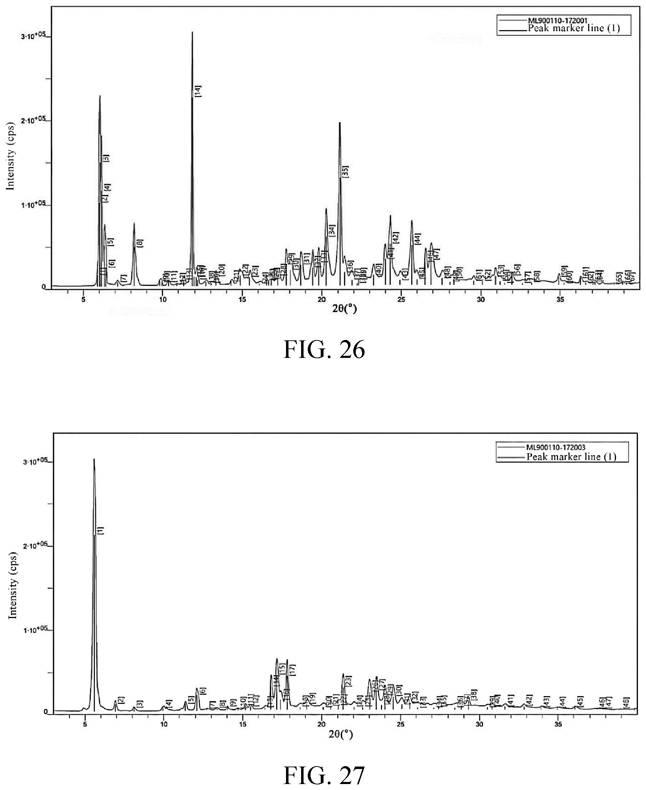

In one embodiment of the first aspect, the crystal form F-5 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 26.

In one embodiment of the first aspect, the present disclosure provides a crystal form F-6 of (E)-4-(dimethylamino)-N-(4-((4-(4-fluoro-phenoxy)phenyl)amino)-7-ethoxyquinazolin-6-yl)but-2-enamide, wherein the crystal form F-6 has a purity greater than 90%. In some embodiments, the PXRD pattern of the crystal form F-6 comprises at least one of the characteristic peaks at 2θ values of about 5.6±0.2 degrees, 17.2±0.2 degrees, 17.8±0.2 degrees, and 23.8±0.2 degrees. In some embodiments, the PXRD pattern of the crystal form F-6 comprises characteristic peaks at 2θ values of about 5.6±0.2 degrees, 17.2±0.2 degrees, 17.8±0.2 degrees, and 23.8±0.2 degrees. In some embodiments, the PXRD pattern of the crystal form F-6 further comprises characteristic peaks at 2θ values of about 12.1±0.2 degrees, 16.8±0.2 degrees, 21.3±0.2 degrees, 23.0±0.2 degrees, 23.5±0.2 degrees, and 24.5±0.2 degrees. In some embodiments, the PXRD pattern thereof comprises characteristic peaks at 2θ values of about 5.6±0.2 degrees, 12.1±0.2 degrees, 16.8±0.2 degrees, 17.2±0.2 degrees, 17.8±0.2 degrees, 21.3±0.2 degrees, 23.0±0.2 degrees, 23.5±0.2 degrees, 23.8±0.2 degrees, and 24.5±0.2 degrees. In one embodiment, the PXRD pattern thereof comprises the following characteristic peaks:

| **2θ** | **d-value** | **I/I₀** |
|---|---|---|
| 5.604(4) | 15.757(10) | 85.19 |
| 6.918(2) | 12.766(4) | 3.11 |
| 8.099(11) | 10.909(15) | 1.57 |
| 9.959(5) | 8.875(4) | 2.33 |
| 11.369(9) | 7.777(6) | 3.34 |
| 12.098(3) | 7.310(2) | 6.51 |
| 12.715(18) | 6.956(10) | 0.92 |
| 13.365(6) | 6.619(3) | 0.79 |
| 14.036(10) | 6.304(4) | 0.81 |
| 14.69(2) | 6.027(9) | 0.43 |
| 15.171(6) | 5.835(2) | 1.30 |
| 15.488(9) | 5.717(3) | 1.38 |
| 16.40(2) | 5.401(8) | 0.40 |
| 16.786(2) | 5.2773(7) | 7.78 |
| 17.152(3) | 5.1657(9) | 18.28 |
| 17.419(3) | 5.0870(10) | 3.71 |
| 17.793(2) | 4.9807(7) | 13.49 |
| 18.640(13) | 4.756(3) | 1.46 |
| 19.073(12) | 4.649(3) | 3.54 |
| 20.092(6) | 4.4158(14) | 0.76 |
| 20.548(4) | 4.3190(8) | 1.10 |
| 21.034(6) | 4.2202(12) | 2.15 |
| 21.334(2) | 4.1614(4) | 7.99 |
| 22.048(8) | 4.0282(14) | 0.55 |
| 22.597(10) | 3.9317(17) | 0.96 |
| 23.028(4) | 3.8590(6) | 8.35 |
| 23.467(4) | 3.7879(6) | 8.62 |
| 23.77(8) | 3.740(13) | 100.00 |
| 24.003(3) | 3.7044(5) | 5.34 |
| 24.533(3) | 3.6256(5) | 5.63 |
| 25.057(5) | 3.5510(7) | 1.78 |
| 25.562(4) | 3.4819(5) | 2.41 |
| 26.07(2) | 3.416(3) | 0.49 |
| 27.101(15) | 3.2876(18) | 0.41 |
| 27.39(2) | 3.254(2) | 1.09 |
| 28.436(10) | 3.1362(11) | 0.18 |
| 28.855(14) | 3.0917(15) | 2.72 |
| 29.286(5) | 3.0471(5) | 2.45 |
| 30.49(2) | 2.9292(19) | 0.87 |
| 30.776(7) | 2.9029(6) | 0.74 |
| 31.625(18) | 2.8269(16) | 1.25 |
| 32.80(2) | 2.7285(18) | 1.59 |
| 33.91(3) | 2.641(3) | 0.64 |
| 34.914(16) | 2.5678(12) | 0.64 |
| 36.041(6) | 2.4900(4) | 0.84 |
| 37.44(5) | 2.400(3) | 0.29 |
| 37.84(2) | 2.3754(14) | 0.18 |
| 38.946(18) | 2.3107(10) | 0.12 |
| 39.838(11) | 2.2610(6) | 0.34 |

In one embodiment of the first aspect, the crystal form F-6 of (E)-4-(dimethylamino)-N-(4-((4-(4-fluoro-phenoxy)phenyl)amino)-7-ethoxyquinazolin-6-yl)but-2-enamide provided herein has a PXRD pattern substantially the same as that shown in FIG. 27.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt or solvate or hydrate thereof, wherein the acid in the acid addition salt is hydrochloric acid, sulfuric acid, nitric acid, phosphoric acid, acetic acid, propionic acid, glycolic acid, oxalic acid, maleic acid, fumaric acid, malonic acid, succinic acid, tartaric acid, citric acid, mandelic acid, methanesulfonic acid, ethanesulfonic acid, benzenesulfonic acid, p-toluenesulfonic acid, trifluoroacetic acid, 1-hydroxy-2-naphthoic acid, or benzoic acid.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide hydrochloride, or solvate or hydrate thereof.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide sulfate, or solvate or hydrate thereof.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide maleate, or solvate or hydrate thereof.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide fumarate, or solvate or hydrate thereof.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide tartrate, or solvate or hydrate thereof.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide p-toluenesulfonate, or solvate or hydrate thereof.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-eneamide acid addition salt in a solvate form, wherein the solvate includes, but is not limited to, a solvate formed with one or more solvents selected from the group consisting of water, ethanol, ethylene glycol, (S)-propylene glycol, (R)-propylene glycol, and methyl tert-butyl ether. When the solvent is water, the solvate is a hydrate. The solvate and/or hydrate is preferably present in a solid form. The solvate and/or hydrate is preferably present in a crystalline solid form. The solvate can also be an intermediate solvate used to prepare a more desirable solvate. The solvent in the intermediate solvate includes, but is not limited to, one or more solvents selected from the group consisting of methanol, methyl tert-butyl ether, ethyl acetate, and methyl acetate.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt in a hydrate form, such as, for example, 0.25 hydrate (1/4 hydrate), hemihydrate (1/2 hydrate), monohydrate, 5/4 hydrate, sesquihydrate, dihydrate, or trihydrate, etc.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt in an anhydrous form, such as, for example, its water content does not exceed 0.5 wt%, 0.4 wt%, 0.3 wt%, 0.2 wt%, 0.1 wt%, or 0.09 wt%.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt, or solvate or hydrate thereof, in a solid form, preferably, in a crystalline solid form.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt, or solvate or hydrate thereof, in a solid form, preferably, in an amorphous solid form.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt, or solvate or hydrate thereof, in a crystalline solid form of (E)-4-(dimethylamino)-N-(4-((4-(4-fluoro-phenoxy)phenyl)amino)-7-ethoxyquinazolin-6-yl)but-2-enoamide maleate salt, or solvate or hydrate thereof, wherein the molar ratio of (E)-4-(dimethylamino)-N-(4-((4-(4-fluoro-phenoxy)phenyl)amino)-7-ethoxyquinazolin-6-yl)but-2-enoamide to maleic acid is 1:1 or 1:2.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt, or solvate or hydrate thereof, in an amorphous form of (E)-4-(dimethylamino)-N-(4-((4-(4-fluoro-phenoxy)phenyl)amino)-7-ethoxyquinazolin-6-yl)but-2-enoamide maleate, or solvate or hydrate thereof, wherein the molar ratio of (E)-4-(dimethylamino)-N-(4-((4-(4-fluoro-phenoxy)phenyl)amino)-7-ethoxyquinazolin-6-yl)but-2-enoamide to maleic acid is 1:1 or 1:2.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide maleate, which maleate is an anhydrate.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide maleate hydrate.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide maleate hydrate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:2. In one embodiment, the water content of the maleate hydrate is from 2.1 wt% to 10.0 wt%, or from 2.2 wt% to 9.2 wt%, or from 2.3 wt% to 9.1 wt%, or from 2.3 wt% to 8.0 wt%, or from 2.1 wt% to 3.1 wt%, or from 2.2 wt% to 2.8 wt%, or from 2.3 wt% to 2.6 wt%, or 2.3 wt%, or 2.4 wt%, or 2.5 wt%, or 2.6 wt%. In one embodiment, the maleate hydrate is a monohydrate.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:2, and the maleate is an anhydrate.

In one embodiment of the first aspect, the present disclosure provides (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:1.

In some embodiments of the first aspect, the present disclosure provides (E)-4-(dimethylamino)-N-(4-((4-(4-fluoro-phenoxy)phenyl)amino)-7-ethoxyquinazolin-6-yl)but-2-enoamide acid addition salt, or solvate or hydrate thereof, which is crystalline.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide hydrochloride, or solvate or hydrate thereof.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide hydrochloride, or solvate or hydrate thereof.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide sulfate, or solvate or hydrate thereof.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide sulfate, or solvate or hydrate thereof.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate, which is an anhydrate.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide maleate, which is an anhydrate.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate hydrate.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide maleate hydrate.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide fumarate, or solvate or hydrate thereof.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide fumarate, or solvate or hydrate thereof.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide tartrate, or solvate or hydrate thereof.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide tartrate, or solvate or hydrate thereof.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide p-toluenesulfonate, or solvate or hydrate thereof.

In one embodiment of the first aspect, the present disclosure provides amorphous (E)-4-(dimethylamino)-N-(4-((4-(4-fluoro-phenoxy)phenyl)amino)-7-ethoxyquinazolin-6-yl)but-2-enamide p-toluenesulfonate, or solvate or hydrate thereof.

In some embodiments of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide hydrochloride, wherein the molar ratio of (E)-4-(dimethylamino)-N-(4-((4-(4-fluoro-phenoxy)phenyl)amino)-7-ethoxyquinazolin-6-yl)but-2-enamide to HCl is 1:1.

In some embodiments of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide sulfate, wherein the molar ratio of (E)-4-(dimethylamino)-N-(4-((4-(4-fluoro-phenoxy)phenyl)amino)-7-ethoxyquinazolin-6-yl)but-2-enamide to sulfuric acid is 1:1.

In some embodiments of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate, or hydrate thereof, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:1 or 1:2.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate, or hydrate thereof, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:2. In one embodiment, the maleate, or hydrate thereof, is crystalline. In one embodiment, the maleate hydrate is a monohydrate. In one embodiment, the maleate is an anhydrate.

In one embodiment of the first aspect, the present disclosure provides a crystal form A of (E)-4-(dimethylamino)-N-(4-((4-(4-fluoro-phenoxy)phenyl)amino)-7-ethoxyquinazolin-6-yl)but-2-enamide maleate hydrate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form A is 1:2. Preferably, the crystal form A has a purity greater than 90%. In some embodiments, the PXRD pattern of the crystal form A comprises at least one of the characteristic peaks at 2θ values of about 5.1±0.2 degrees, 5.7±0.2 degrees, 18.4±0.2 degrees, and 26.9±0.2 degrees. In some embodiments, the PXRD pattern of Form A comprises characteristic peaks at 2θ values of about 5.1±0.2 degrees, 5.7±0.2 degrees, 18.4±0.2 degrees, and 26.9±0.2 degrees. In some embodiments, the PXRD pattern of Form A further comprises characteristic peaks at 2θ values of about 18.0±0.2 degrees, 20.4±0.2 degrees, 21.1±0.2 degrees, and 25.5±0.2 degrees. In some embodiments, the PXRD pattern thereof comprises characteristic peaks at 2θ values of about 5.1±0.2 degrees, 5.7±0.2 degrees, 18.0±0.2 degrees, 18.4±0.2 degrees, 20.4±0.2 degrees, 21.1±0.2 degrees, 25.5±0.2 degrees, and 26.9±0.2 degrees. In one embodiment, the PXRD pattern thereof comprises the following characteristic peaks:

| **2θ** | **d-value** | **I/I₀** |
|---|---|---|
| 5.100 | 17.3131 | 64 |
| 5.680 | 15.5465 | 100 |
| 10.500 | 8.4182 | 22 |
| 11.240 | 7.8656 | 26 |
| 11.300 | 7.8240 | 28 |
| 16.700 | 5.3042 | 22 |
| 16.900 | 5.2419 | 29 |
| 18.020 | 4.9186 | 34 |
| 18.400 | 4.8178 | 47 |
| 18.480 | 4.7972 | 44 |
| 18.900 | 4.6915 | 22 |
| 19.000 | 4.6670 | 23 |
| 19.780 | 4.4847 | 22 |
| 20.420 | 4.3456 | 31 |
| 20.880 | 4.2509 | 29 |
| 21.080 | 4.2110 | 31 |
| 22.000 | 4.0369 | 23 |
| 25.180 | 3.5338 | 25 |
| 25.360 | 3.5092 | 33 |
| 25.480 | 3.4929 | 36 |
| 25.940 | 3.4320 | 23 |
| 26.040 | 3.4190 | 24 |
| 26.720 | 3.3336 | 48 |
| 26.780 | 3.3262 | 56 |
| 26.880 | 3.3141 | 61 |
| 27.260 | 3.2687 | 29 |
| 27.380 | 3.2547 | 28 |
| 27.540 | 3.2361 | 27 |

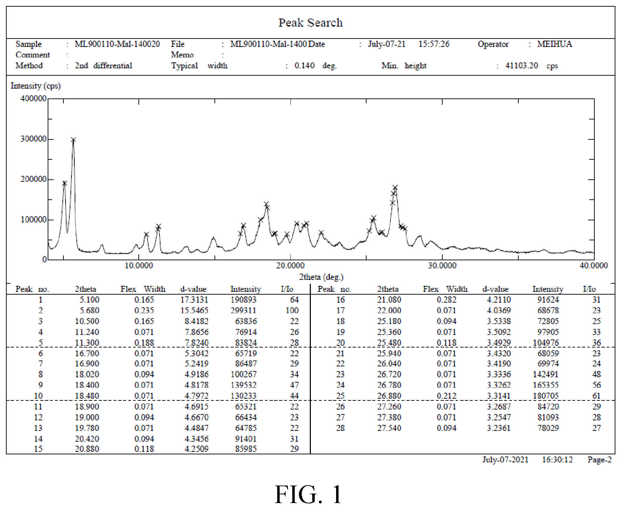

In one embodiment of the first aspect, the crystal form A of (E)-4-(dimethylamino)-N-(4-((4-(4-fluoro-phenoxy)phenyl)amino)-7-ethoxyquinazolin-6-yl)but-2-enamide maleate hydrate provided herein has a PXRD pattern substantially the same as that shown in FIG. 1.

In one embodiment of the first aspect, the present disclosure provides a crystal form A of (E)-4-(dimethylamino)-N-(4-((4-(4-fluoro-phenoxy)phenyl)amino)-7-ethoxyquinazolin-6-yl)but-2-enamide maleate hydrate, wherein the water content of the crystal form A is from 2.1 wt% to 10.0 wt%, or from 2.2 wt% to 9.2 wt%, or from 2.3 wt% to 9.1 wt%, or from 2.3 wt% to 8.0 wt%, or from 2.1 wt% to 3.1 wt%, or from 2.2 wt% to 2.8 wt%, or from 2.3 wt% to 2.6 wt%, or 2.3 wt%, or 2.4 wt%, or 2.5 wt%, or 2.6 wt%. In one embodiment, the crystal form A is a monohydrate.

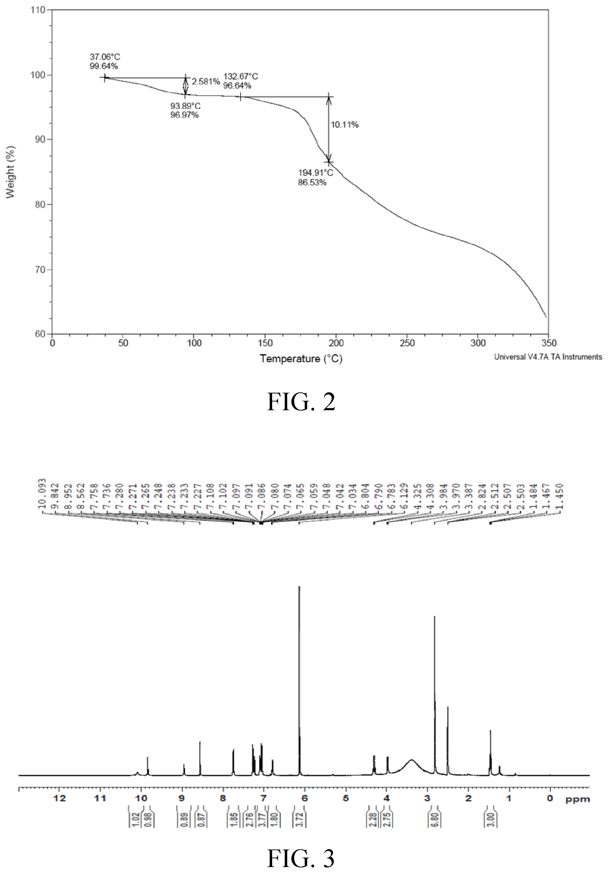

In one embodiment of the first aspect, the crystal form A of (E)-4-(dimethylamino)-N-(4-((4-(4-fluoro-phenoxy)phenyl)amino)-7-ethoxyquinazolin-6-yl)but-2-enamide maleate hydrate provided herein has a TGA diagram substantially the same as that shown in FIG. 2.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:2; and the maleate is an anhydrate.

In one embodiment of the first aspect, the present disclosure provides a crystal form B of (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form B is 1:2. Preferably, the crystal form B has a purity greater than 90%. In some embodiments, the PXRD pattern of the crystal form B comprises at least one of the characteristic peaks at 2θ values of about 5.1±0.2 degrees, 9.8±0.2 degrees, 10.1±0.2 degrees, 19.4±0.2 degrees, and 20.3±0.2 degrees. In some embodiments, the PXRD pattern of the crystal form B comprises characteristic peaks at 2θ values of about 5.1±0.2 degrees, 9.8±0.2 degrees, 10.1±0.2 degrees, 19.4±0.2 degrees, and 20.3±0.2 degrees. In some embodiments, the PXRD pattern of the crystal form B further comprises characteristic peaks at 2θ values of about 7.0±0.2 degrees, 18.4±0.2 degrees, 19.6±0.2 degrees, 22.3±0.2 degrees, and 25.1±0.2 degrees. In some embodiments, the PXRD pattern of the crystal form B comprises characteristic peaks at 2θ values of about 5.1±0.2 degrees, 7.0±0.2 degrees, 9.8±0.2 degrees, 10.1±0.2 degrees, 18.4±0.2 degrees, 19.4±0.2 degrees, 19.6±0.2 degrees, 20.3±0.2 degrees, 22.3±0.2 degrees, and 25.1±0.2 degrees. In one embodiment, the PXRD pattern of the crystal form B comprises the following characteristic peaks:

| **2θ** | **d-value** | **I/I₀** |
|---|---|---|
| 5.080 | 17.3812 | 100 |
| 7.040 | 12.5459 | 14 |
| 9.800 | 9.0179 | 28 |
| 10.120 | 8.7335 | 46 |
| 14.080 | 6.2848 | 11 |
| 16.520 | 5.3616 | 10 |
| 17.700 | 5.0068 | 12 |
| 17.800 | 4.9789 | 11 |
| 18.080 | 4.9024 | 11 |
| 18.400 | 4.8178 | 15 |
| 19.380 | 4.5764 | 27 |
| 19.620 | 4.5209 | 20 |
| 20.280 | 4.3753 | 35 |
| 20.720 | 4.2833 | 11 |
| 22.120 | 4.0153 | 14 |
| 22.280 | 3.9868 | 16 |
| 22.480 | 3.9518 | 15 |
| 22.560 | 3.9380 | 16 |
| 23.100 | 3.8471 | 15 |
| 23.420 | 3.7953 | 15 |
| 25.120 | 3.5421 | 17 |
| 25.740 | 3.4582 | 12 |
| 26.640 | 3.3434 | 12 |
| 29.600 | 3.0154 | 13 |
| 29.680 | 3.0075 | 12 |

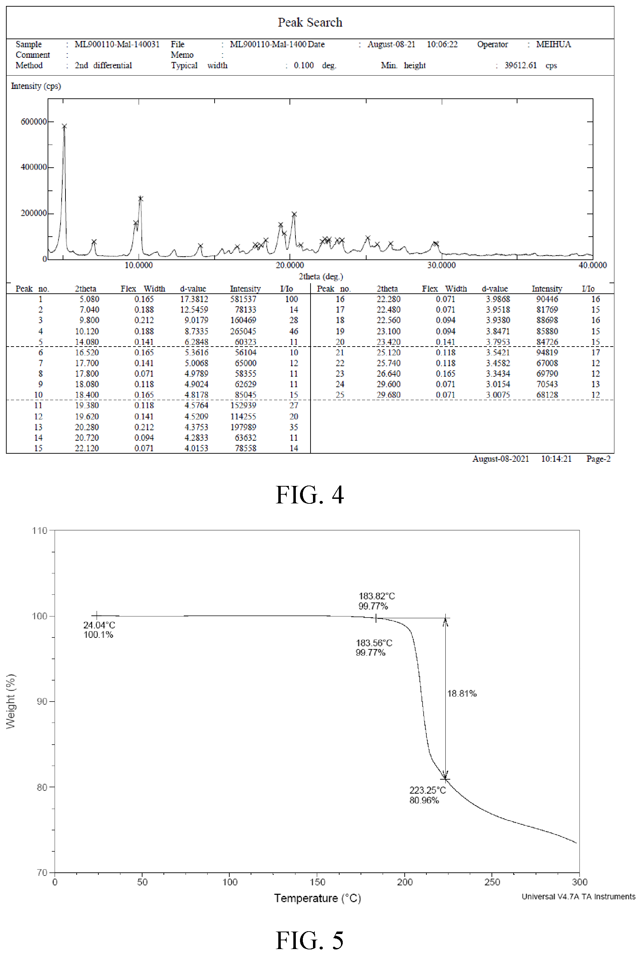

In one embodiment of the first aspect, the crystal form B of (E)-4-(dimethylamino)-N-(4-((4-(4-fluoro-phenoxy)phenyl)amino)-7-ethoxyquinazolin-6-yl)but-2-enamide maleate provided herein has a PXRD pattern substantially the same as that shown in FIG. 4.

In one embodiment of the first aspect, the present disclosure provides a crystal form B of (E)-4-(dimethylamino)-N-(4-((4-(4-fluoro-phenoxy)phenyl)amino)-7-ethoxyquinazolin-6-yl)but-2-enamide maleate, wherein the crystal form B is an anhydrate.

In one embodiment of the first aspect, the crystal form B of (E)-4-(dimethylamino)-N-(4-((4-(4-fluoro-phenoxy)phenyl)amino)-7-ethoxyquinazolin-6-yl)but-2-enamide maleate provided herein has a TGA thermogram substantially the same as that shown in FIG. 5.

In one embodiment of the first aspect, the present disclosure provides crystalline (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:1.

In one embodiment of the first aspect, the present disclosure provides a crystal form C of (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form C is 1:1. Preferably, the crystal form C has a purity greater than 90%. In some embodiments, the PXRD pattern of the crystal form C comprises at least one of the characteristic peaks at 2θ values of about 5.4±0.2 degrees, 5.8±0.2 degrees, 18.2±0.2 degrees, 18.7±0.2 degrees, 19.0±0.2 degrees, and 26.8±0.2 degrees. In some embodiments, the PXRD pattern of the crystal form C comprises characteristic peaks at 2θ values of about 5.4±0.2 degrees, 5.8±0.2 degrees, 18.2±0.2 degrees, 18.7±0.2 degrees, 19.0±0.2 degrees, and 26.8±0.2 degrees. In some embodiments, the PXRD pattern of the crystal form C further comprises characteristic peaks at 2θ values of about 6.1±0.2 degrees, 22.3±0.2 degrees, and 25.6±0.2 degrees. In some embodiments, the PXRD pattern of the crystal form C comprises characteristic peaks at 2θ values of about 5.4±0.2 degrees, 5.8±0.2 degrees, 6.1±0.2 degrees, 18.2±0.2 degrees, 18.7±0.2 degrees, 19.0±0.2 degrees, 22.3±0.2 degrees, 25.6±0.2 degrees, and 26.8±0.2 degrees. In some embodiments, the PXRD pattern of Form C comprises characteristic peaks at 2θ values of about 5.4±0.2 degrees, 5.8±0.2 degrees, 6.1±0.2 degrees, 18.2±0.2 degrees, 18.7±0.2 degrees, 19.0±0.2 degrees, 19.8±0.2 degrees, 21.5±0.2 degrees, 22.3±0.2 degrees, 22.8±0.2 degrees, 23.7±0.2 degrees, 25.6±0.2 degrees, and 26.8±0.2 degrees. In one embodiment, the PXRD pattern of Form C comprises the following characteristic peaks:

| **2θ** | **d-value** | **I/I₀** |
|---|---|---|
| 5.380 | 16.4127 | 81 |
| 5.760 | 15.3307 | 100 |
| 6.100 | 14.4770 | 74 |
| 18.200 | 4.8703 | 84 |
| 18.700 | 4.7412 | 100 |
| 19.020 | 4.6622 | 80 |
| 19.800 | 4.4802 | 63 |
| 21.460 | 4.1373 | 64 |
| 22.260 | 3.9904 | 70 |
| 22.840 | 3.8903 | 62 |
| 23.740 | 3.7448 | 64 |
| 25.620 | 3.4741 | 77 |
| 26.820 | 3.3214 | 81 |

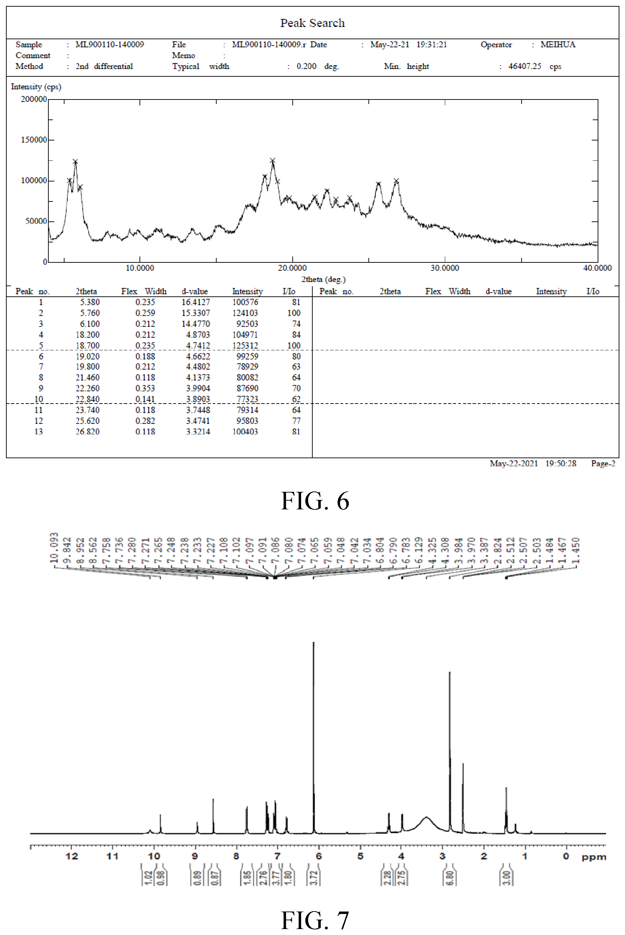

In one embodiment of the first aspect, the crystal form C of (E)-4-(dimethylamino)-N-(4-((4-(4-fluoro-phenoxy)phenyl)amino)-7-ethoxyquinazolin-6-yl)but-2-enamide maleate provided herein has a PXRD pattern substantially the same as that shown in FIG. 6.

In one embodiment of the first aspect, the (E)-4-(dimethylamino)-N-(4-((4-(4-fluoro-phenoxy)phenyl)amino)-7-ethoxyquinazolin-6-yl)but-2-enamide hydrochloride provided herein is crystalline, preferably, in crystal form S-1, wherein the crystal form S-1 of the hydrochloride has a purity greater than 90%. In some embodiments, the PXRD pattern of the crystal form S-1 comprises at least one of the characteristic peaks at 2θ values of about 6.1±0.2 degrees, 15.5±0.2 degrees, 18.1±0.2 degrees, 19.7±0.2 degrees, and 24.1±0.2 degrees. In some embodiments, the PXRD pattern of the Form S-1 comprises characteristic peaks at 2θ values of about 6.1±0.2 degrees, 15.5±0.2 degrees, 18.1±0.2 degrees, 19.7±0.2 degrees, and 24.1±0.2 degrees. In some embodiments, the PXRD pattern of the crystal form S-1 further comprises characteristic peaks at 2θ values of about 14.9±0.2 degrees, 22.6±0.2 degrees, 23.3±0.2 degrees, 25.6±0.2 degrees, and 26.0±0.2 degrees. In some embodiments, the PXRD pattern of the crystal form S-1 comprises characteristic peaks at 2θ values of about 6.1±0.2 degrees, 14.9±0.2 degrees, 15.5±0.2 degrees, 18.1±0.2 degrees, 19.7±0.2 degrees, 22.6±0.2 degrees, 23.3±0.2 degrees, 24.1±0.2 degrees, 25.6±0.2 degrees, and 26.0±0.2 degrees. In one embodiment, the PXRD pattern comprises the following characteristic peaks:

| **2θ** | **d-value** | **I/I₀** |
|---|---|---|
| 6.120 | 14.4297 | 100 |
| 14.920 | 5.9328 | 52 |
| 15.500 | 5.7121 | 60 |
| 18.140 | 4.8863 | 78 |
| 19.620 | 4.5209 | 69 |
| 19.740 | 4.4937 | 78 |
| 20.360 | 4.3582 | 48 |
| 20.480 | 4.3330 | 48 |
| 22.580 | 3.9345 | 59 |
| 23.300 | 3.8145 | 56 |
| 24.120 | 3.6867 | 67 |
| 24.980 | 3.5617 | 48 |
| 25.560 | 3.4822 | 54 |
| 26.020 | 3.4216 | 51 |
| 28.560 | 3.1228 | 46 |
| 28.700 | 3.1079 | 47 |

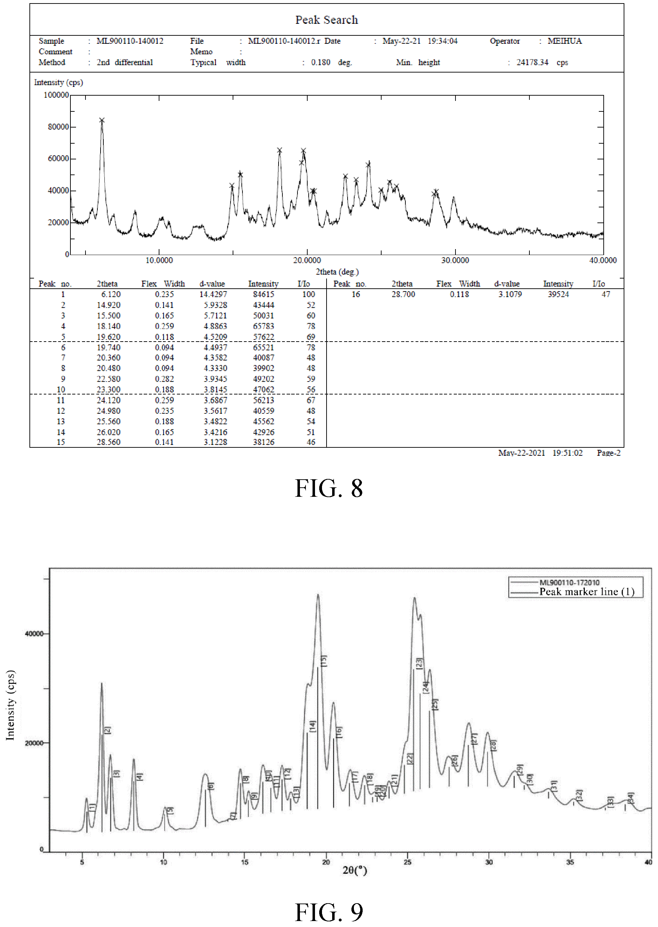

In one embodiment of the first aspect, the crystal form S-1 of (E)-4-(dimethylamino)-N-(4-((4-(4-fluoro-phenoxy)phenyl)amino)-7-ethoxyquinazolin-6-yl)but-2-enamide hydrochloride provided herein has a PXRD pattern substantially the same as that shown in FIG. 8.

In one embodiment of the first aspect, the present disclosure provides a crystal form S-2 of the (E)-4-(dimethylamino)-N-(4-((4-(4-fluoro-phenoxy)phenyl)amino)-7-ethoxyquinazolin-6-yl)but-2-enamide hydrochloride, wherein the crystal form S-2 of the hydrochloride has a purity greater than 90%. In some embodiments, the PXRD pattern of the crystal form S-2 comprises at least one of the characteristic peaks at 2θ values of about 18.8±0.2 degrees, 19.5±0.2 degrees, 20.4±0.2 degrees, 25.4±0.2 degrees, 25.8±0.2 degrees, and 26.3±0.2 degrees. In some embodiments, the PXRD pattern of the crystal form S-2 comprises characteristic peaks at 2θ values of about 18.8±0.2 degrees, 19.5±0.2 degrees, 20.4±0.2 degrees, 25.4±0.2 degrees, 25.8±0.2 degrees, and 26.3±0.2 degrees. , 20.4±0.2 degrees, 25.4±0.2 degrees, 25.8±0.2 degrees, and 26.3±0.2 degrees. In some embodiments, the PXRD pattern of the crystal form S-2 further comprises characteristic peaks at 2θ values of about 6.2±0.2 degrees, 12.6±0.2 degrees, 28.7±0.2 degrees, and 29.9±0.2 degrees. In some embodiments, the PXRD pattern of the crystal form S-2 comprises characteristic peaks at 2θ values of about 6.2±0.2 degrees, 12.6±0.2 degrees, 18.8±0.2 degrees, 19.5±0.2 degrees, 20.4±0.2 degrees, 25.4±0.2 degrees, 25.8±0.2 degrees, 26.3±0.2 degrees, 28.7±0.2 degrees, and 29.9±0.2 degrees. In one embodiment, the PXRD pattern comprises the following characteristic peaks:

| **2θ** | **d-value** | **I/I₀** |
|---|---|---|
| 5.277(11) | 16.73(3) | 5.57 |
| 6.202(3) | 14.240(8) | 37.36 |
| 6.751(4) | 13.084(7) | 20.98 |
| 8.169(4) | 10.815(5) | 17.98 |
| 10.068(11) | 8.778(10) | 8.45 |
| 12.568(8) | 7.038(4) | 25.72 |
| 13.96(4) | 6.34(2) | 0.63 |
| 14.718(3) | 6.0141(11) | 13.26 |
| 15.234(6) | 5.811(2) | 7.98 |
| 16.104(7) | 5.499(2) | 16.17 |
| 16.602(9) | 5.335(3) | 13.32 |
| 17.275(3) | 5.1292(9) | 13.91 |
| 17.833(7) | 4.970(2) | 4.82 |
| 18.825(4) | 4.7101(10) | 49.66 |
| 19.482(2) | 4.5528(5) | 100.00 |
| 20.4303(19) | 4.3435(4) | 44.35 |
| 21.418(5) | 4.1454(9) | 15.06 |
| 22.335(6) | 3.9772(11) | 8.98 |
| 22.84(6) | 3.891(10) | 1.44 |
| 23.126(18) | 3.843(3) | 1.03 |
| 23.852(6) | 3.7275(10) | 3.79 |
| 24.788(9) | 3.5890(12) | 15.04 |
| 25.363(8) | 3.5088(10) | 64.36 |
| 25.773(9) | 3.4539(11) | 45.86 |
| 26.326(7) | 3.3826(9) | 61.24 |
| 27.538(7) | 3.2364(8) | 16.73 |
| 28.729(3) | 3.1049(4) | 34.78 |
| 29.911(4) | 2.9849(4) | 25.73 |
| 31.56(3) | 2.832(2) | 7.89 |
| 32.17(7) | 2.780(6) | 2.16 |
| 33.662(17) | 2.6604(13) | 4.33 |
| 35.19(7) | 2.548(5) | 2.03 |
| 37.14(5) | 2.419(3) | 0.53 |
| 38.36(6) | 2.345(4) | 9.67 |

In one embodiment of the first aspect, the crystal form S-2 of (E)-4-(dimethylamino)-N-(4-((4-(4-fluoro-phenoxy)phenyl)amino)-7-ethoxyquinazolin-6-yl)but-2-enamide hydrochloride provided herein has a PXRD pattern substantially the same as that shown in FIG. 9.

In one embodiment of the first aspect, the (E)-4-(dimethylamino)-N-(4-((4-(4-fluoro-phenoxy)phenyl)amino)-7-ethoxyquinazolin-6-yl)but-2-enamide sulfate provided herein is crystalline, preferably, in a crystal form S-3, wherein the crystal form S-3 of the sulfate has a purity of greater than 90%. In some embodiments, the PXRD pattern of the crystal form S-3 comprises at least one of the characteristic peaks at 2θ values of about 5.2±0.2 degrees, 16.0±0.2 degrees, 25.4±0.2 degrees, and 26.1±0.2 degrees. In some embodiments, the PXRD pattern of the crystal form S-3 comprises characteristic peaks at 2θ values of about 5.2±0.2 degrees, 16.0±0.2 degrees, 25.4±0.2 degrees, and 26.1±0.2 degrees. In some embodiments, the PXRD pattern thereof further comprises characteristic peaks at 2θ values of about 17.7±0.2 degrees, 22.5±0.2 degrees, 24.5±0.2 degrees, 25.1±0.2 degrees, and 26.6±0.2 degrees; or, the PXRD pattern thereof comprises characteristic peaks at 2θ values of about 5.2±0.2 degrees, 16.0±0.2 degrees, 17.7±0.2 degrees, 22.5±0.2 degrees, 24.5±0.2 degrees, 25.1±0.2 degrees, 25.4±0.2 degrees, 26.1±0.2 degrees, and 26.6±0.2 degrees. In one embodiment, the PXRD pattern thereof comprises the following characteristic peaks:

| **2θ** | **d-value** | **I/I₀** |
|---|---|---|
| 5.200 | 16.9804 | 100 |
| 16.040 | 5.5210 | 29 |
| 17.700 | 5.0068 | 24 |
| 22.540 | 3.9414 | 24 |
| 24.540 | 3.6245 | 23 |
| 25.120 | 3.5421 | 25 |
| 25.400 | 3.5037 | 30 |
| 26.080 | 3.4139 | 30 |
| 26.160 | 3.4036 | 29 |
| 26.600 | 3.3483 | 22 |

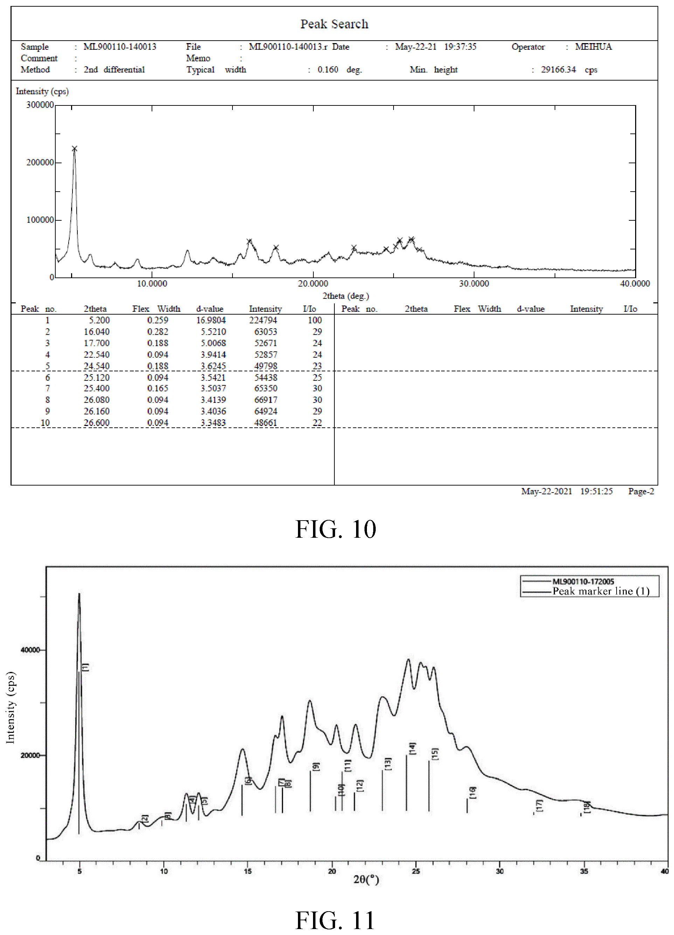

In one embodiment of the first aspect, the crystal form S-3 of (E)-4-(dimethylamino)-N-(4-((4-(4-fluoro-phenoxy)phenyl)amino)-7-ethoxyquinazolin-6-yl)but-2-enamide sulfate provided herein has a PXRD pattern substantially the same as that shown in FIG. 10.

In one embodiment of the first aspect, the (E)-4-(dimethylamino)-N-(4-((4-(4-fluoro-phenoxy)phenyl)amino)-7-ethoxyquinazolin-6-yl)but-2-enamide sulfate provided herein is in a crystal form S-4. Preferably, the crystal form S-4 of the sulfate has a purity greater than 90%. In some embodiments, the PXRD pattern of the crystal form S-4 comprises at least one of the characteristic peaks at 2θ values of about 5.0±0.2 degrees, 20.6±0.2 degrees, 24.4±0.2 degrees, and 25.8±0.2 degrees. In some embodiments, the PXRD pattern of the crystal form S-4 comprises characteristic peaks at 2θ values of about 5.0±0.2 degrees, 20.6±0.2 degrees, 24.4±0.2 degrees, and 25.8±0.2 degrees. In some embodiments, the PXRD pattern thereof further comprises characteristic peaks at 2θ values of about 14.7±0.2 degrees, 16.7±0.2 degrees, 18.7±0.2 degrees, and 23.0±0.2 degrees; or, the PXRD pattern thereof comprises characteristic peaks at 2θ values of about 5.0±0.2 degrees, 14.7±0.2 degrees, 16.7±0.2 degrees, 18.7±0.2 degrees, 20.6±0.2 degrees, 23.0±0.2 degrees, 24.4±0.2 degrees, and 25.8±0.2 degrees. In one embodiment, the PXRD pattern thereof comprises the following characteristic peaks:

| **2θ** | **d-value** | **I/I₀** |
|---|---|---|
| 4.975(3) | 17.748(12) | 13.50 |
| 8.522(16) | 10.367(19) | 0.39 |
| 9.88(4) | 8.94(3) | 0.81 |
| 11.333(11) | 7.801(8) | 1.20 |
| 12.051(12) | 7.338(8) | 1.10 |
| 14.67(2) | 6.033(9) | 4.35 |
| 16.65(3) | 5.320(8) | 3.84 |
| 17.03(2) | 5.201(7) | 1.51 |
| 18.693(13) | 4.743(3) | 7.24 |
| 20.216(14) | 4.389(3) | 0.80 |
| 20.604(19) | 4.307(4) | 100.00 |
| 21.323(10) | 4.164(2) | 1.29 |
| 22.989(16) | 3.866(3) | 8.11 |
| 24.42(2) | 3.642(3) | 14.72 |
| 25.77(3) | 3.455(4) | 17.49 |
| 28.030(18) | 3.181(2) | 2.22 |
| 31.99(5) | 2.796(4) | 0.15 |
| 34.81(5) | 2.575(3) | 0.32 |

In one embodiment of the first aspect, the crystal form S-4 of (E)-4-(dimethylamino)-N-(4-((4-(4-fluoro-phenoxy)phenyl)amino)-7-ethoxyquinazolin-6-yl)but-2-enamide sulfate provided herein has a PXRD pattern substantially the same as that shown in FIG. 11.

In one embodiment of the first aspect, the (E)-4-(dimethylamino)-N-(4-((4-(4-fluoro-phenoxy)phenyl)amino)-7-ethoxyquinazolin-6-yl)but-2-enamide sulfate provided herein is in the crystal form S-5. Preferably, the crystal form S-5 of the sulfate has a purity greater than 90%. In some embodiments, the PXRD pattern of the crystal form S-5 comprises at least one of the characteristic peaks at 2θ values of about 21.0±0.2 degrees, 22.7±0.2 degrees, 24.7±0.2 degrees, and 26.4±0.2 degrees. In some embodiments, the PXRD pattern of the crystal form S-5 comprises characteristic peaks at 2θ values of about 21.0±0.2 degrees, 22.7±0.2 degrees, 24.7±0.2 degrees, and 26.4±0.2 degrees. In some embodiments, the PXRD pattern thereof further comprises characteristic peaks at 2θ values of about 5.5±0.2 degrees, 18.9±0.2 degrees, 19.7±0.2 degrees, 22.3±0.2 degrees, and 23.3±0.2 degrees; or, the PXRD pattern thereof comprises characteristic peaks at 2θ values of about 5.5±0.2 degrees, 18.9±0.2 degrees, 19.7±0.2 degrees, 21.0±0.2 degrees, 22.3±0.2 degrees, 22.7±0.2 degrees, 23.3±0.2 degrees, 24.7±0.2 degrees, and 26.4±0.2 degrees. In one embodiment, the PXRD pattern comprises the following characteristic peaks:

| **2θ** | **d-value** | **I/I₀** |
|---|---|---|
| 4.72(3) | 18.71(11) | 0.48 |
| 5.503(3) | 16.048(8) | 7.08 |
| 6.99(3) | 12.64(5) | 1.05 |
| 9.34(4) | 9.46(4) | 0.29 |
| 10.75(2) | 8.226(17) | 0.90 |
| 12.959(18) | 6.826(10) | 2.79 |
| 13.996(19) | 6.323(9) | 0.89 |
| 15.112(15) | 5.858(6) | 2.05 |
| 16.041(7) | 5.521(2) | 0.81 |
| 16.476(13) | 5.376(4) | 0.36 |
| 18.32(2) | 4.838(6) | 0.30 |
| 18.880(5) | 4.6965(13) | 4.16 |
| 19.748(9) | 4.492(2) | 4.13 |
| 20.988(13) | 4.229(3) | 8.57 |
| 22.270(7) | 3.9887(13) | 6.13 |
| 22.66(2) | 3.921(4) | 100.00 |
| 23.343(9) | 3.8078(15) | 4.91 |
| 23.930(10) | 3.7156(16) | 2.11 |
| 24.745(12) | 3.5950(17) | 8.12 |
| 26.35(4) | 3.380(5) | 23.07 |
| 27.90(4) | 3.195(4) | 1.22 |
| 31.92(3) | 2.801(3) | 0.52 |
| 33.31(13) | 2.688(11) | 0.53 |
| 35.66(4) | 2.516(3) | 0.25 |

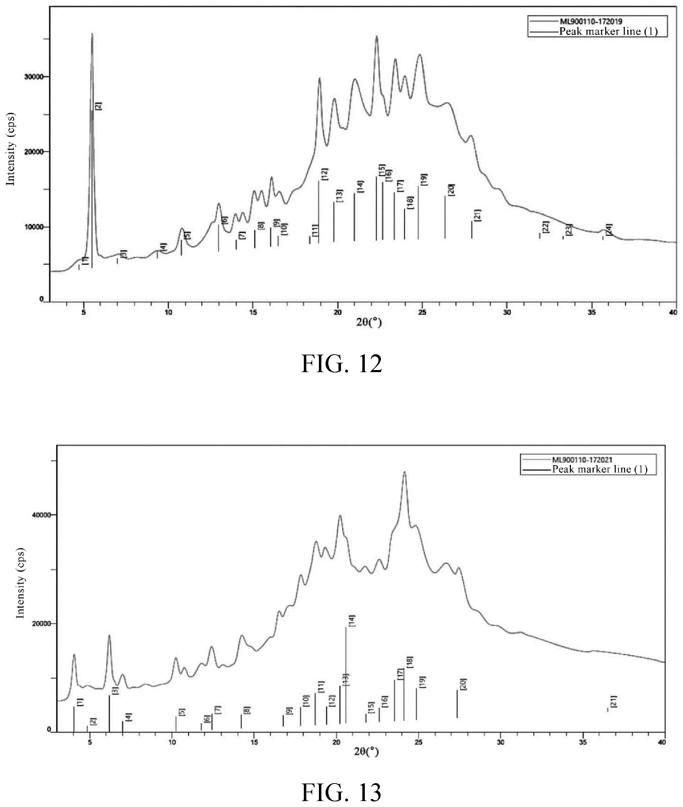

In one embodiment of the first aspect, the crystal form S-5 of (E)-4-(dimethylamino)-N-(4-((4-(4-fluoro-phenoxy)phenyl)amino)-7-ethoxyquinazolin-6-yl)but-2-enamide sulfate provided herein has a PXRD pattern substantially the same as that shown in FIG. 12.

In one embodiment of the first aspect, the (E)-4-(dimethylamino)-N-(4-((4-(4-fluoro-phenoxy)phenyl)amino)-7-ethoxyquinazolin-6-yl)but-2-enamide tartrate provided herein is crystalline; preferably, in crystal form S-6; and the crystal form S-6 of the tartrate has a purity greater than 90%. In some embodiments, the PXRD pattern of the crystal form S-6 comprises at least one of the characteristic peaks at 2θ values of about 20.2±0.2 degrees, 20.6±0.2 degrees, 23.5±0.2 degrees, and 27.4±0.2 degrees. In some embodiments, the PXRD pattern of the crystal form S-6 comprises characteristic peaks at 2θ values of about 20.2±0.2 degrees, 20.6±0.2 degrees, 23.5±0.2 degrees, and 27.4±0.2 degrees. In some embodiments, the PXRD pattern thereof further comprises characteristic peaks at 2θ values of about 18.7±0.2 degrees, 24.1±0.2 degrees, 24.9±0.2 degrees, and 36.5±0.2 degrees; or, the PXRD pattern thereof comprises characteristic peaks at 2θ values of about 18.7±0.2 degrees, 20.2±0.2 degrees, 20.6±0.2 degrees, 23.5±0.2 degrees, 24.1±0.2 degrees, 24.9±0.2 degrees, 27.4±0.2 degrees, and 36.5±0.2 degrees. In one embodiment, PXRD pattern thereof comprises the following characteristic peaks:

| **2θ** | **d-value** | **I/I₀** |
|---|---|---|
| 4.038(5) | 21.86(3) | 0.32 |
| 4.84(4) | 18.23(16) | 0.66 |
| 6.186(5) | 14.277(12) | 0.50 |
| 6.997(9) | 12.624(15) | 0.16 |
| 10.237(18) | 8.634(15) | 0.27 |
| 11.776(17) | 7.509(11) | 0.08 |
| 12.43(2) | 7.117(12) | 0.22 |
| 14.202(11) | 6.231(5) | 0.25 |
| 16.78(5) | 5.280(14) | 0.52 |
| 17.805(19) | 4.978(5) | 0.42 |
| 18.710(18) | 4.739(4) | 0.98 |
| 19.390(15) | 4.574(4) | 0.29 |
| 20.208(18) | 4.391(4) | 1.13 |
| 20.59(3) | 4.311(7) | 100 |
| 21.77(7) | 4.079(14) | 0.35 |
| 22.59(3) | 3.933(5) | 0.28 |
| 23.54(3) | 3.776(5) | 1.38 |
| 24.112(10) | 3.6879(16) | 1.09 |
| 24.869(16) | 3.577(2) | 0.86 |
| 27.359(12) | 3.2572(14) | 2.19 |
| 36.51(10) | 2.459(7) | 0.85 |

In one embodiment of the first aspect, the crystal form S-6 of (E)-4-(dimethylamino)-N-(4-((4-(4-fluoro-phenoxy)phenyl)amino)-7-ethoxyquinazolin-6-yl)but-2-enamide tartrate provided herein has a PXRD pattern substantially the same as that shown in FIG. 13.

In one embodiment of the first aspect, the (E)-4-(dimethylamino)-N-(4-((4-(4-fluoro-phenoxy)phenyl)amino)-7-ethoxyquinazolin-6-yl)but-2-enamide fumarate provided herein is crystalline; preferably, in crystal form S-7, wherein the crystal form S-7 of the fumarate has a purity greater than 90%. In some embodiments, the PXRD pattern of the crystal form S-7 comprises at least one of the characteristic peaks at 2θ values of about 6.9±0.2 degrees, 17.9±0.2 degrees, 22.7±0.2 degrees, 24.2±0.2 degrees, 25.4±0.2 degrees, and 25.7±0.2 degrees. In some embodiments, the PXRD pattern of the crystal form S-7 comprises characteristic peaks at 2θ values of about 6.9±0.2 degrees, 17.9±0.2 degrees, 22.7±0.2 degrees, 24.2±0.2 degrees, 25.4±0.2 degrees, and 25.7±0.2 degrees. In one embodiment, the PXRD pattern thereof further comprises characteristic peaks at 2θ values of about 8.8±0.2 degrees, 24.9±0.2 degrees, and 28.1±0.2 degrees. In one embodiment, the PXRD pattern thereof comprises characteristic peaks at 2θ values of about 6.9±0.2 degrees, 8.8±0.2 degrees, 17.9±0.2 degrees, 22.7±0.2 degrees, 24.2±0.2 degrees, 24.9±0.2 degrees, 25.4±0.2 degrees, 25.7±0.2 degrees, and 28.1±0.2 degrees. In one embodiment, the PXRD pattern thereof comprises the following characteristic peaks:

| **2θ** | **d-value** | **I/I₀** |
|---|---|---|
| 3.701(3) | 23.853(19) | 1.42 |
| 4.361(2) | 20.244(10) | 3.61 |
| 6.8914(15) | 12.816(3) | 9.02 |
| 7.504(3) | 11.771(4) | 1.54 |
| 7.931(2) | 11.138(3) | 1.08 |
| 8.820(2) | 10.017(2) | 5.37 |
| 10.203(18) | 8.662(16) | 0.13 |
| 10.902(11) | 8.109(8) | 1.29 |
| 11.804(8) | 7.491(5) | 2.79 |
| 12.3(2) | 7.17(13) | 0.80 |
| 13.52(4) | 6.54(2) | 1.01 |
| 13.812(7) | 6.406(3) | 1.66 |
| 15.280(9) | 5.794(3) | 0.99 |
| 16.086(10) | 5.505(3) | 1.13 |
| 16.421(11) | 5.394(4) | 2.48 |
| 16.971(16) | 5.220(5) | 1.12 |
| 17.870(6) | 4.9597(16) | 8.08 |
| 18.859(17) | 4.702(4) | 1.66 |
| 19.548(16) | 4.537(4) | 1.35 |
| 20.282(14) | 4.375(3) | 1.04 |
| 21.740(9) | 4.0846(16) | 4.57 |
| 22.708(9) | 3.9128(16) | 8.95 |
| 24.155(5) | 3.6815(8) | 12.14 |
| 24.885(4) | 3.5752(6) | 7.73 |
| 25.43(5) | 3.499(6) | 100.00 |
| 25.650(7) | 3.4702(9) | 19.37 |
| 28.078(12) | 3.1754(13) | 6.43 |
| 29.15(3) | 3.061(3) | 2.33 |
| 31.79(5) | 2.812(4) | 0.29 |
| 32.87(4) | 2.723(3) | 0.70 |
| 34.1(9) | 2.62(6) | 0.87 |
| 34.77(4) | 2.578(3) | 0.53 |
| 38.33(9) | 2.346(5) | 0.54 |

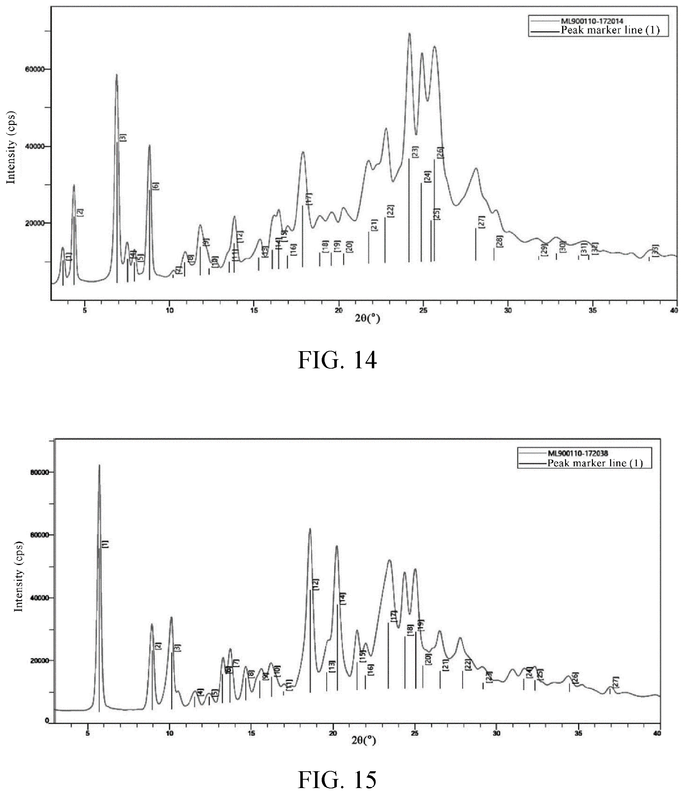

In one embodiment of the first aspect, the crystal form S-7 of (E)-4-(dimethylamino)-N-(4-((4-(4-fluoro-phenoxy)phenyl)amino)-7-ethoxyquinazolin-6-yl)but-2-enamide fumarate provided herein has a PXRD pattern substantially the same as that shown in FIG. 14.

In one embodiment of the first aspect, the (E)-4-(dimethylamino)-N-(4-((4-(4-fluoro-phenoxy)phenyl)amino)-7-ethoxyquinazolin-6-yl)but-2-enamide p-toluenesulfonate provided herein is crystalline; preferably, in crystal form S-8; wherein the crystal form S-8 of the p-toluenesulfonate has a purity greater than 90%. In some embodiments, the PXRD pattern of the crystal form S-8 comprises at least one of the characteristic peaks at 2θ values of about 5.7±0.2 degrees, 18.6±0.2 degrees, 20.2±0.2 degrees, 23.4±0.2 degrees, and 25.5±0.2 degrees. In some embodiments, the PXRD pattern of the crystal form S-8 comprises characteristic peaks at 2θ values of about 5.7±0.2 degrees, 18.6±0.2 degrees, 20.2±0.2 degrees, 23.4±0.2 degrees, and 25.5±0.2 degrees. In one embodiment, the PXRD pattern thereof further comprises characteristic peaks at 2θ values of about 8.9±0.2 degrees, 10.1±0.2 degrees, 24.4±0.2 degrees, 25.0±0.2 degrees, and 31.7±0.2 degrees. In one embodiment, the PXRD pattern thereof comprises characteristic peaks at 2θ values of about 5.7±0.2 degrees, 8.9±0.2 degrees, 10.1±0.2 degrees, 18.6±0.2 degrees, 20.2±0.2 degrees, 23.4±0.2 degrees, 24.4±0.2 degrees, 25.0±0.2 degrees, 25.5±0.2 degrees, and 31.7±0.2 degrees. In one embodiment, the PXRD pattern thereof comprises the following characteristic peaks:

| **2θ** | **d-value** | **I/I₀** |
|---|---|---|
| 5.7102(15) | 15.465(4) | 31.61 |
| 8.948(5) | 9.875(5) | 13.46 |
| 10.127(7) | 8.728(6) | 18.62 |
| 11.534(11) | 7.666(7) | 2.57 |
| 12.411(13) | 7.126(8) | 2.02 |
| 13.254(6) | 6.675(3) | 6.45 |
| 13.702(6) | 6.457(3) | 7.56 |
| 14.642(9) | 6.045(4) | 5.09 |
| 15.521(14) | 5.705(5) | 6.43 |
| 16.237(12) | 5.455(4) | 7.04 |
| 16.96(3) | 5.223(9) | 0.83 |
| 18.588(4) | 4.7695(10) | 36.66 |
| 19.590(9) | 4.528(2) | 5.82 |
| 20.231(4) | 4.3859(8) | 30.93 |
| 21.465(5) | 4.1363(10) | 5.68 |
| 21.949(11) | 4.046(2) | 3.72 |
| 23.373(8) | 3.8029(12) | 45.79 |
| 24.369(4) | 3.6496(6) | 14.02 |
| 25.014(5) | 3.5569(7) | 20.99 |
| 25.47(6) | 3.494(8) | 100.00 |
| 26.532(9) | 3.3569(11) | 5.01 |
| 27.905(11) | 3.1947(12) | 8.76 |
| 29.18(2) | 3.058(2) | 1.99 |
| 31.65(5) | 2.825(4) | 11.84 |
| 32.308(14) | 2.7687(12) | 4.09 |
| 34.45(2) | 2.6012(17) | 7.63 |
| 36.92(4) | 2.432(3) | 0.98 |

In one embodiment of the first aspect, the crystal form S-8 of (E)-4-(dimethylamino)-N-(4-((4-(4-fluoro-phenoxy)phenyl)amino)-7-ethoxyquinazolin-6-yl)but-2-enamide p-toluenesulfonate provided herein has a PXRD pattern substantially the same as that shown in FIG. 15.

In a second aspect, the present disclosure provides a method for preparing the acid addition salt of (E)-4-(dimethylamino)-N-(4-((4-(4-fluoro-phenoxy)phenyl)amino)-7-ethoxyquinazolin-6-yl)but-2-enamide, or solvate or hydrate thereof, according to the first aspect. The method comprises: mixing (E)-4-(dimethylamino)-N-(4-((4-(4-fluoro-phenoxy)phenyl)amino)-7-ethoxyquinazolin-6-yl)but-2-enamide (free base) and an acid in an organic solvent or a mixture of an organic solvent and water to react, and then performing solidliquid separation to obtain the acid addition salt of (E)-4-(dimethylamino)-N-(4-((4-(4-fluoro-phenoxy)phenyl)amino)-7-ethoxyquinazolin-6-yl)but-2-enamide, or solvate or hydrate thereof.

In some embodiments of the second aspect, the organic solvent is one or more selected from the group consisting of methanol, ethanol, n-propanol, isopropanol, ethyl acetate, dichloromethane and acetonitrile. Optionally, the organic solvent is anhydrous, or an aqueous mixture, such as 90% v/v ethanol.

In some embodiments of the second aspect, the present disclosure provides a method for preparing an acid addition salt of (E)-4-(dimethylamino)-N-(4-((4-(4-fluoro-phenoxy)phenyl)amino)-7-ethoxyquinazolin-6-yl)but-2-enamide, or solvate or hydrate thereof. The method comprises preparing (E)-4-(dimethylamino)-N-(4-((4-(4-fluoro-phenoxy)phenyl)amino)-7-ethoxyquinazolin-6-yl)but-2-enamide (free base) by the following steps:
(1) Reacting 7-fluoro-N⁴-(4-(4-fluorophenoxy)phenyl)-6-nitroquinazolin-4-amine with sodium ethoxide to obtain 7-ethoxy-N⁴-(4-(4-fluorophenoxy)phenyl)-6-nitroquinazolin-4-amine;
(2) subjecting 7-ethoxy-N⁴-(4-(4-fluorophenoxy)phenyl)-6-nitroquinazolin-4-amine to a reduction reaction to obtain 7-ethoxy-N⁴-(4-(4-fluorophenoxy)phenyl)-quinazoline-4,6-diamine;
(3) reacting 7-ethoxy-N⁴-(4-(4-fluorophenoxy)phenyl)quinazoline-4,6-diamine with diethylphosphonoacetic acid to obtain diethyl (2-((7-ethoxy-4-((4-(4-fluoro-phenoxy)phenyl)amino)quinazolin-6-yl)amino)-2-oxoethyl)phosphonate;
(4) reacting diethyl (2-((7-ethoxy-4-((4-(4-fluorophenoxy)phenyl)amino)-quinazolin-6-yl)amino)-2-oxoethyl)phosphonate with 2-dimethylaminoacetaldehyde to give (E)-4-(dimethylamino)-N-(4-((4-(4-fluoro-phenoxy)phenyl)amino)-7-ethoxy-quinazolin-6-yl)but-2-enamide (free base).

Furthermore, in step (1), a solution of sodium ethoxide in anhydrous ethanol may be added dropwise; and/or
in step (2), the reduction may be carried out by palladium/carbon catalyzed hydrogenation or iron powder with acetic acid; and/or
in step (3), 1,1'-carbonyldiimidazole may react with diethylphosphonoacetic acid first and then with 7-ethoxy-N ⁴ -(4-(4-fluorophenoxy)phenyl)quinazoline-4,6-diamine; and/or
in step (4), the temperature of the reaction system may be controlled from -10 to 0 °C; then the sodium bisulfite salt of 2-dimethylaminoacetaldehyde may be added dropwise; and after the reaction is completed, the product may be recrystallized from n-heptane.

In some embodiments of the second aspect, the present disclosure provides a method for preparing a crystal form F-1 of (E)-4-(dimethylamino)-N-(4-((4-(4-fluoro-phenoxy)phenyl)amino)-7-ethoxyquinazolin-6-yl)but-2-enamide (free base). The method comprises: mixing (E)-4-(dimethylamino)-N-(4-((4-(4-fluoro-phenoxy)phenyl)amino)-7-ethoxyquinazolin-6-yl)but-2-enamide (free base) with ethyl acetate; heating to reflux to dissolve the solids; cooling to room temperature to crystallize (i.e., standing crystallization); then stirring at room temperature for more than 1 hour; using suction filtration to obtained the solids; and drying the obtained product. Alternatively, mixing (E)-4-(dimethylamino)-N-(4-((4-(4-fluoro-phenoxy)phenyl)amino)-7-ethoxyquinazolin-6-yl)but-2-enamide (free base) with n-heptane/water, or methanol/water solvent system (e.g., a v/v ratio of 1:2); stirring at room temperature for 12-24 hours; using suction filtration to obtained the solids; and drying the obtained product.

In some embodiments of the second aspect, the present disclosure provides a method for preparing a crystal form F-2 of (E)-4-(dimethylamino)-N-(4-((4-(4-fluoro-phenoxy)phenyl)amino)-7-ethoxyquinazolin-6-yl)but-2-enamide (free base). The method comprises: mixing (E)-4-(dimethylamino)-N-(4-((4-(4-fluoro-phenoxy)phenyl)amino)-7-ethoxyquinazolin-6-yl)but-2-enamide (free base) with ethyl acetate; heating to reflux to dissolve the solids; then cooling to room temperature for crystallization (i.e., standing crystallization); using filtration to obtain the solid; and drying the obtained product.

In some embodiments of the second aspect, the present disclosure provides a method for preparing a crystal form F-3 of (E)-4-(dimethylamino)-N-(4-((4-(4-fluoro-phenoxy)phenyl)amino)-7-ethoxyquinazolin-6-yl)but-2-enamide (free base). The preparation method comprises: mixing (E)-4-(dimethylamino)-N-(4-((4-(4-fluoro-phenoxy)phenyl)amino)-7-ethoxyquinazolin-6-yl)but-2-enamide (free base) and an acetone-water solvent system (e.g., a v/v ratio of 3:2) or an ethanol-water solution (e.g., 80% v/v); hating to reflux to dissolve the solids; cooling to room temperature for crystallization (i.e., standing crystallization); stirring at room temperature for more than 1 hour, using suction filtration to obtain the solids; and drying the product. Alternatively, mixing (E)-4-(dimethylamino)-N-(4-((4-(4-fluoro-phenoxy)phenyl)amino)-7-ethoxyquinazolin-6-yl)but-2-enamide (free base) with ethyl acetate, acetonitrile, an acetone-water solvent system (e.g., a v/v ratio of 1:1), an ethanol-water solvent system (e.g., a v/v ratio of 1:1), or isopropanol; stirring at room temperature for 12-24 hours, using filtration to obtain the solids; and drying the product.

In some embodiments of the second aspect, the present disclosure provides a method for preparing a crystal form F-4 of (E)-4-(dimethylamino)-N-(4-((4-(4-fluoro-phenoxy)phenyl)amino)-7-ethoxyquinazolin-6-yl)but-2-enamide (free base). The method comprises: mixing (E)-4-(dimethylamino)-N-(4-((4-(4-fluoro-phenoxy)phenyl)amino)-7-ethoxyquinazolin-6-yl)but-2-enamide (free base) with acetonitrile or ethyl acetate-n-heptane solvent system (e.g., a v/v ratio of 1:1); heating to reflux to dissolve the solids; cooling to room temperature to crystallize (i.e., standing crystallization), stirring at room temperature for more than 1 hour; using suction filtration to obtain the solids; and drying the products.

In some embodiments of the second aspect, the present disclosure provides a method for preparing a crystal form F-5 of (E)-4-(dimethylamino)-N-(4-((4-(4-fluoro-phenoxy)phenyl)amino)-7-ethoxyquinazolin-6-yl)but-2-enamide (free base). The method comprises: mixing (E)-4-(dimethylamino)-N-(4-((4-(4-fluoro-phenoxy)phenyl)amino)-7-ethoxyquinazolin-6-yl)but-2-enamide (free base) with a methanol-water solvent system (e.g., a v/v ratio of 9:4); heating to reflux to dissolve the solids; cooling to room temperature for crystallization (i.e., standing crystallization); stirring at room temperature for more than 1 hour; using suction filtration to obtain the solids; and drying the product.

In some embodiments of the second aspect, the present disclosure provides a method for preparing a crystal form F-6 of (E)-4-(dimethylamino)-N-(4-((4-(4-fluoro-phenoxy)phenyl)amino)-7-ethoxyquinazolin-6-yl)but-2-enamide (free base). The method comprises: mixing (E)-4-(dimethylamino)-N-(4-((4-(4-fluoro-phenoxy)phenyl)amino)-7-ethoxyquinazolin-6-yl)but-2-enamide (free base) with isopropanol; heating to reflux to dissolve the solids; cooling to room temperature to crystallize (i.e., standing crystallization); stirring at room temperature for more than 1 hour; using suction filtration to obtain the solids; and drying the product.

In some embodiments of the second aspect, the present disclosure provides a method for preparing a crystal form A of (E)-4-(dimethylamino)-N-(4-((4-(4-fluoro-phenoxy)phenyl)amino)-7-ethoxyquinazolin-6-yl)but-2-enamide maleate. The method comprise: mixing (E)-4-(dimethylamino)-N-(4-((4-(4-fluoro-phenoxy)phenyl)amino)-7-ethoxyquinazolin-6-yl)but-2-enamide (free base) and maleic acid in acetone; stirring the mixture at room temperature for half an hour; using suction filtration to obtain the solids; and drying the product. Alternatively, stirring (E)-4-(dimethylamino)-N-(4-((4-(4-fluoro-phenoxy)phenyl)amino)-7-ethoxyquinazolin-6-yl)but-2-enamide (free base) and maleic acid in methanol or 90% ethanol at room temperature for half an hour; cooling the mixture to about -5 °C and standing for more than 2 hours or overnight; collecting the precipitated solid by filtration; and drying the product.

In some embodiments of the second aspect, the present disclosure provides a method for preparing a crystal form B of (E)-4-(dimethylamino)-N-(4-((4-(4-fluoro-phenoxy)phenyl)amino)-7-ethoxyquinazolin-6-yl)but-2-enamide maleate. The method comprises: stirring (E)-4-(dimethylamino)-N-(4-((4-(4-fluoro-phenoxy)phenyl)amino)-7-ethoxyquinazolin-6-yl)but-2-enamide (free base) and maleic acid in ethyl acetate at room temperature for half an hour; using suction filtration to obtain the solids; and drying the product.

In some embodiments of the second aspect, the present disclosure provides a method for preparing a crystal form C of (E)-4-(dimethylamino)-N-(4-((4-(4-fluoro-phenoxy)phenyl)amino)-7-ethoxyquinazolin-6-yl)but-2-enamide maleate. The method comprises: stirring (E)-4-(dimethylamino)-N-(4-((4-(4-fluoro-phenoxy)phenyl)amino)-7-ethoxyquinazolin-6-yl)but-2-enamide (free base) and maleic acid in anhydrous ethanol or acetonitrile at room temperature for half an hour; using suction filtration to obtain the solids; and drying the product.

In some embodiments of the second aspect, the present disclosure provides a method for preparing a crystal form S-1 of (E)-4-(dimethylamino)-N-(4-((4-(4-fluoro-phenoxy)phenyl)amino)-7-ethoxyquinazolin-6-yl)but-2-enamide hydrochloride. The method comprises: stirring (E)-4-(dimethylamino)-N-(4-((4-(4-fluoro-phenoxy)phenyl)amino)-7-ethoxyquinazolin-6-yl)but-2-enamide (free base) and hydrochloric acid in anhydrous ethanol at room temperature for half an hour; cooling to about -5 °C and standing for more than 2 hours or overnight; using suction filtration to obtain the solids; and drying the product.

In some embodiments of the second aspect, the present disclosure provides a method for preparing a crystal form S-3 of (E)-4-(dimethylamino)-N-(4-((4-(4-fluoro-phenoxy)phenyl)amino)-7-ethoxyquinazolin-6-yl)but-2-enamide sulfate. The method comprises: stirring (E)-4-(dimethylamino)-N-(4-((4-(4-fluoro-phenoxy)phenyl)amino)-7-ethoxyquinazolin-6-yl)but-2-enamide (free base) and sulfuric acid in anhydrous ethanol at room temperature for half an hour; using suction filtration to obtain the solids; and drying the product.

In some embodiments of the second aspect, the present disclosure provides a method for preparing a crystal form S-6 of (E)-4-(dimethylamino)-N-(4-((4-(4-fluoro-phenoxy)phenyl)amino)-7-ethoxyquinazolin-6-yl)but-2-enamide tartrate. The method comprising: mixing (E)-4-(dimethylamino)-N-(4-((4-(4-fluoro-phenoxy)phenyl)amino)-7-ethoxyquinazolin-6-yl)but-2-enamide (free base) and tartaric acid in an acetone-water solvent system (e.g., a v/v ratio of 4:1), anhydrous ethanol-water solvent system (e.g., a v/v ratio of 3:1), an isopropanol-water solvent system (e.g., a v/v ratio of 3:1), or an acetonitrile-water solvent system (e.g., a v/v ratio of 12:1); stirring the mixture at room temperature for half an hour; using suction filtration to obtain the solids; and drying the product. Alternatively, stirring (E)-4-(dimethylamino)-N-(4-((4-(4-fluoro-phenoxy)phenyl)amino)-7-ethoxyquinazolin-6-yl)but-2-enamide (free base) and tartaric acid in 90% ethanol at room temperature for half an hour; cooling to about -5 °C and standing for more than 2 hours or overnight; using suction filtration to obtain the solids; and drying the product.

In some embodiments of the second aspect, the present disclosure provides a method for preparing crystal form S-7 of (E)-4-(dimethylamino)-N-(4-((4-(4-fluoro-phenoxy)phenyl)amino)-7-ethoxyquinazolin-6-yl)but-2-enamide fumarate. The method comprising: stirring (E)-4-(dimethylamino)-N-(4-((4-(4-fluoro-phenoxy)phenyl)amino)-7-ethoxyquinazolin-6-yl)but-2-enamide (free base) and fumaric acid are in methanol at room temperature for half an hour; cooling to about -5°C and standing for more than 2 hours or overnight; using suction filtration to obtain the solids; and drying the product.

In some embodiments of the second aspect, the present disclosure provides a method for preparing a crystal form S-8 of (E)-4-(dimethylamino)-N-(4-((4-(4-fluoro-phenoxy)phenyl)amino)-7-ethoxyquinazolin-6-yl)but-2-enamide p-toluenesulfonate. The method comprising: stirring (E)-4-(dimethylamino)-N-(4-((4-(4-fluoro-phenoxy)phenyl)amino)-7-ethoxyquinazolin-6-yl)but-2-enamide (free base) and p-toluenesulfonic acid in acetonitrile at room temperature for half an hour; cooling to about -5°C and standing for more than 2 hours or overnight; using suction filtration to obtain the solids; and drying the product.

In a third aspect, the present disclosure provides a pharmaceutical composition comprising the (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, according to the first aspect.

In some embodiments of the third aspect, the pharmaceutical composition may further comprise an excipient.

In some embodiments of the third aspect, the pharmaceutical composition is a solid oral dosage form.

In some embodiments of the third aspect, the unit dose of the pharmaceutical composition is from 25 mg to 700 mg, or from 35 mg to 500 mg, or from 40 mg to 400 mg, or from 50 mg to 350 mg, or from 50 mg to 250 mg; or the unit dose is 30 mg, 31 mg, 32 mg, 33 mg, 34 mg, 35 mg, 36 mg, 37 mg, 38 mg, 39 mg, 40 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, or 450 mg, wherein the weight is calculated based on (E)-4-(dimethylamino)-N-(4-((4-(4-fluoro-phenoxy)phenyl)amino)-7-ethoxyquinazolin-6-yl)but-2-enamide.

In some embodiments of the third aspect, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide (free base), or solvate or hydrate thereof. In some embodiments, the (E)-4-(dimethylamino)-N-(4-((4-(4-fluoro-phenoxy)phenyl)amino)-7-ethoxyquinazolin-6-yl)but-2-enamide (free base) is an amorphous solid form. In some embodiments, the (E)-4-(dimethylamino)-N-(4-((4-(4-fluoro-phenoxy)phenyl)amino)-7-ethoxyquinazolin-6-yl)but-2-enamide (free base) is a crystalline solid form; in essence, comprising one or a mixture of crystal forms F-1 to F-6.

In some embodiments of the third aspect, the present disclosure provides a pharmaceutical composition comprising an acid addition salt of (E)-4-(dimethylamino)-N-(4-((4-(4-fluoro-phenoxy)phenyl)amino)-7-ethoxyquinazolin-6-yl)but-2-enoamide, or solvate or hydrate thereof; wherein the acid in the acid addition salt is hydrochloric acid, sulfuric acid, nitric acid, phosphoric acid, acetic acid, propionic acid, glycolic acid, oxalic acid, maleic acid, fumaric acid, malonic acid, succinic acid, tartaric acid, citric acid, mandelic acid, methanesulfonic acid, ethanesulfonic acid, benzenesulfonic acid, p-toluenesulfonic acid, trifluoroacetic acid, 1-hydroxy-2-naphthoic acid, or benzoic acid.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide hydrochloride, or solvate or hydrate thereof. In some examples, the (E)-4-(dimethylamino)-N-(4-((4-(4-fluoro-phenoxy)phenyl)amino)-7-ethoxyquinazolin-6-yl)but-2-enamide hydrochloride, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide hydrochloride, or the solvate or hydrate thereof, is crystal form S-1, or crystal form S-2, or a mixture thereof.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide maleate, or solvate or hydrate thereof. In some embodiments, the (E)-4-(dimethylamino)-N-(4-((4-(4-fluoro-phenoxy)phenyl)amino)-7-ethoxyquinazolin-6-yl)but-2-enamide maleate, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide maleate, or the solvate or hydrate thereof, is crystal form A, crystal form B, or crystal form C, or a mixture thereof.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide sulfate, or solvate or hydrate thereof. In some embodiments, the (E)-4-(dimethylamino)-N-(4-((4-(4-fluoro-phenoxy)phenyl)amino)-7-ethoxyquinazolin-6-yl)but-2-enamide sulfate, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide sulfate, or the solvate or hydrate thereof, is crystal form S-3, crystal form S-4, or crystal form S-5, or a mixture thereof.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide tartrate, or solvate or hydrate thereof. In some embodiments, the (E)-4-(dimethylamino)-N-(4-((4-(4-fluoro-phenoxy)phenyl)amino)-7-ethoxyquinazolin-6-yl)but-2-enamide tartrate, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide tartrate, or the solvate or hydrate thereof, is crystal form S-6.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide fumarate, or solvate or hydrate thereof. In some embodiments, the (E)-4-(dimethylamino)-N-(4-((4-(4-fluoro-phenoxy)phenyl)amino)-7-ethoxyquinazolin-6-yl)but-2-enamide fumarate, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide fumarate, or the solvate or hydrate thereof, is crystal form S-7.

In some embodiments, the present disclosure provides a pharmaceutical composition comprising (E)-4-(dimethylamino)-N-(4-((4-(4-fluoro-phenoxy)phenyl)amino)-7-ethoxyquinazolin-6-yl)but-2-enamide p-toluenesulfonate, or solvate or hydrate thereof. In some examples, the (E)-4-(dimethylamino)-N-(4-((4-(4-fluoro-phenoxy)phenyl)amino)-7-ethoxyquinazolin-6-yl)but-2-enamide p-toluenesulfonate, or the solvate or hydrate thereof, can be a crystalline solid form or an amorphous solid form. In some embodiments, the (E)-4-(dimethylamino)-N-(4-((4-(4-fluoro-phenoxy)phenyl)amino)-7-ethoxyquinazolin-6-yl)but-2-enamide p-toluenesulfonate, or the solvate or hydrate thereof, is crystal form S-8.

In a fourth aspect, the present disclosure provides the use of the (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, of the first aspect, or the pharmaceutical composition of the third aspect, in the preparation of a medicament for preventing, inhibiting or treating cancer.

In a fifth aspect, the present disclosure provides the use of the (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, of the first aspect, or the pharmaceutical composition of the third aspect, as a medicament.

In a sixth aspect, the present disclosure provides the use of the (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, of the first aspect, or the pharmaceutical composition of the third aspect, in preventing, inhibiting or treating cancer.

In a seventh aspect, the present disclosure provides a method for preventing, inhibiting or treating cancer, the method comprising: administering a therapeutically effective amount of the (E)-4-(dimethylamino)-N-(4-((4-(4-fluoro-phenoxy)phenyl)amino)-7-ethoxyquinazolin-6-yl)but-2-enoamide acid, acid addition salt thereof, or solvate or hydrate thereof, of the first aspect, or the pharmaceutical composition of the third aspect, to a subject in need thereof.

In the embodiments of the fourth, sixth, and seventh aspects of the present disclosure, the cancer is a HER2-positive cancer; particularly, a HER2-positive advanced cancer. The HER2-positive includes, but is not limited to, one or more of HER2 protein overexpression, HER2 gene amplification, or HER2 gene mutation(s). Particularly, the cancer is one or more of breast cancer, ovarian cancer, epithelial cancer, colorectal cancer, renal cancer, bladder cancer, laryngeal cancer, esophageal cancer, salivary gland cancer, submandibular cancer, gastric cancer, or lung cancer; more particularly, breast cancer, ovarian cancer, gastric cancer, and/or colorectal cancer; even more particularly, cancer with brain metastasis, including but not limited to, breast cancer with brain metastasis, ovarian cancer with brain metastasis, gastric cancer with brain metastasis, and/or colorectal cancer with brain metastasis.

In the embodiments of the fourth, sixth, and seventh aspects of the present disclosure, regarding the (E)-4-(dimethylamino)-N-(4-((4-(4-fluoro-phenoxy)phenyl)amino)-7-ethoxyquinazolin-6-yl)but-2-enoamide, acid addition salt thereof, or solvate or hydrate thereof, of the first aspect, or the pharmaceutical composition of the third aspect, in some embodiments, an administration mode of the present disclosure is a unit dosage form, the unit dose is from 25 mg to 700 mg, or from 35 mg to 500 mg, or from 40 mg to 400 mg, or from 50 mg to 350 mg, or from 50 mg to 250 mg; or the unit dose is 30 mg, 31 mg, 32 mg, 33 mg, 34 mg, 35 mg, 36 mg, 37 mg, 38 mg, 39 mg, 40 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 195 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, or 450 mg, wherein the weight is calculated based on (E)-4-(dimethylamino)-N-(4-((4-(4-fluoro-phenoxy)phenyl)amino)-7-ethoxyquinazolin-6-yl)but-2-enamide. Suitable unit dosage forms comprise tablets in a sachet or a bottle, capsules, and powders. In one embodiment, the (E)-4-(dimethylamino)-N-(4-((4-(4-fluoro-phenoxy)phenyl)amino)-7-ethoxyquinazolin-6-yl)but-2-enamide, acid addition salt thereof, or solvate or hydrate thereof, or pharmaceutical composition thereof of the present disclosure can be administered orally; may be administered from 1 to 6 times daily, more usually from 1 to 4 times daily, or once daily. The therapeutically effective amount can be known to those skilled in the art. The therapeutically effective amount may also depend on the form of the compound, the mode of administration, and the severity of the condition being treated. The therapeutically effective amount may be 0.01-500 mg/kg, preferably 0.01-50 mg/kg, more preferably 0.1-10 mg/kg. The number of administrations may be 1 to 4 times daily. For example, satisfactory results may be obtained when the compounds of the present disclosure is administered in the range of about 0.5 mg/kg to about 10 mg/kg body weight daily; and generally an effective dose is from about 0.5 mg/kg/day to about 8 mg/kg/day.

### Definitions

Unless expressly indicated otherwise, the term "approximately" is synonymous with "about." When used with respect to a recited amount, value, or duration, the term "approximately" or "about" refers to ±10%, ±8%, ±6%, ±5%, ±4%, or ±2% of the recited amount, value, or duration. In one embodiment, "approximately" and "about" refer to ±5% of the recited amount, value, or duration. In another embodiment, "approximately" and "about" refer to ±2% of the recited amount, value, or duration. When the term "approximately" or "about" is used with respect to recited PXRD peaks, the term refers to the recited PXRD at 2θ±0.3°, 2θ±0.2°, or 2θ±0.1°. In one embodiment, the term "approximately" or "about" refers to the recited PXRD peaks at 2θ±0.2°. In another embodiment, the term "approximately" or "about" refers to the recited PXRD peaks at 2θ±0.1°. When the terms "approximately" or "about" are used when reciting a temperature or a temperature range, these terms refer to the recited temperature or temperature range ±5°C, ±2°C, or ±1°C. In one embodiment, the terms "approximately" or "about" refer to the recited temperature or temperature range ±2°C. In another embodiment, the terms "approximately" or "about" refer to the recited temperature or temperature range ±1°C.

As used herein, the term "acid addition salt" refers to a pharmaceutically acceptable salt of an amine-type compound that is suitable for pharmaceutical use, preferably for use in humans and animals without undue irritation, allergic response and the like. For example, S. M. Berge et al. describe pharmaceutically acceptable salts in detail in J. Pharmaceutical Sciences, 66: 1-19 (1977), the content of which is incorporated herein by reference. As generally described herein, the salts can be prepared in situ during the final isolation and purification step of the trisubstituted quinazoline derivatives of the present disclosure, or separately by reacting the free base of the compound with a suitable reagent. For example, the type of acid addition salt includes, but is not limited to, acid addition salts formed by reacting the free base form of the compound with a pharmaceutically acceptable acid: for example, an inorganic acid such as hydrochloric acid, hydrobromic acid, sulfuric acid, nitric acid, and phosphoric acid, or an organic acid such as acetic acid, propionic acid, glycolic acid, oxalic acid, pyruvic acid, lactic acid, malonic acid, succinic acid, malic acid, maleic acid, fumaric acid, tartaric acid, citric acid, mandelic acid, methanesulfonic acid, ethanesulfonic acid, benzenesulfonic acid, p-toluenesulfonic acid, trifluoroacetic acid, 1-hydroxy-2-naphthoic acid, benzoic acid, gluconic acid, glutamic acid, hydroxynaphthoic acid, or salicylic acid and the like. In one embodiment, the salt precipitates and crystallizes (i.e., if crystallizable) after cooling (either slowly or rapidly). In addition, the present disclosure also includes hemi-salts, mono-salts, di-salts, tri-salts, and poly-salts of the compounds of the present disclosure. Similarly, the present disclosure also includes the compound, salt thereof, or hemihydrate, monohydrate, dihydrate, trihydrate, or polyhydrate thereof. In some embodiments, the acid addition salts of the compound include, but are not limited to, hydrochloride, hydrobromide, sulfate, nitrate, phosphate or metaphosphate, acetate, propionate, hexanoate, 3-cyclopentylpropionate, glycolate, pyruvate, lactate, malonate, succinate, malate, maleate, oxalate, fumarate, trifluoroacetate, tartrate, citrate, fumarate, benzoate, 3-(4-hydroxybenzoyl)benzoate, cinnamate, mandelate, methanesulfonate, ethanesulfonate, 1,2-ethanedisulfonate, 2-hydroxyethanesulfonate, benzenesulfonate, p-toluenesulfonate, 2-naphthalenesulfonate, 4-methylbicyclo-[2.2.2]oct-2-ene-1-carboxylate, glucoheptonate, 4,4'-methylenebis-(3-hydroxy-2-ene-1-carboxylate), 3-phenylpropionate, pivalate, tert-butylacetate, lauryl sulfate, gluconate, glutamate, hydroxynaphthoate, salicylate, stearate, muconate, butyrate, phenylacetate, phenylbutyrate, valproate and the like. In a preferred embodiment, the compound is a hydrochloride, sulfate, maleate, tartrate, fumarate, or p-toluenesulfonate, or hydrate thereof, such as monohydrate.

As used herein, the term "crystalline" means having a regular, repeating pattern of molecules or external surface planes. Crystal forms may differ in thermodynamic stability, physical parameters, x-ray structure, and method of preparation.

As used herein, the term "anhydrous" refers to a crystal form that does not contain water molecules in a crystalline lattice.

The term "hydrate" or "solvate" refers to a crystal form of a compound bound with water or other solvent molecules. These solvent molecules (e.g., water or organic solvents) are physically or chemically bound to the host compound and become part of the crystalline lattice.

As used herein, the term "substantially the same" refers to the typical variability considered for a particular method. For example, with reference to X-ray diffraction peak positions, the term "substantially the same" refers to the typical variability of peak positions and intensities. Those skilled in the art understand that peak positions (2θ) may show some variability, typically up to ±0.2 degrees. In addition, those skilled in the art understand that relative peak intensities may show variability between devices, as well as variability due to crystallinity, preferred orientation, surfaces of the prepared sample, and other factors known to those skilled in the art, thus, may be considered as qualitative measurements. Substantially identical peak shape, peak position, and peak intensity are equivalent to the same crystal form, even though there are differences in some details of the X-ray diffraction pattern, which may be caused by the sample preparation process and/or testing conditions.

The terms "crystal form" or "crystalline modification" or "polymorphic form" or "polymorph" refer to the phenomenon in which the same chemical substance exists in different crystal structures. These different crystal structures may differ in molecular arrangement, lattice parameters and physicochemical properties, but have the same chemical composition.

As used herein, the term "amorphous" refers to the non-crystalline solid state form of a matter. Amorphous solids may be composed of a disordered arrangement of molecules and have no distinguishable crystal lattice.

As used herein, the term "substantially pure" with respect to a crystal form means that the crystal form of the compound comprises at least 90 wt%, preferably at least 95 wt%, more preferably at least 97 wt%, and most preferably at least 99 wt% of the specified crystal form (e.g., crystal form A). Alternatively, it can be understood that "substantially phase pure" means that the crystal form contains less than 10 wt%, preferably less than 5 wt%, more preferably less than 3 wt%, and most preferably less than 1 wt% of substance impurities, such as other polymorphs, solvates, or amorphous forms.

As used herein, "substantially pure" includes "substantially phase pure" and/or "substantially chemically pure", wherein "substantially phase pure" refers to the purity relative to other solid forms of the compound and does not necessarily mean a high degree of chemical purity relative to other compounds; "substantially chemically pure" refers to a substance with extremely high purity and extremely low impurity content, which can generally meet the requirements of most chemical experiments or industrial applications. Although it may not be absolutely 100% pure, its purity is sufficient to ensure that it does not significantly affect the results in a specific application.

As used herein, "seed crystals" refer to small crystals introduced during the crystallization process to promote the crystallization of the target substance. They act as templates, guiding the orderly arrangement of molecules or ions from a solution or melt to form larger crystals.

As used herein, the term "therapeutically effective amount" refers to an amount of a compound administered that alleviates, to some extent, one or more symptoms of the disorder being treated. With reference to the treatment of cancer, a therapeutically effective amount refers to an amount that: (1) reduces tumor size, (2) inhibits (i.e., slows to some extent, preferably stops) tumor metastasis, (3) inhibits (i.e., slows to some extent, preferably stops), to some extent, tumor growth or tumor invasion, and/or (4) alleviates (or preferably eliminates), to some extent, one or more signs or symptoms associated with cancer.

As used herein, the terms "individual" or "subject" refer to an animal used in experiment or research; preferably, the animal is a mammal; the "individual" or "subject" may be a primate (e.g., human), cow, sheep, goat, horse, dog, cat, rabbit, rat, mouse, fish, bird and the like. In one embodiment, the "individual" or "subject" is a human.

As used herein, the terms "a," "an," and "the," and any similar referents, in the context of the claims and the specification, should be understood to include both the singular reference and the plural reference, unless explicitly stated otherwise or the context clearly indicates a singular meaning.

As used herein, the term "treat" refers to the application or administration of a therapeutic agent to a patient, or to a tissue or cell line isolated from the patient, wherein the patient has a disease, a symptom of the disease, or a predisposition to the disease, with the intention of curing, recovering, alleviating, ameliorating, altering, reducing, correcting, improving, or otherwise affecting the disease, the symptom of a disease, or the predisposition to the disease.

As used herein, the term "preventing" any disease or condition refers to delaying the onset or development or progression of the disease or condition.

As used herein, the terms "include" or "comprising" and "composed of" refer to "including".

As used herein, the terms "pharmaceutical composition" or "composition product" or "combination product" refer to a pharmaceutical composition that can be administered to a patient in need of treatment, which can be in any conventional formulation (for example, in the form of powder, granules, pills, capsules, lozenges, solutions, suspensions or patches, etc.).

The terms "co-administration" or "combination administration", "combined administration", "combination therapy" or "combined therapy" refer to the administration of E)-4-(dimethylamino)-N-(4-((4-(4-fluoro-phenoxy)phenyl)amino)-7-ethoxyquinazolin-6-yl)but-2-enamide, an acid addition salt thereof, or solvate or hydrate thereof, or a pharmaceutical composition thereof, together with one or more therapeutic agents as part of a specific treatment regimen intended to provide a beneficial effect from the combined action of these therapeutic agents. The combined administration of these therapeutic agents is generally carried out within a defined time period (usually minutes, hours, days, or weeks, depending on the combination selected). "Combination therapy" or "combined therapy" is intended to include sequential administration of these therapeutic agents, i.e., each therapeutic agent is administered at a different time, as well as administration of these therapeutic agents or at least two therapeutic agents in a substantially simultaneous manner. For example, substantially simultaneous administration can be achieved by administering to the subject a single dose having a fixed ratio of each or more therapeutic agents or multiple separate doses of each therapeutic agent. Sequential or substantially simultaneous administration of each therapeutic agent is achieved by any suitable route (including but not limited to, oral, intravenous, intramuscular, and direct absorption through mucosal tissues). The therapeutic agents can be administered by the same route or by different routes. The order in which the therapeutic agents are administered is not strictly critical.

### Examples

### 1. Powder X-ray Diffraction (PXRD, or XRD)

Instrument name: X-ray powder diffractometer
Instrument Model: Ultima IV/Miniflex
Merchant: Rigaku
Detector: D/tex high energy and high speed detector.

### Testing conditions:

X-ray: Cu/40KV/40mA
Wavelength: 0.15406 nm
Scanning speed: 60.000 degrees/min/10.000 degrees/min.
Sampling width: 0.0200 degrees/0.0200 degrees
Scanning range: 4.0000 to 60.0000 degrees / 3.0000 to 60.0000 degrees.

### 2. Thermogravimetric analysis (TGA)

### TGA experimental instrument: American TA Q5000

Test method: heating from room temperature to 300.00 °C at 10.00 °C/min; or heating from room temperature to 300.00 °C at 5.00 °C/min.

### 3. Nuclear Magnetic Resonance Analysis (NMR)

NMR instrument: Superconducting high-resolution nuclear magnetic resonance spectrometer Bruke AVANCE II 400MHz.

### 4. LC-MS

LC-MS instrument: Agilent 6120 Single Quadrupole LC-MS instrument.

### Example 1. Preparation of (E)-4-(dimethylamino)-N-(4-((4-(4-fluoro-phenoxy)phenyl)-amino)-7-ethoxyquinazolin-6-yl)but-2-enamide

A 30 L reactor was charged with 7-fluoro-N⁴-(4-(4-fluorophenoxy)phenyl)-6-nitroquinazolin-4-amine (980 g) and anhydrous ethanol (9.8 L). The mixture was cooled to 5-10 °C, and a 21% sodium ethoxide solution in anhydrous ethanol (1611 g) was slowly added dropwise over 1 hour. The mixture was heated to 60-70 °C and allowed to react for 16 hours. The reaction solution was cooled and slowly poured into a mixture of dichloromethane and water (20L/20L), and the layers were separated. The separated aqueous phase was extracted with dichloromethane (13L), and the combined organic phases were washed with water (13L), and separated. The organic phase was then dried over anhydrous sodium sulfate, evaporated to dryness under reduced pressure, and then slurried with n-heptane, filtered, and dried to obtain 7-ethoxy-N⁴-(4-(4-fluorophenoxy)phenyl)-6-nitroquinazolin-4-amine as a yellow solid (940 g, 89.9% yield). LC-MS: M⁺¹=420.9.

At room temperature, 7-ethoxy-N⁴-(4-(4-fluorophenoxy)phenyl)-6-nitroquinazolin-4-amine (200 g) was dissolved in tetrahydrofuran (2 L) and added to a 5 L reaction flask along with anhydrous ethanol (1 L). Then 10% wet palladium on carbon (20 g) was added, and the mixture was stirred thoroughly. The reaction mixture was then placed in a 5 L autoclave. Hydrogen was introduced and pressurized to 0.3 MPa. The temperature was raised to 50-55 °C, and the reaction was allowed to react for 5 hours to obtain a crude product. This crude product was then mixed with the crude products from other batches and dried under reduced pressure. The product was then slurried in n-heptane, filtered, and dried (15-20°C) to obtain IM1 (7-ethoxy-N⁴-(4-(4-fluorophenoxy)phenyl)quinazolin-4,6-diamine) as an orange solid (600g, 99.76% purity, and 81% yield). LC-MS: M⁺¹ = 391.2.

At room temperature, add 1,1'-carbonyldiimidazole (126.2 g) and tetrahydrofuran (0.3 L) to a 5 L three-necked reaction flask and stir steadily. Add a solution of diethylphosphoacetic acid (150 g) in tetrahydrofuran (0.3 L) dropwise; heat to 40 °C; and stir for 30 min. 7-Ethoxy-N⁴-(4-(4-fluorophenoxy)phenyl)quinazoline-4,6-diamine (143 g) was suspended in tetrahydrofuran (0.9 L) and added to the above reaction system. The reaction was continued at 40 °C for at least 2 h. After completion of the reaction, the mixture was filtered while hot, and dried by rotary evaporation. The residue was recrystallized from 95% ethanol, and solids were collected by filtration, and dried to obtain IM2 (diethyl (2-((7-ethoxy-4-((4-(4-fluorophenoxy)phenyl)amino)-quinazolin-6-yl)amino)-2-oxoethyl)phosphonate) as a yellow solid (170 g, 99.41% purity, and 77.8% yield). LC-MS: M⁺¹=569.2.

At room temperature, IM2 (156 g), tetrahydrofuran (0.78 L), and anhydrous lithium chloride (13.9 g) were added to a 5 L three-necked flask. The mixture was stirred, and cooled to below -5°C. Potassium hydroxide solution (154 g/0.77 L water) was added dropwise, while controlling the system temperature to from -10 °C to 0 °C. A 2-dimethylaminoacetaldehyde sodium bisulfite solution (105 g/0.315 L water) was then added dropwise. After the reaction was complete, the mixture was rotavapped to obtain a solid crude compound. This crude compound was then recrystallized from n-heptane; solids were filtered, and dried to obtain the title compound (E)-4-(dimethylamino)-N-(4-((4-(4-fluoro-phenoxy)phenyl)amino)-7-ethoxyquinazolin-6-yl)but-2-enamide (110 g, 99.20% purity). LC-MS: M⁺¹=502.2, M⁺²/2= 251.6; ¹HNMR (DMSO-*d*₆, 400 MHz): 1.457 (t, J₁ =6.8, J₂ =7.2, 3H), 2.188 (s, 6H), 3.092 (t, J₁ =0.8, J₂ =5.2, 2H), 4.275 (q, J₁ =6.8, J₂ =7.2, 2H), 6.598 (s,1H), 6.793-6.832 (m,1H), 7.017-7.089 (m,4H),7.202-7.246(m,3H), 7.797 (q, J₁ =3.2, J₂ =2.4, 1H), 8.461 (s, 1H), 8.925 (s, 1H), 9.505 (s, 1H), and 9.697 (s, 1H).

### Example 2. Preparation of (E)-4-(dimethylamino)-N-(4-((4-(4-fluoro-phenoxy)phenyl)amino)-7-ethoxyquinazolin-6-yl)but-2-enamide maleate crystal form A

### Preparation of maleate crystal form A:

At room temperature, 278.9 mg (0.56 mmol, 1.0 eq) of (E)-4-(dimethylamino)-N-(4-((4-(4-fluoro-phenoxy)phenyl)amino)-7-ethoxyquinazolin-6-yl)but-2-enamide (from Example 1) was weighed into a 50 mL single-necked bottle; 12 mL of 90% isopropanol was added; and the mixture was stirred to dissolve the solids. Separately, 129 mg (1.11 mmol, 2.0 eq) of maleic acid was weighed and dissolved in 1 mL of 90% isopropanol, and the solution was added dropwise to the reaction flask. After stirring at room temperature overnight, the mixture was filtered, rinsed with a small amount of 90% isopropanol, and dried at 40 °C for 8 h to obtain 346 mg of a yellow solid (maleate crystal form A).

Its PXRD pattern, TGA diagram and NMR spectrum are shown in FIGs. 1-3, respectively. The PXRD pattern data is shown below:

| **Peak No.** | **2θ** | **Flex breadth** | **d-value** | **Peak Intensity** | **I/I₀** |
|---|---|---|---|---|---|
| 1 | 5.100 | 0.165 | 17.3131 | 190893 | 64 |
| 2 | 5.680 | 0.235 | 15.5465 | 299311 | 100 |
| 3 | 10.500 | 0.165 | 8.4182 | 63836 | 22 |
| 4 | 11.240 | 0.071 | 7.8656 | 76914 | 26 |
| 5 | 11.300 | 0.188 | 7.8240 | 83824 | 28 |
| 6 | 16.700 | 0.071 | 5.3042 | 65719 | 22 |
| 7 | 16.900 | 0.071 | 5.2419 | 86487 | 29 |
| 8 | 18.020 | 0.094 | 4.9186 | 100267 | 34 |
| 9 | 18.400 | 0.071 | 4.8178 | 139532 | 47 |
| 10 | 18.480 | 0.071 | 4.7972 | 130233 | 44 |
| 11 | 18.900 | 0.071 | 4.6915 | 65321 | 22 |
| 12 | 19.000 | 0.094 | 4.6670 | 66434 | 23 |
| 13 | 19.780 | 0.071 | 4.4847 | 64785 | 22 |
| 14 | 20.420 | 0.094 | 4.3456 | 91401 | 31 |
| 15 | 20.880 | 0.118 | 4.2509 | 85985 | 29 |
| 16 | 21.080 | 0.282 | 4.2110 | 91624 | 31 |
| 17 | 22.000 | 0.071 | 4.0369 | 68678 | 23 |
| 18 | 25.180 | 0.094 | 3.5338 | 72805 | 25 |
| 19 | 25.360 | 0.071 | 3.5092 | 97905 | 33 |
| 20 | 25.480 | 0.118 | 3.4929 | 104976 | 36 |
| 21 | 25.940 | 0.071 | 3.4320 | 68059 | 23 |
| 22 | 26.040 | 0.071 | 3.4190 | 69974 | 24 |
| 23 | 26.720 | 0.071 | 3.3336 | 142491 | 48 |
| 24 | 26.780 | 0.071 | 3.3262 | 165355 | 56 |
| 25 | 26.880 | 0.212 | 3.3141 | 180705 | 61 |
| 26 | 27.260 | 0.071 | 3.2687 | 84720 | 29 |
| 27 | 27.380 | 0.071 | 3.2547 | 81093 | 28 |
| 28 | 27.540 | 0.094 | 3.2361 | 78029 | 27 |

Similarly, crystal form A was prepared in the following manner:

| **solvent** | **free base batch** | **solvent amount** | **product** | **solid state** | **crystal form** | **yield** | **note** |
|---|---|---|---|---|---|---|---|
| MeOH | 233 mg | 1.2 mL | 120 mg | light yellow solids | crystal form A | 33.0% | standing overnight at -5 °C |

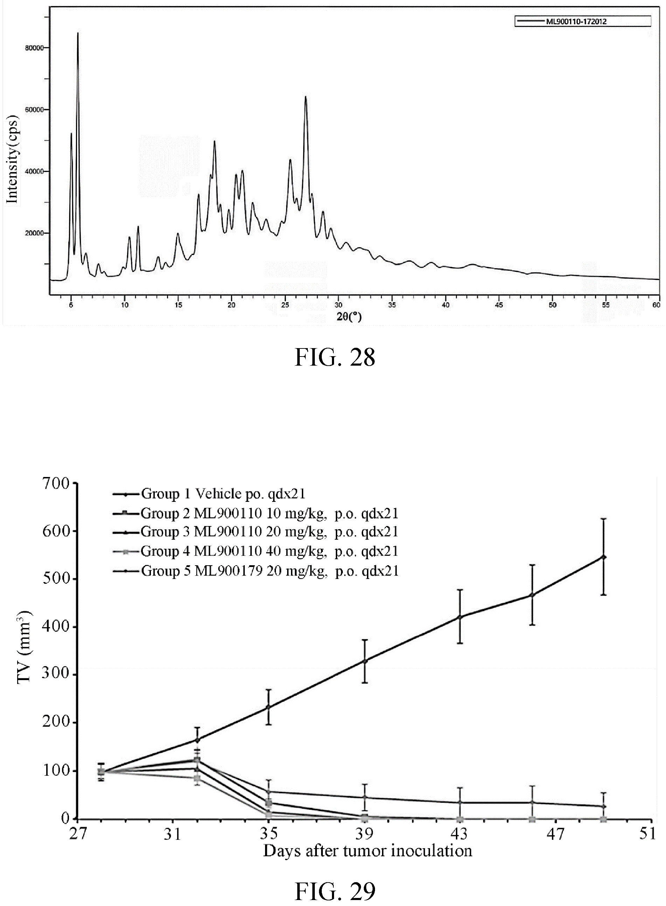

Its PXRD is shown in FIG. 28.

### Example 3. Preparation of (E)-4-(dimethylamino)-N-(4-((4-(4-fluoro-phenoxy)phenyl)amino)-7-ethoxyquinazolin-6-yl)but-2-enamide maleate crystal form B

### Preparation of maleate crystal form B:

At room temperature, 247.3 mg (0.493 mmol, 1.0 eq) of (E)-4-(dimethylamino)-N-(4-((4-(4-fluoro-phenoxy)phenyl)amino)-7-ethoxyquinazolin-6-yl)but-2-enamide (from Example 1) was weighed into a 50 mL single-necked flask and 3 mL of 95% ethanol was added and stirred to dissolve. Separately, 114.5 mg (0.986 mmol, 2.0 eq) of maleic acid was weighed and dissolved in 2 mL of 95% ethanol (aqueous solution) and added dropwise to the reaction flask. The temperature was raised to 60 °C to dissolve the reagents. An additional 95% ethanol (37 mL) was added, and the solids were completely dissolved. The mixture was allowed to stand in an oil bath, and cooled to room temperature for crystallization overnight. The solids were collected by suction filtration, and air-dried at 40 °C for 7 h to obtain 194 mg of a solid (maleate crystal form B). Its PXRD pattern and TGA diagram are shown in FIGs. 4 and 5, respectively. The PXRD pattern data is shown below:

| **Peak No.** | **2θ** | **Flex breadth** | **d-value** | **Peak Intensity** | **I/I₀** |
|---|---|---|---|---|---|
| 1 | 5.080 | 0.165 | 17.3812 | 581537 | 100 |
| 2 | 7.040 | 0.188 | 12.5459 | 78133 | 14 |
| 3 | 9.800 | 0.212 | 9.0179 | 160469 | 28 |
| 4 | 10.120 | 0.188 | 8.7335 | 265045 | 46 |
| 5 | 14.080 | 0.141 | 6.2848 | 60323 | 11 |
| 6 | 16.520 | 0.165 | 5.3616 | 56104 | 10 |
| 7 | 17.700 | 0.141 | 5.0068 | 65000 | 12 |
| 8 | 17.800 | 0.071 | 4.9789 | 58355 | 11 |
| 9 | 18.080 | 0.118 | 4.9024 | 62629 | 11 |
| 10 | 18.400 | 0.165 | 4.8178 | 85045 | 15 |
| 11 | 19.380 | 0.118 | 4.5764 | 152939 | 27 |
| 12 | 19.620 | 0.141 | 4.5209 | 114255 | 20 |
| 13 | 20.280 | 0.212 | 4.3753 | 197989 | 35 |
| 14 | 20.720 | 0.094 | 4.2833 | 63632 | 11 |
| 15 | 22.120 | 0.071 | 4.0153 | 78558 | 14 |
| 16 | 22.280 | 0.071 | 3.9868 | 90446 | 16 |
| 17 | 22.480 | 0.071 | 3.9518 | 81769 | 15 |
| 18 | 22.560 | 0.094 | 3.9380 | 88698 | 16 |
| 19 | 23.100 | 0.094 | 3.8471 | 85880 | 15 |
| 20 | 23.420 | 0.141 | 3.7953 | 84726 | 15 |
| 21 | 25.120 | 0.118 | 3.5421 | 94819 | 17 |
| 22 | 25.740 | 0.118 | 3.4582 | 67008 | 12 |
| 23 | 26.640 | 0.165 | 3.3434 | 69790 | 12 |
| 24 | 29.600 | 0.071 | 3.0154 | 70543 | 13 |
| 25 | 29.680 | 0.071 | 3.0075 | 68128 | 12 |

### Example 4. Preparation of (E)-4-(dimethylamino)-N-(4-((4-(4-fluoro-phenoxy)phenyl)amino)-7-ethoxyquinazolin-6-yl)but-2-enamide maleate crystal form C

### Preparation of maleate crystal form C:

At room temperature, 231 mg (0.46 mmol, 1.0 eq) of (E)-4-(dimethylamino)-N-(4-((4-(4-fluoro-phenoxy)phenyl)amino)-7-ethoxyquinazolin-6-yl)but-2-enamide (from Example 1) was weighed into a 5 mL plastic tube; 1 mL of anhydrous ethanol was added; and the mixture was stirred to dissolve the solids. Separately, 53.5 mg (0.46 mmol, 1.0 eq) of maleic acid was weighed and dissolved in 0.2 mL of anhydrous ethanol; and the solution was then added dropwise to the plastic tube from the previous step. The mixture was stirred at room temperature for 25 min, resulting in the precipitation of a large amount of solids. After standing overnight, the solids were collected by suction filtration, rinsed with 0.2 mL of anhydrous ethanol, and air-dried at 40 °C for 1 hour 50 minutes to obtain 171 mg of a yellow solid (maleate crystal form C). Its PXRD pattern and NMR spectrum are shown in FIGs. 6 and 7, respectively. The PXRD pattern data is shown below:

| **Peak No.** | **2θ** | **Flex breadth** | **d-value** | **Peak Intensity** | **I/I₀** |
|---|---|---|---|---|---|
| 1 | 5.380 | 0.235 | 16.4127 | 100576 | 81 |
| 2 | 5.760 | 0.259 | 15.3307 | 124103 | 100 |
| 3 | 6.100 | 0.212 | 14.4770 | 92503 | 74 |
| 4 | 18.200 | 0.212 | 4.8703 | 104971 | 84 |
| 5 | 18.700 | 0.235 | 4.7412 | 125312 | 100 |
| 6 | 19.020 | 0.188 | 4.6622 | 99259 | 80 |
| 7 | 19.800 | 0.212 | 4.4802 | 78929 | 63 |
| 8 | 21.460 | 0.118 | 4.1373 | 80082 | 64 |
| 9 | 22.260 | 0.353 | 3.9904 | 87690 | 70 |
| 10 | 22.840 | 0.141 | 3.8903 | 77323 | 62 |
| 11 | 23.740 | 0.118 | 3.7448 | 79314 | 64 |
| 12 | 25.620 | 0.282 | 3.4741 | 95803 | 77 |
| 13 | 26.820 | 0.118 | 3.3214 | 100403 | 81 |

### Example 5. Preparation of (E)-4-(dimethylamino)-N-(4-((4-(4-fluoro-phenoxy)phenyl)amino)-7-ethoxyquinazolin-6-yl)but-2-enamide hydrochloride crystal forms

### Preparation of hydrochloride crystal form S-1:

At room temperature, 221 mg (0.44 mmol, 1.0 eq) of (E)-4-(dimethylamino)-N-(4-((4-(4-fluoro-phenoxy)phenyl)amino)-7-ethoxyquinazolin-6-yl)but-2-enamide (from Example 1) was weighed into a 5 mL plastic tube; 1 mL of anhydrous ethanol was added; and the mixture was stirred to dissolve the solids. Separately, 45 mg of hydrochloric acid (36%) (0.44 mmol, 1.0 eq) was weighed and dissolved in 0.2 mL of anhydrous ethanol; and the solution was then added dropwise to the plastic tube from the previous step. The mixture was stirred at room temperature for 1 hour. No solid precipitated. After standing overnight at 0-5 °C, a white solid precipitated. This solid was collected by suction filtration and air-dried at 40 °C for 4 hours and 10 minutes to yield 159 mg of a yellow solid. Its PXRD pattern is shown in FIG. 8. The PXRD pattern data is shown below:

| **Peak No.** | **2θ** | **Flex breadth** | **d-value** | **Peak Intensity** | **I/I₀** |
|---|---|---|---|---|---|
| 1 | 6.120 | 0.235 | 14.4297 | 84615 | 100 |
| 2 | 14.920 | 0.141 | 5.9328 | 43444 | 52 |
| 3 | 15.500 | 0.165 | 5.7121 | 50031 | 60 |
| 4 | 18.140 | 0.259 | 4.8863 | 65783 | 78 |
| 5 | 19.620 | 0.118 | 4.5209 | 57622 | 69 |
| 6 | 19.740 | 0.094 | 4.4937 | 65521 | 78 |
| 7 | 20.360 | 0.094 | 4.3582 | 40087 | 48 |
| 8 | 20.480 | 0.094 | 4.3330 | 39902 | 48 |
| 9 | 22.580 | 0.282 | 3.9345 | 49202 | 59 |
| 10 | 23.300 | 0.188 | 3.8145 | 47062 | 56 |
| 11 | 24.120 | 0.259 | 3.6867 | 56213 | 67 |
| 12 | 24.980 | 0.235 | 3.5617 | 40559 | 48 |
| 13 | 25.560 | 0.188 | 3.4822 | 45562 | 54 |
| 14 | 26.020 | 0.165 | 3.4216 | 42926 | 51 |
| 15 | 28.560 | 0.141 | 3.1228 | 38126 | 46 |
| 16 | 28.700 | 0.118 | 3.1079 | 39524 | 47 |

### Preparation of hydrochloride crystal form S-2:

The operation is the same as above for Form S-1:

| **solvent** | **free base batch** | **solvent amount** | **product** | **solid state** | **crystal form** | **yield** | **note** |
|---|---|---|---|---|---|---|---|
| MeOH | 274 mg | 1.2 mL | 170 mg | light yellow solids | crystal form S-2 | 62.0% | standing overnight at 5 °C |

The PXRD pattern of the hydrochloride crystal form S-2 is shown in FIG. 9. The PXRD pattern data is shown below:

| **Peak No.** | **2θ** | **Flex breadth** | **d-value** | **Peak Intensity** | **I/I₀** |
|---|---|---|---|---|---|
| 1 | 5.277(11) | 0.233(9) | 16.73(3) | 915(47) | 5.57 |
| 2 | 6.202(3) | 0.267(4) | 14.240(8) | 6139(66) | 37.36 |
| 3 | 6.751(4) | 0.274(6) | 13.084(7) | 3447(53) | 20.98 |
| 4 | 8.169(4) | 0.282(3) | 10.815(5) | 2955(34) | 17.98 |
| 5 | 10.068(11) | 0.347(12) | 8.778(10) | 1389(32) | 8.45 |
| 6 | 12.568(8) | 0.601(6) | 7.038(4) | 4227(57) | 25.72 |
| 7 | 13.96(4) | 0.41(11) | 6.34(2) | 103(23) | 0.63 |
| 8 | 14.718(3) | 0.315(6) | 6.0141(11) | 2178(38) | 13.26 |
| 9 | 15.234(6) | 0.414(16) | 5.811(2) | 1311(40) | 7.98 |
| 10 | 16.104(7) | 0.425(12) | 5.499(2) | 2658(99) | 16.17 |
| 11 | 16.602(9) | 0.45(2) | 5.335(3) | 2189(109) | 13.32 |
| 12 | 17.275(3) | 0.369(9) | 5.1292(9) | 2286(46) | 13.91 |
| 13 | 17.833(7) | 0.354(19) | 4.970(2) | 793(35) | 4.82 |
| 14 | 18.825(4) | 0.535(6) | 4.7101(10) | 8160(129) | 49.66 |
| 15 | 19.482(2) | 0.583(5) | 4.5528(5) | 16432(145) | 100.00 |
| 16 | 20.4303(19) | 0.530(5) | 4.3435(4) | 7287(56) | 44.35 |
| 17 | 21.418(5) | 0.552(12) | 4.1454(9) | 2474(42) | 15.06 |
| 18 | 22.335(6) | 0.405(14) | 3.9772(11) | 1475(49) | 8.98 |
| 19 | 22.84(6) | 0.25(11) | 3.891(10) | 237(45) | 1.44 |
| 20 | 23.126(18) | 0.21(7) | 3.843(3) | 169(49) | 1.03 |
| 21 | 23.852(6) | 0.286(16) | 3.7275(10) | 623(28) | 3.79 |
| 22 | 24.788(9) | 0.439(15) | 3.5890(12) | 2471(101) | 15.04 |
| 23 | 25.363(8) | 0.437(14) | 3.5088(10) | 10575(512) | 64.36 |
| 24 | 25.773(9) | 0.397(18) | 3.4539(11) | 7537(588) | 45.86 |
| 25 | 26.326(7) | 0.660(13) | 3.3826(9) | 10063(224) | 61.24 |
| 26 | 27.538(7) | 0.720(18) | 3.2364(8) | 2749(56) | 16.73 |
| 27 | 28.729(3) | 0.694(8) | 3.1049(4) | 5715(56) | 34.78 |
| 28 | 29.911(4) | 0.617(9) | 2.9849(4) | 4229(51) | 25.73 |
| 29 | 31.56(3) | 0.59(5) | 2.832(2) | 1296(120) | 7.89 |
| 30 | 32.17(7) | 0.47(13) | 2.780(6) | 355(118) | 2.16 |
| 31 | 33.662(17) | 0.61(4) | 2.6604(13) | 711(39) | 4.33 |
| 32 | 35.19(7) | 0.46(6) | 2.548(5) | 333(60) | 2.03 |
| 33 | 37.14(5) | 0.30(14) | 2.419(3) | 86(40) | 0.53 |
| 34 | 38.36(6) | 1.27(7) | 2.345(4) | 1589(94) | 9.67 |

### Example 6. Preparation of (E)-4-(dimethylamino)-N-(4-((4-(4-fluoro-phenoxy)phenyl)amino)-7-ethoxyquinazolin-6-yl)but-2-enamide sulfate crystal forms

### Preparation of sulfate crystal form S-3:

At room temperature, 243 mg (0.48 mmol, 1.0 eq) of (E)-4-(dimethylamino)-N-(4-((4-(4-fluoro-phenoxy)phenyl)amino)-7-ethoxyquinazolin-6-yl)but-2-enamide (from Example 1) was weighed into a 5 mL plastic tube; 1 mL of anhydrous ethanol was added; and the mixture was stirred to dissolve the solids. Separately, 48 mg of sulfuric acid (0.48 mmol, 1.0 eq) was weighed and dissolved in 0.2 mL of anhydrous ethanol; and the solution was added dropwise to the plastic tube from the previous step. The mixture was stirred at room temperature for 35 minutes, resulting in the precipitation of a large amount of white solid. The solids were collected by suction filtration, and dried under forced air at 40 °C for 4 hours to yield 225 mg of a white solid. Its PXRD pattern is shown in FIG. 10. The PXRD pattern data is shown below:

| **Peak No.** | **2θ** | **Flex breadth** | **d-value** | **Peak Intensity** | **I/I₀** |
|---|---|---|---|---|---|
| 1 | 5.200 | 0.259 | 16.9804 | 224794 | 100 |
| 2 | 16.040 | 0.282 | 5.5210 | 63053 | 29 |
| 3 | 17.700 | 0.188 | 5.0068 | 52671 | 24 |
| 4 | 22.540 | 0.094 | 3.9414 | 52857 | 24 |
| 5 | 24.540 | 0.188 | 3.6245 | 49798 | 23 |
| 6 | 25.120 | 0.094 | 3.5421 | 54438 | 25 |
| 7 | 25.400 | 0.165 | 3.5037 | 65350 | 30 |
| 8 | 26.080 | 0.094 | 3.4139 | 66917 | 30 |
| 9 | 26.160 | 0.094 | 3.4036 | 64924 | 29 |
| 10 | 26.600 | 0.094 | 3.3483 | 48661 | 22 |

### Preparation of sulfate crystal forms S-4 and S-5:

The operation is the same as above for Form S-3:

| **solvent** | **free base batch** | **solvent amount** | **product** | **solid state** | **crystal form** | **yield** | **note** |
|---|---|---|---|---|---|---|---|
| acetone | 242 mg | 2.3 mL | 258 mg | light yellow solids | crystal form S-4 | 106.6% | standing at room temperature |
| i-PrOH | 270 mg | 2.3 mL | 208 mg | yellow solids | crystal form S-5 | 77.0% | standing at room temperature |

The PXRD patterns of sulfate crystal forms S-4 and S-5 are shown in FIGs. 11 and 12, respectively.

The PXRD pattern data of sulfate crystal form S-4 is shown below:

| **Peak No.** | **2θ** | **Flex breadth** | **d-value** | **Peak Intensity** | **I/I₀** |
|---|---|---|---|---|---|
| 1 | 4.975(3) | 0.332(3) | 17.748(12) | 14199(75) | 13.50 |
| 2 | 8.522(16) | 0.34(3) | 10.367(19) | 415(33) | 0.39 |
| 3 | 9.88(4) | 0.81(7) | 8.94(3) | 847(52) | 0.81 |
| 4 | 11.333(11) | 0.324(15) | 7.801(8) | 1265(44) | 1.20 |
| 5 | 12.051(12) | 0.351(18) | 7.338(8) | 1153(50) | 1.10 |
| 6 | 14.67(2) | 0.736(19) | 6.033(9) | 4577(151) | 4.35 |
| 7 | 16.65(3) | 0.64(16) | 5.320(8) | 4035(754) | 3.84 |
| 8 | 17.03(2) | 0.27(4) | 5.201(7) | 1589(732) | 1.51 |
| 9 | 18.693(13) | 0.584(19) | 4.743(3) | 7618(142) | 7.24 |
| 10 | 20.216(14) | 0.29(4) | 4.389(3) | 838(144) | 0.80 |
| 11 | 20.604(19) | 13.67(5) | 4.307(4) | 105148(456) | 100.00 |
| 12 | 21.323(10) | 0.36(2) | 4.164(2) | 1361(80) | 1.29 |
| 13 | 22.989(16) | 1.01(5) | 3.866(3) | 8526(364) | 8.11 |
| 14 | 24.42(2) | 1.32(8) | 3.642(3) | 15480(1148) | 14.72 |
| 15 | 25.77(3) | 1.72(3) | 3.455(4) | 18389(1072) | 17.49 |
| 16 | 28.030(18) | 0.83(5) | 3.181(2) | 2334(149) | 2.22 |
| 17 | 31.99(5) | 0.41(13) | 2.796(4) | 161(48) | 0.15 |
| 18 | 34.81(5) | 0.69(13) | 2.575(3) | 340(81) | 0.32 |

The PXRD pattern data of sulfate crystal form S-5 is shown below:

| **Peak No.** | **2θ** | **Flex breadth** | **d-value** | **Peak Intensity** | **I/I₀** |
|---|---|---|---|---|---|
| 1 | 4.72(3) | 0.56(7) | 18.71(11) | 448(53) | 0.48 |
| 2 | 5.503(3) | 0.226(3) | 16.048(8) | 6647(59) | 7.08 |
| 3 | 6.99(3) | 1.02(8) | 12.64(5) | 986(59) | 1.05 |
| 4 | 9.34(4) | 0.22(4) | 9.46(4) | 271(30) | 0.29 |
| 5 | 10.75(2) | 0.37(2) | 8.226(17) | 842(54) | 0.90 |
| 6 | 12.959(18) | 0.40(3) | 6.826(10) | 2613(82) | 2.79 |
| 7 | 13.996(19) | 0.66(7) | 6.323(9) | 833(77) | 0.89 |
| 8 | 15.112(15) | 0.79(4) | 5.858(6) | 1927(103) | 2.05 |
| 9 | 16.041(7) | 0.29(2) | 5.521(2) | 763(57) | 0.81 |
| 10 | 16.476(13) | 0.24(3) | 5.376(4) | 334(49) | 0.36 |
| 11 | 18.32(2) | 0.22(8) | 4.838(6) | 278(87) | 0.30 |
| 12 | 18.880(5) | 0.315(11) | 4.6965(13) | 3908(103) | 4.16 |
| 13 | 19.748(9) | 0.49(2) | 4.492(2) | 3874(132) | 4.13 |
| 14 | 20.988(13) | 0.85(3) | 4.229(3) | 8042(193) | 8.57 |
| 15 | 22.270(7) | 0.454(16) | 3.9887(13) | 5753(141) | 6.13 |
| 16 | 22.66(2) | 11.58(3) | 3.921(4) | 93831(283) | 100.00 |
| 17 | 23.343(9) | 0.49(3) | 3.8078(15) | 4604(198) | 4.91 |
| 18 | 23.930(10) | 0.33(3) | 3.7156(16) | 1979(173) | 2.11 |
| 19 | 24.745(12) | 0.73(3) | 3.5950(17) | 7618(242) | 8.12 |
| 20 | 26.35(4) | 2.55(5) | 3.380(5) | 21651(231) | 23.07 |
| 21 | 27.90(4) | 0.37(5) | 3.195(4) | 1141(96) | 1.22 |
| 22 | 31.92(3) | 0.62(11) | 2.801(3) | 492(100) | 0.52 |
| 23 | 33.31(13) | 1.4(3) | 2.688(11) | 493(99) | 0.53 |
| 24 | 35.66(4) | 0.54(11) | 2.516(3) | 231(62) | 0.25 |

### Example 7. Preparation of (E)-4-(dimethylamino)-N-(4-((4-(4-fluoro-phenoxy)phenyl)amino)-7-ethoxyquinazolin-6-yl)but-2-enamide tartrate crystal form

Preparation of tartrate crystal form S-6: the operation is the same as above for Example 6

| **solvent** | **free base batch** | **solvent amount** | **product** | **solid state** | **crystal form** | **yield** | **note** |
|---|---|---|---|---|---|---|---|
| i-PrOH + water | 229 mg | 3 mL + 1 mL | 192 mg | yellow solids | crystal form S-6 | 83.8% | standing at room temperature |

The PXRD pattern of tartrate crystal form S-6 is shown in FIG. 13. The PXRD pattern data is shown below:

| **Peak No.** | **2θ** | **Flex breadth** | **d-value** | **Peak Intensity** | **I/I₀** |
|---|---|---|---|---|---|
| 1 | 4.038(5) | 0.248(9) | 21.86(3) | 1860(96) | 0.32 |
| 2 | 4.84(4) | 1.72(12) | 18.23(16) | 3804(198) | 0.66 |
| 3 | 6.186(5) | 0.277(8) | 14.277(12) | 2850(71) | 0.50 |
| 4 | 6.997(9) | 0.30(2) | 12.624(15) | 934(55) | 0.16 |
| 5 | 10.237(18) | 0.38(3) | 8.634(15) | 1566(74) | 0.27 |
| 6 | 11.776(17) | 0.33(3) | 7.509(11) | 439(44) | 0.08 |
| 7 | 12.43(2) | 0.42(2) | 7.117(12) | 1260(55) | 0.22 |
| 8 | 14.202(11) | 0.53(3) | 6.231(5) | 1412(112) | 0.25 |
| 9 | 16.78(5) | 1.28(15) | 5.280(14) | 2988(316) | 0.52 |
| 10 | 17.805(19) | 0.62(6) | 4.978(5) | 2420(262) | 0.42 |
| 11 | 18.710(18) | 0.83(6) | 4.739(4) | 5625(366) | 0.98 |
| 12 | 19.390(15) | 0.45(6) | 4.574(4) | 1662(264) | 0.29 |
| 13 | 20.208(18) | 0.81(3) | 4.391(4) | 6485(242) | 1.13 |
| 14 | 20.59(3) | 18.24(5) | 4.311(7) | 573453(547) | 100 |
| 15 | 21.77(7) | 1.1(3) | 4.079(14) | 1984(433) | 0.35 |
| 16 | 22.59(3) | 0.55(9) | 3.933(5) | 1591(357) | 0.28 |
| 17 | 23.54(3) | 0.91(11) | 3.776(5) | 7914(1025) | 1.38 |
| 18 | 24.112(10) | 0.56(4) | 3.6879(16) | 6242(870) | 1.09 |
| 19 | 24.869(16) | 0.74(4) | 3.577(2) | 4940(437) | 0.86 |
| 20 | 27.359(12) | 2.21(4) | 3.2572(14) | 12541(168) | 2.19 |
| 21 | 36.51(10) | 4.6(2) | 2.459(7) | 4866(203) | 0.85 |

### Example 8. Preparation of (E)-4-(dimethylamino)-N-(4-((4-(4-fluoro-phenoxy)phenyl)amino)-7-ethoxyquinazolin-6-yl)but-2-enamide fumarate crystal form

Preparation of fumarate crystal form S-7: the operation is the same as above for Example 6

| **solvent** | **free base batch** | **solvent amount** | **product** | **solid state** | **crystal form** | **yield** | **note** |
|---|---|---|---|---|---|---|---|
| MeOH | 271 mg | 2.5 mL | 142 mg | yellow solids | crystal form S-7 | 52.4% | standing overnight at -5 °C |

The PXRD pattern of the fumarate crystal form S-7 is shown in FIG. 14. The PXRD pattern data is shown below:

| **Peak No.** | **2θ** | **Flex breadth** | **d-value** | **Peak Intensity** | **I/I₀** |
|---|---|---|---|---|---|
| 1 | 3.701(3) | 0.245(5) | 23.853(19) | 1954(26) | 1.42 |
| 2 | 4.361(2) | 0.222(2) | 20.244(10) | 4967(37) | 3.61 |
| 3 | 6.8914(15) | 0.2739(13) | 12.816(3) | 12418(53) | 9.02 |
| 4 | 7.504(3) | 0.287(6) | 11.771(4) | 2118(36) | 1.54 |
| 5 | 7.931(2) | 0.249(5) | 11.138(3) | 1490(26) | 1.08 |
| 6 | 8.820(2) | 0.2707(17) | 10.017(2) | 7384(44) | 5.37 |
| 7 | 10.203(18) | 0.22(5) | 8.662(16) | 177(38) | 0.13 |
| 8 | 10.902(11) | 0.47(2) | 8.109(8) | 1782(66) | 1.29 |
| 9 | 11.804(8) | 0.481(12) | 7.491(5) | 3843(139) | 2.79 |
| 10 | 12.3(2) | 0.73(7) | 7.17(13) | 1095(109) | 0.80 |
| 11 | 13.52(4) | 0.46(8) | 6.54(2) | 1392(331) | 1.01 |
| 12 | 13.812(7) | 0.281(14) | 6.406(3) | 2283(337) | 1.66 |
| 13 | 15.280(9) | 0.35(2) | 5.794(3) | 1368(70) | 0.99 |
| 14 | 16.086(10) | 0.26(2) | 5.505(3) | 1561(240) | 1.13 |
| 15 | 16.421(11) | 0.40(4) | 5.394(4) | 3407(336) | 2.48 |
| 16 | 16.971(16) | 0.40(5) | 5.220(5) | 1541(165) | 1.12 |
| 17 | 17.870(6) | 0.559(9) | 4.9597(16) | 11126(147) | 8.08 |
| 18 | 18.859(17) | 0.55(5) | 4.702(4) | 2284(180) | 1.66 |
| 19 | 19.548(16) | 0.47(5) | 4.537(4) | 1851(173) | 1.35 |
| 20 | 20.282(14) | 0.41(4) | 4.375(3) | 1430(108) | 1.04 |
| 21 | 21.740(9) | 0.62(2) | 4.0846(16) | 6289(239) | 4.57 |
| 22 | 22.708(9) | 0.84(2) | 3.9128(16) | 12318(266) | 8.95 |
| 23 | 24.155(5) | 0.500(8) | 3.6815(8) | 16710(219) | 12.14 |
| 24 | 24.885(4) | 0.419(10) | 3.5752(6) | 10634(308) | 7.73 |
| 25 | 25.43(5) | 9.98(5) | 3.499(6) | 137624(471) | 100.00 |
| 26 | 25.650(7) | 0.809(15) | 3.4702(9) | 26653(433) | 19.37 |
| 27 | 28.078(12) | 0.87(3) | 3.1754(13) | 8847(267) | 6.43 |
| 28 | 29.15(3) | 0.86(7) | 3.061(3) | 3206(264) | 2.33 |
| 29 | 31.79(5) | 0.53(11) | 2.812(4) | 397(72) | 0.29 |
| 30 | 32.87(4) | 0.60(6) | 2.723(3) | 959(143) | 0.70 |
| 31 | 34.1(9) | 1.2(3) | 2.62(6) | 1197(207) | 0.87 |
| 32 | 34.77(4) | 0.62(9) | 2.578(3) | 724(162) | 0.53 |
| 33 | 38.33(9) | 0.59(11) | 2.346(5) | 744(86) | 0.54 |

### Example 9. Preparation of (E)-4-(dimethylamino)-N-(4-((4-(4-fluoro-phenoxy)phenyl)amino)-7-ethoxyquinazolin-6-yl)but-2-enamide p-toluenesulfonate crystal form

Preparation of p-toluenesulfonate crystal form S-8: the operation is the same as above for Example 6

| **solvent** | **free base batch** | **solvent amount** | **product** | **solid state** | **crystal form** | **yield** | **note** |
|---|---|---|---|---|---|---|---|
| acetonitrile | 254 mg | 14 mL | 247 mg | light yellow solids | crystal form S-8 | 97.2% | standing overnight at -5 °C |

The PXRD pattern of p-toluenesulfonate crystal form S-8 is shown in FIG. 15. The PXRD pattern data is shown below:

| **Peak No.** | **2θ** | **Flex breadth** | **d-value** | **Peak Intensity** | **I/I₀** |
|---|---|---|---|---|---|
| 1 | 5.7102(15) | 0.2635(12) | 15.465(4) | 16363(61) | 31.61 |
| 2 | 8.948(5) | 0.295(6) | 9.875(5) | 6968(91) | 13.46 |
| 3 | 10.127(7) | 0.428(8) | 8.728(6) | 9639(122) | 18.62 |
| 4 | 11.534(11) | 0.397(17) | 7.666(7) | 1330(53) | 2.57 |
| 5 | 12.411(13) | 0.40(3) | 7.126(8) | 1047(60) | 2.02 |
| 6 | 13.254(6) | 0.343(16) | 6.675(3) | 3341(167) | 6.45 |
| 7 | 13.702(6) | 0.334(14) | 6.457(3) | 3913(159) | 7.56 |
| 8 | 14.642(9) | 0.368(11) | 6.045(4) | 2635(62) | 5.09 |
| 9 | 15.521(14) | 0.59(3) | 5.705(5) | 3327(197) | 6.43 |
| 10 | 16.237(12) | 0.57(3) | 5.455(4) | 3641(216) | 7.04 |
| 11 | 16.96(3) | 0.37(7) | 5.223(9) | 428(85) | 0.83 |
| 12 | 18.588(4) | 0.471(5) | 4.7695(10) | 18977(146) | 36.66 |
| 13 | 19.590(9) | 0.41(2) | 4.528(2) | 3015(161) | 5.82 |
| 14 | 20.231(4) | 0.472(7) | 4.3859(8) | 16010(193) | 30.93 |
| 15 | 21.465(5) | 0.276(12) | 4.1363(10) | 2941(114) | 5.68 |
| 16 | 21.949(11) | 0.37(3) | 4.046(2) | 1928(138) | 3.72 |
| 17 | 23.373(8) | 0.910(11) | 3.8029(12) | 23703(223) | 45.79 |
| 18 | 24.369(4) | 0.354(10) | 3.6496(6) | 7259(171) | 14.02 |
| 19 | 25.014(5) | 0.490(11) | 3.5569(7) | 10862(194) | 20.99 |
| 20 | 25.47(6) | 6.62(5) | 3.494(8) | 51760(444) | 100.00 |
| 21 | 26.532(9) | 0.35(2) | 3.3569(11) | 2595(103) | 5.01 |
| 22 | 27.905(11) | 0.60(2) | 3.1947(12) | 4533(127) | 8.76 |
| 23 | 29.18(2) | 0.42(5) | 3.058(2) | 1029(98) | 1.99 |
| 24 | 31.65(5) | 1.26(6) | 2.825(4) | 6131(450) | 11.84 |
| 25 | 32.308(14) | 0.49(8) | 2.7687(12) | 2119(362) | 4.09 |
| 26 | 34.45(2) | 1.14(6) | 2.6012(17) | 3950(191) | 7.63 |
| 27 | 36.92(4) | 0.31(4) | 2.432(3) | 509(62) | 0.98 |

### Example 10. (E)-4-(dimethylamino)-N-(4-((4-(4-fluoro-phenoxy)phenyl)amino)-7-ethoxyquinazolin-6-yl)but-2-enamide (free base) in different crystal forms

### (1) Stirring at room temperature

The free base of (E)-4-(dimethylamino)-N-(4-((4-(4-fluoro-phenoxy)phenyl)amino)-7-ethoxyquinazolin-6-yl)but-2-enamide was stirred in different solvent systems (or a mixture of two solvents) at room temperature for 24 h. The solids were collected by suction filtration, and the obtained solid was dried and characterized (PXRD, DSC-TGA).

**Summary table of standing crystallization of the free base at room temperature**

| **free base batch** | **solvent** | **solvent amount** | **product** | **crystal form** | **yield** |
|---|---|---|---|---|---|
| 209 mg | n-heptane | 2 mL | 116 mg | crystal form F-1 | 55.5% |
| 253 mg | EtOAc | 2 mL | 126 mg | crystal form F-3 | 49.8% |
| 296 mg | water | 2 mL | 211 mg | crystal form F-1 | 71.3% |
| 238 mg | CH₃CN | 2 mL | 182 mg | crystal form F-3 | 76.5% |
| 206 mg | acetone/ water=1/1 | 4 mL | 183 mg | crystal form F-3 | 88.8% |
| 242 mg | MeOH/ water=1/2 | 1.5 mL | 173 mg | crystal form F-1 | 71.5% |
| 231 mg | EtOH/ water=1:1 | 1.0 mL | 183 mg | crystal form F-3 | 79.2% |
| 303 mg | i-PrOH | 0.5 mL | 209 ng | crystal form F-3 | 69.0% |

### (2) Heating, dissolving, then standing crystallization

The free base of (E)-4-(dimethylamino)-N-(4-((4-(4-fluoro-phenoxy)phenyl)amino)-7-ethoxyquinazolin-6-yl)but-2-enamide was dissolved in different solvent systems or a mixture of two solvents (e.g., 90% ethanol, 90% isopropanol and the like), and then cooled to room temperature for crystallization (standing crystallization). Then the mixture was stirred at room temperature for more than 1 hour. The solid was collected by suction filtration, dried, and characterized by DSC-TGA, PXRD.

Recrystallization (standing crystallization) was performed twice using ethyl acetate + activated carbon to obtain Form F-2.

| **free base batch** | **solvent** | **solvent amount** | **product** | **crystal form** | **yield** |
|---|---|---|---|---|---|
| 551 mg | EtOAc | 2 mL | 395 mg | crystal form F-1 | 71.7% |
| 571 mg | CH₃CN | 4 mL | 485 mg | crystal form F-4 | 84.9% |
| 518 mg | EtOAc/n-heptane=1/1 | 5 mL | 467 mg | crystal form F-4 | 90.2% |
| 516 mg | CH₃CN/ water =3/2 | 10 mL | 412 mg +20 mg (single crystal) | crystal forms F-1 and F-3 (mixed crystals) | 83.7% |
| 504 mg | acetone/ water=3/2 | 10 mL | 437 mg | crystal form F-3 | 86.7% |
| 527 mg | MeOH/ water=9/4 | 13 mL | 441 mg | crystal form F-5 | 83.7% |
| 516 mg | 80% EtOH | 4 mL | 344 mg | crystal form F-3 (with some mixed crystals) | 66.7% |
| 539 mg | i-PrOH | 2 mL | 412 ng | crystal form F-6 | 76.4% |

Among them, the PXRD patterns of crystal forms F-1 to F-6 are shown in FIGs. 16, 19, 22, and 25-27, respectively. The TGA diagrams of crystal forms F-1, F-2 and F-3 are shown in FIGs. 18, 21, and 24, respectively.

The PXRD pattern data of the free base crystal form F-1 is shown below:

| **Peak No.** | **2θ** | **Flex breadth** | **d-value** | **Peak Intensity** | **I/I₀** |
|---|---|---|---|---|---|
| 1 | 5.9199(17) | 0.2292(13) | 14.917(4) | 15339(85) | 100 |
| 2 | 7.674(5) | 0.268(4) | 11.511(8) | 1579(30) | 10.29 |
| 3 | 9.753(2) | 0.2667(19) | 9.062(2) | 8950(67) | 58.35 |
| 4 | 11.474(11) | 0.270(17) | 7.706(7) | 625(33) | 4.07 |
| 5 | 11.979(11) | 0.282(11) | 7.382(7) | 1182(53) | 7.7 |
| 6 | 12.901(4) | 0.279(3) | 6.8565(19) | 6360(68) | 41.46 |
| 7 | 14.203(18) | 0.26(5) | 6.231(8) | 169(29) | 1.1 |
| 8 | 15.527(6) | 0.363(6) | 5.702(2) | 2470(68) | 16.11 |
| 9 | 15.99(2) | 0.27(8) | 5.539(7) | 139(45) | 0.9 |
| 10 | 17.004(4) | 0.291(10) | 5.2103(14) | 1581(57) | 10.31 |
| 11 | 17.554(3) | 0.262(3) | 5.0481(8) | 5138(80) | 33.49 |
| 12 | 18.112(6) | 0.329(12) | 4.8939(15) | 1903(54) | 12.41 |
| 13 | 18.9(2) | 0.45(3) | 4.69(5) | 1475(88) | 9.62 |
| 14 | 19.140(5) | 0.265(9) | 4.6332(11) | 2444(88) | 15.93 |
| 15 | 19.741(2) | 0.288(5) | 4.4936(5) | 3893(52) | 25.38 |
| 16 | 20.743(10) | 0.530(15) | 4.279(2) | 6614(223) | 43.12 |
| 17 | 21.120(3) | 0.242(7) | 4.2031(5) | 5674(241) | 36.99 |
| 18 | 21.596(3) | 0.419(6) | 4.1116(5) | 9583(107) | 62.47 |
| 19 | 22.397(4) | 0.362(9) | 3.9663(7) | 2659(57) | 17.33 |
| 20 | 23.428(3) | 0.425(7) | 3.7941(5) | 4047(58) | 26.38 |
| 21 | 24.316(16) | 0.28(4) | 3.657(2) | 400(46) | 2.61 |
| 22 | 24.834(9) | 0.36(3) | 3.5823(13) | 1266(71) | 8.25 |
| 23 | 25.314(2) | 0.281(6) | 3.5154(3) | 4736(78) | 30.88 |
| 24 | 25.8258(16) | 0.292(3) | 3.4470(2) | 9208(83) | 60.03 |
| 25 | 26.985(12) | 0.96(3) | 3.3015(14) | 3590(92) | 23.4 |
| 26 | 27.934(6) | 0.291(15) | 3.1914(7) | 1030(46) | 6.71 |
| 27 | 29.49(4) | 0.43(7) | 3.026(4) | 1990(128) | 12.97 |
| 28 | 30.418(14) | 0.11(5) | 2.9362(14) | 721(384) | 4.7 |
| 29 | 30.620(17) | 0.28(4) | 2.9173(16) | 4668(437) | 30.44 |
| 30 | 31.82(3) | 0.16(8) | 2.810(3) | 136(40) | 0.89 |
| 31 | 32.45(4) | 0.28(4) | 2.757(3) | 540(64) | 3.52 |
| 32 | 33.11(3) | 0.22(5) | 2.704(3) | 303(50) | 1.98 |
| 33 | 35.091(7) | 0.40(2) | 2.5552(5) | 978(47) | 6.38 |
| 34 | 36.29(2) | 0.57(6) | 2.4737(14) | 495(35) | 3.23 |
| 35 | 38.27(3) | 0.50(3) | 2.3501(17) | 434(27) | 2.83 |
| 36 | 39.068(19) | 0.33(3) | 2.3037(11) | 286(19) | 1.87 |
| 37 | 39.89(3) | 0.41(5) | 2.2580(15) | 240(20) | 1.57 |

The PXRD pattern data of the free base crystal form F-2 is shown below:

| **Peak No.** | **2θ** | **Flex breadth** | **d-value** | **Peak Intensity** | **I/I₀** |
|---|---|---|---|---|---|
| 1 | 5.780(4) | 0.136((3) | 15.279(10) | 20234(387) | 100.00 |
| 2 | 6.092(4) | 0.110(5) | 14.495(9) | 4942(208) | 24.43 |
| 3 | 7.54858 | 0.1600 | 11.70199 | 1256(27) | 6.21 |
| 4 | 9.64871 | 0.1246 | 9.15913 | 9548(61) | 47.19 |
| 5 | 11.35649 | 0.1432 | 7.78534 | 116(15) | 0.57 |
| 6 | 11.90967 | 0.1414 | 7.42494 | 2747(36) | 13.57 |
| 7 | 12.82850 | 0.2282 | 6.89512 | 5157(48) | 25.48 |
| 8 | 15.40875 | 0.1649 | 5.74583 | 2422(35) | 11.97 |
| 9 | 15.76820 | 0.1384 | 5.61565 | 137(18) | 0.68 |
| 10 | 16.85818 | 0.1550 | 5.25494 | 652(26) | 3.22 |
| 11 | 17.46472 | 0.1181 | 5.07378 | 1848(33) | 9.13 |
| 12 | 17.94327 | 0.0803 | 4.93952 | 1225(33) | 6.05 |
| 13 | 18.02760 | 0.0992 | 4.91661 | 2419(40) | 11.96 |
| 14 | 18.69056 | 0.1185 | 4.74368 | 782(29) | 3.86 |
| 15 | 19.07566 | 0.1328 | 4.64878 | 4143(47) | 20.48 |
| 16 | 19.61250 | 0.1746 | 4.52272 | 2446(38) | 12.09 |
| 17 | 20.27436 | 0.0673 | 4.37655 | 251(22) | 1.24 |
| 18 | 20.65610 | 0.2037 | 4.29652 | 10764(73) | 53.20 |
| 19 | 20.93881 | 0.1482 | 4.23914 | 4364(56) | 21.57 |
| 20 | 21.06347 | 0.0777 | 4.21433 | 2246(41) | 11.10 |
| 21 | 21.62491 | 0.1775 | 4.10617 | 8066(62) | 39.87 |
| 22 | 22.33265 | 0.2318 | 3.97762 | 2279(43) | 11.26 |
| 23 | 23.39352 | 0.1655 | 3.79958 | 2490(41) | 12.31 |
| 24 | 24.23688 | 0.0984 | 3.66924 | 1251(31) | 6.18 |
| 25 | 24.67637 | 0.1342 | 3.60488 | 941(38) | 4.65 |
| 26 | 24.78825 | 0.0765 | 3.58886 | 468(31) | 2.31 |
| 27 | 25.18490 | 0.1496 | 3.53323 | 2603(43) | 12.87 |
| 28 | 25.63768 | 0.0794 | 3.47185 | 1122(35) | 5.54 |
| 29 | 25.77871 | 0.1636 | 3.45318 | 1381(42) | 6.82 |
| 30 | 26.42938 | 0.1534 | 3.36962 | 1484(36) | 7.33 |
| 31 | 26.85995 | 0.1382 | 3.31657 | 990(32) | 4.89 |
| 32 | 27.12498 | 0.1310 | 3.28477 | 611(29) | 3.02 |
| 33 | 27.85367 | 0.1783 | 3.20047 | 456(26) | 2.25 |
| 34 | 29.39670 | 0.0730 | 3.03589 | 109(21) | 0.54 |
| 35 | 29.81641 | 0.6181 | 2.99410 | 578(53) | 2.86 |
| 36 | 30.51388 | 0.1557 | 2.92724 | 2951(44) | 14.58 |
| 37 | 30.86484 | 0.1705 | 2.89475 | 1663(38) | 8.22 |
| 38 | 32.30962 | 0.2453 | 2.76852 | 515(28) | 2.54 |
| 39 | 33.10894 | 0.1738 | 2.70349 | 296(23) | 1.46 |
| 40 | 34.07558 | 0.1033 | 2.62898 | 104(18) | 0.51 |
| 41 | 34.99409 | 0.1126 | 2.56205 | 293(24) | 1.45 |
| 42 | 35.55966 | 0.1370 | 2.52258 | 103(23) | 0.51 |
| 43 | 38.34600 | 0.1416 | 2.34535 | 118(23) | 0.58 |
| 44 | 39.85098 | 0.1663 | 2.26027 | 237(22) | 1.17 |

The PXRD pattern data of the free base crystal form F-3 is shown below:

| **Peak No.** | **2θ** | **Flex breadth** | **d-value** | **Peak Intensity** | **I/I₀** |
|---|---|---|---|---|---|
| 1 | 6.247(3) | 0.270(2) | 14.137(6) | 3453(32) | 4.11 |
| 2 | 7.153(8) | 0.288(8) | 12.348(14) | 1035(29) | 1.23 |
| 3 | 8.164(4) | 0.342(4) | 10.821(5) | 5237(63) | 6.24 |
| 4 | 10.24(3) | 1.02(7) | 8.63(3) | 1061(63) | 1.26 |
| 5 | 11.236(5) | 0.301(11) | 7.868(3) | 1520(51) | 1.81 |
| 6 | 11.755(3) | 0.298(4) | 7.522(2) | 6374(79) | 7.59 |
| 7 | 12.566(17) | 0.26(4) | 7.039(9) | 242(33) | 0.29 |
| 8 | 13.291(4) | 0.299(6) | 6.6563(19) | 2482(48) | 2.96 |
| 9 | 14.273(11) | 0.31(3) | 6.201(5) | 705(65) | 0.84 |
| 10 | 14.873(9) | 0.435(18) | 5.952(3) | 4033(157) | 4.80 |
| 11 | 15.406(8) | 0.344(16) | 5.747(3) | 3031(163) | 3.61 |
| 12 | 16.678(8) | 0.374(19) | 5.311(3) | 1884(89) | 2.24 |
| 13 | 17.183(5) | 0.312(12) | 5.1562(14) | 3402(189) | 4.05 |
| 14 | 17.765(17) | 0.38(2) | 4.989(5) | 7667(695) | 9.13 |
| 15 | 18.027(16) | 0.23(3) | 4.917(4) | 2484(655) | 2.96 |
| 16 | 18.624(3) | 0.313(6) | 4.7604(9) | 4888(75) | 5.82 |
| 17 | 19.284(11) | 0.32(2) | 4.599(2) | 2031(144) | 2.42 |
| 18 | 19.685(4) | 0.302(13) | 4.5061(9) | 4933(189) | 5.88 |
| 19 | 20.203(3) | 0.372(4) | 4.3919(6) | 18575(158) | 22.13 |
| 20 | 21.015(3) | 0.353(4) | 4.2239(5) | 21466(248) | 25.57 |
| 21 | 21.395(6) | 0.20(2) | 4.1498(11) | 1753(227) | 2.09 |
| 22 | 21.76(3) | 0.73(7) | 4.081(6) | 3410(383) | 4.06 |
| 23 | 23.154(9) | 0.27(2) | 3.8384(14) | 1037(68) | 1.24 |
| 24 | 24.112(4) | 0.443(4) | 3.6880(6) | 13968(138) | 16.64 |
| 25 | 25.29(8) | 11.14(9) | 3.519(11) | 83936(449) | 100.00 |
| 26 | 25.506(10) | 0.362(9) | 3.4895(13) | 6088(137) | 7.25 |
| 27 | 26.364(14) | 0.27(3) | 3.3778(17) | 2257(564) | 2.69 |
| 28 | 26.721(9) | 0.44(2) | 3.3335(11) | 10656(637) | 12.70 |
| 29 | 27.464(17) | 0.39(4) | 3.245(2) | 1507(150) | 1.80 |
| 30 | 28.106(14) | 0.32(3) | 3.1723(16) | 1011(90) | 1.20 |
| 31 | 30.09(2) | 0.73(8) | 2.967(2) | 2277(247) | 2.71 |
| 32 | 30.885(16) | 0.57(2) | 2.8929(14) | 2943(207) | 3.51 |
| 33 | 31.804(11) | 0.318(19) | 2.8114(10) | 1104(48) | 1.32 |
| 34 | 33.02(4) | 1.25(7) | 2.711(3) | 2463(102) | 2.93 |
| 35 | 34.807(18) | 0.50(4) | 2.5754(13) | 643(47) | 0.77 |
| 36 | 36.37(2) | 0.66(6) | 2.4683(13) | 1102(76) | 1.31 |
| 37 | 36.947(10) | 0.21(3) | 2.4310(6) | 397(57) | 0.47 |
| 38 | 39.84(2) | 0.86(9) | 2.2610(13) | 2025(111) | 2.41 |

The PXRD pattern data of the free base crystal form F-4 is shown below:

| **Peak No.** | **2θ** | **Flex breadth** | **d-value** | **Peak Intensity** | **I/I₀** |
|---|---|---|---|---|---|
| 1 | 6.0036(13) | 0.1932(10) | 14.709(3) | 31030(150) | 100 |
| 2 | 7.231(2) | 0.1791(19) | 12.215(3) | 6537(56) | 21.07 |
| 3 | 7.743(3) | 0.183(4) | 11.408(4) | 2235(64) | 7.2 |
| 4 | 8.06(5) | 0.46(11) | 10.96(6) | 333(80) | 1.07 |
| 5 | 9.293(3) | 0.17 (3) | 9.509(3) | 749(21) | 2.41 |
| 6 | 9.8366(15) | 0.1862(12) | 8.9846(14) | 7973(46) | 25.7 |
| 7 | 11.558(5) | 0.245(11) | 7.650(3) | 629(24) | 2.03 |
| 8 | 12.068(2) | 0.183(3) | 7.3277(14) | 1554(26) | 5.01 |
| 9 | 12.559(10) | 0.173(3) | 7.043(5) | 177(24) | 0.57 |
| 10 | 13.0275(19) | 0.1891(15) | 6.7902(10) | 6240(53) | 20.11 |
| 11 | 15.348(7) | 0.187(11) | 5.768(3) | 1177(58) | 3.79 |
| 12 | 15.633(5) | 0.177(6) | 5.6640(17) | 1785(89) | 5.75 |
| 13 | 15.907(9) | 0.25(3) | 5.567(3) | 842(96) | 2.71 |
| 14 | 17.099(4) | 0.172(10) | 5.1815(13) | 1221(161) | 3.93 |
| 15 | 17.634(4) | 0.229(4) | 5.0255(10) | 5984(103) | 19.28 |
| 16 | 18.233(5) | 0.204(9) | 4.8617(13) | 1756(63) | 5.66 |
| 17 | 18.801(8) | 0.238(16) | 4.7160(9) | 1178(63) | 3.8 |
| 18 | 19.217(4) | 0.189(7) | 4.6149(8) | 2303(65) | 7.42 |
| 19 | 19.911(5) | 0.282(8) | 4.4555(12) | 3583(82) | 11.55 |
| 20 | 20.500(4) | 0.120(11) | 4.3290(8) | 1049(84) | 3.38 |
| 21 | 20.818(6) | 0.333(13) | 4.2635(12) | 5328(159) | 17.17 |
| 22 | 21.237(2) | 0.168(4) | 4.1804(4) | 6867(116) | 22.13 |
| 23 | 21.638(6) | 0.160(13) | 4.1038(12) | 2761(259) | 8.9 |
| 24 | 21.826(5) | 0.151(15) | 4.0689(9) | 3307(318) | 10.66 |
| 25 | 22.03(2) | 0.14(4) | 4.031(4) | 459(130) | 1.48 |
| 26 | 22.447(2) | 0.175(5) | 3.9576(4) | 4027(90) | 12.98 |
| 27 | 22.745(4) | 0.192(10) | 3.9064(7) | 2186(85) | 7.05 |
| 28 | 23.234(3) | 0.220(8) | 3.8253(5) | 2976(86) | 9.59 |
| 29 | 23.612(5) | 0.224(16) | 3.7650(9) | 1728(90) | 5.57 |
| 30 | 23.898(8) | 0.09(2) | 3.7205(12) | 352(53) | 1.13 |
| 31 | 24.990(6) | 0.136(9) | 3.5604(8) | 1161(47) | 3.74 |
| 32 | 25.455(6) | 0.162(5) | 3.4963(8) | 4159(83) | 13.4 |
| 33 | 25.941(2) | 0.1479(16) | 3.4319(3) | 8559(54) | 27.58 |
| 34 | 26.630(11) | 0.12(2) | 3.3447(13) | 214(21) | 0.69 |
| 35 | 27.059(13) | 0.197(15) | 3.2926(16) | 715(35) | 2.3 |
| 36 | 27.369(9) | 0.143(10) | 3.2561(11) | 655(41) | 2.11 |
| 37 | 28.051(15) | 0.196(11) | 3.1784(16) | 850(53) | 2.74 |
| 38 | 29.36(4) | 0.27(10) | 3.040(4) | 277(106) | 0.89 |
| 39 | 29.598(6) | 0.146(10) | 3.0157(6) | 1038(85) | 3.35 |
| 40 | 30.014(8) | 0.172(14) | 2.9748(7) | 835(32) | 2.69 |
| 41 | 30.372(8) | 0.16(2) | 2.9406(7) | 480(53) | 1.55 |
| 42 | 30.732(8) | 0.239(8) | 2.9070(8) | 2340(80) | 7.54 |
| 43 | 31.136(7) | 0.199(13) | 2.8701(7) | 1066(66) | 3.44 |
| 44 | 32.55(3) | 0.18(2) | 2.749(2) | 364(45) | 1.17 |
| 45 | 33.262(15) | 0.10(2) | 2.6914(12) | 367(36) | 1.18 |
| 46 | 35.22(3) | 0.26(5) | 2.546(2) | 721(56) | 2.32 |
| 47 | 36.41(2) | 0.11(3) | 2.4657(15) | 170(36) | 0.55 |
| 48 | 38.15(3) | 0.22(5) | 2.357(2) | 404(42) | 1.3 |
| 49 | 39.143(13) | 0.13(3) | 2.2995(7) | 208(34) | 0.67 |

The PXRD pattern data of the free base crystal form F-5 is shown below:

| **Peak No.** | **2θ** | **Flex breadth** | **d-value** | **Peak Intensity** | **I/I₀** |
|---|---|---|---|---|---|
| 1 | 5.894(3) | 0.074(6) | 14.982(8) | 1108(114) | 1.26 |
| 2 | 5.9824(12) | 0.056(2) | 14.762(3) | 6474(794) | 7.34 |
| 3 | 6.0432(12) | 0.076(4) | 14.613(3) | 13000(871) | 14.74 |
| 4 | 6.1391(15) | 0.072(3) | 14.385(4) | 9489(330) | 10.76 |
| 5 | 6.342(2) | 0.144(9) | 13.925(5) | 8053(512) | 9.13 |
| 6 | 6.4332(17) | 0.056(8) | 13.728(4) | 1430(400) | 1.62 |
| 7 | 7.16(3) | 0.25(3) | 12.34(4) | 1545(104) | 1.75 |
| 8 | 8.189(5) | 0.173(5) | 10.789(7) | 10485(171) | 11.89 |
| 9 | 9.829(13) | 0.17(2) | 8.992(12) | 852(114) | 0.97 |
| 10 | 9.922(5) | 0.063(18) | 8.907(5) | 229(111) | 0.26 |
| 11 | 10.363(9) | 0.208(12) | 8.529(7) | 785(38) | 0.89 |
| 12 | 10.891(18) | 0.24(4) | 8.117(14) | 284(39) | 0.32 |
| 13 | 11.309(7) | 0.191(8) | 7.818(5) | 1295(59) | 1.47 |
| 14 | 11.8463(12) | 0.0867(9) | 7.4645(7) | 23582(290) | 26.73 |
| 15 | 11.984(4) | 0.050(12) | 7.379(2) | 627(145) | 0.71 |
| 16 | 12.109(5) | 0.116(14) | 7.303(3) | 1289(175) | 1.46 |
| 17 | 12.18(9) | 0.193(16) | 7.26(5) | 1489(130) | 1.69 |
| 18 | 12.721(8) | 0.26(2) | 6.953(4) | 1054(61) | 1.19 |
| 19 | 13.054(7) | 0.10(2) | 6.777(4) | 265(40) | 0.30 |
| 20 | 13.355(4) | 0.209(7) | 6.6247(18) | 2641(64) | 2.99 |
| 21 | 14.302(6) | 0.176(16) | 6.188(2) | 745(58) | 0.84 |
| 22 | 14.853(3) | 0.401(10) | 5.9596(14) | 4928(132) | 5.59 |
| 23 | 15.454(3) | 0.245(10) | 5.7293(12) | 2458(78) | 2.79 |
| 24 | 16.087(16) | 0.11(4) | 5.505(5) | 130(41) | 0.15 |
| 25 | 16.544(16) | 0.10(3) | 5.354(5) | 563(181) | 0.64 |
| 26 | 16.663(13) | 0.09(4) | 5.316(4) | 569(291) | 0.64 |
| 27 | 16.810(14) | 0.16(3) | 5.270(4) | 1110(181) | 1.26 |
| 28 | 17.249(7) | 0.213(9) | 5.1368(19) | 2447(84) | 2.77 |
| 29 | 17.754(4) | 0.173(8) | 4.9917(10) | 4428(223) | 5.02 |
| 30 | 18.036(6) | 0.266(14) | 4.9142(16) | 4872(228) | 5.52 |
| 31 | 18.701(5) | 0.182(5) | 4.7412(13) | 4534(103) | 5.14 |
| 32 | 19.413(9) | 0.183(11) | 4.569(2) | 4577(190) | 5.19 |
| 33 | 19.802(7) | 0.134(9) | 4.4798(15) | 4333(175) | 4.91 |
| 34 | 20.266(7) | 0.169(7) | 4.3783(15) | 11831(306) | 13.41 |
| 35 | 21.111(5) | 0.164(4) | 4.2050(10) | 25783(959) | 29.23 |
| 36 | 21.423(13) | 0.21(4) | 4.144(2) | 3729(702) | 4.23 |
| 37 | 21.864(13) | 0.16(4) | 4.062(2) | 1037(174) | 1.18 |
| 38 | 22.20(4) | 0.21(11) | 4.001(7) | 396(163) | 0.45 |
| 39 | 22.28(10) | 13.91(8) | 3.987(17) | 88220(586) | 100.00 |
| 40 | 23.227(11) | 0.175(10) | 3.8265(18) | 2474(89) | 2.80 |
| 41 | 23.979(4) | 0.152(7) | 3.7081(6) | 5721(187) | 6.49 |
| 42 | 24.291(5) | 0.183(7) | 3.6612(8) | 11152(234) | 12.64 |
| 43 | 24.887(8) | 0.15(4) | 3.5749(12) | 1058(123) | 1.20 |
| 44 | 25.652(5) | 0.176(4) | 3.4699(6) | 9362(294) | 10.61 |
| 45 | 25.961(9) | 0.21(4) | 3.4293(12) | 1474(224) | 1.67 |
| 46 | 26.490(2) | 0.113(5) | 3.3621(3) | 3777(110) | 4.28 |
| 47 | 26.839(5) | 0.273(5) | 3.3191(6) | 10042(135) | 11.38 |
| 48 | 27.551(6) | 0.233(11) | 3.2349(7) | 2163(73) | 2.45 |
| 49 | 28.069(11) | 0.17(4) | 3.1764(12) | 593(107) | 0.67 |
| 50 | 28.293(7) | 0.150(12) | 3.1518(7) | 1321(98) | 1.50 |
| 51 | 29.544(10) | 0.102(13) | 3.0210(10) | 469(42) | 0.53 |
| 52 | 30.09(2) | 0.18(3) | 2.968(2) | 912(74) | 1.03 |
| 53 | 30.922(11) | 0.18(2) | 2.8895(10) | 3356(355) | 3.80 |
| 54 | 31.212(17) | 0.19(7) | 2.8634(15) | 1377(411) | 1.56 |
| 55 | 31.485(19) | 0.12(8) | 2.8391(17) | 390(180) | 0.44 |
| 56 | 31.948(3) | 0.101(6) | 2.7990(3) | 1550(48) | 1.76 |
| 57 | 32.59(2) | 0.08(2) | 2.7453(17) | 136(30) | 0.15 |
| 58 | 33.18(2) | 0.72(4) | 2.6978(17) | 2417(103) | 2.74 |
| 59 | 34.902(6) | 0.124(9) | 2.5686(4) | 1336(43) | 1.51 |
| 60 | 35.214(12) | 0.21(3) | 2.5466(9) | 495(49) | 0.56 |
| 61 | 36.258(6) | 0.111(8) | 2.4756(4) | 903(61) | 1.02 |
| 62 | 36.57(2) | 0.29(5) | 2.4549(13) | 535(53) | 0.61 |
| 63 | 37.025(8) | 0.10(2) | 2.4261(5) | 309(63) | 0.35 |
| 64 | 37.153(10) | 0.11(2) | 2.4180(6) | 343(62) | 0.39 |
| 65 | 38.35(2) | 0.11(6) | 2.3450(14) | 58(17) | 0.07 |
| 66 | 38.912(10) | 0.138(12) | 2.3126(6) | 365(33) | 0.41 |
| 67 | 39.165(14) | 0.13(4) | 2.2982(8) | 107(22) | 0.12 |

The PXRD pattern data of the free base crystal form F-6 is shown below:

| **Peak No.** | **2θ** | **Flex breadth** | **d-value** | **Peak Intensity** | **I/I₀** |
|---|---|---|---|---|---|
| 1 | 5.604(4) | 0.194(3) | 15.757(10) | 50453(537) | 85.19 |
| 2 | 6.918(2) | 0.155(7) | 12.766(4) | 1842(48) | 3.11 |
| 3 | 8.099(11) | 0.157(14) | 10.909(15) | 930(33) | 1.57 |
| 4 | 9.959(5) | 0.358(15) | 8.875(4) | 1378(62) | 2.33 |
| 5 | 11.369(9) | 0.170(10) | 7.777(6) | 1980(51) | 3.34 |
| 6 | 12.098(3) | 0.198(3) | 7.310(2) | 3858(68) | 6.51 |
| 7 | 12.715(18) | 0.42(4) | 6.956(10) | 543(48) | 0.92 |
| 8 | 13.365(6) | 0.208(13) | 6.619(3) | 470(25) | 0.79 |
| 9 | 14.036(10) | 0.111(13) | 6.304(4) | 482(26) | 0.81 |
| 10 | 14.69(2) | 0.137(19) | 6.027(9) | 252(27) | 0.43 |
| 11 | 15.171(6) | 0.159(7) | 5.835(2) | 771(37) | 1.3 |
| 12 | 15.488(9) | 0.228(13) | 5.717(3) | 819(36) | 1.38 |
| 13 | 16.40(2) | 0.09(2) | 5.401(8) | 234(47) | 0.4 |
| 14 | 16.786(2) | 0.122(4) | 5.2773(7) | 4605(78) | 7.78 |
| 15 | 17.152(3) | 0.193(3) | 5.1657(9) | 10826(143) | 18.28 |
| 16 | 17.419(3) | 0.147(9) | 5.0870(10) | 2194(108) | 3.71 |
| 17 | 17.793(2) | 0.147(3) | 4.9808(7) | 7991(80) | 13.49 |
| 18 | 18.640(13) | 0.25(4) | 4.756(3) | 863(106) | 1.46 |
| 19 | 19.073(12) | 0.276(14) | 4.649(3) | 2096(133) | 3.54 |
| 20 | 20.092(6) | 0.208(13) | 4.4158(14) | 452(23) | 0.76 |
| 21 | 20.548(4) | 0.165(7) | 4.3190(8) | 650(23) | 1.1 |
| 22 | 21.034(6) | 0.294(19) | 4.2202(12) | 1273(71) | 2.15 |
| 23 | 21.334(2) | 0.172(2) | 4.1614(4) | 4734(75) | 7.99 |
| 24 | 22.048(8) | 0.13(2) | 4.0282(14) | 325(37) | 0.55 |
| 25 | 22.597(10) | 0.20(3) | 3.9317(17) | 569(79) | 0.96 |
| 26 | 23.028(4) | 0.209(4) | 3.8590(6) | 4944(69) | 8.35 |
| 27 | 23.467(4) | 0.197(4) | 3.7879(6) | 5104(76) | 8.62 |
| 28 | 23.77(8) | 11.48(7) | 3.740(13) | 59223(412) | 100 |
| 29 | 24.003(3) | 0.177(4) | 3.7044(5) | 3164(53) | 5.34 |
| 30 | 24.533(3) | 0.192(4) | 3.6256(5) | 3334(61) | 5.63 |
| 31 | 25.057(5) | 0.187(11) | 3.5510(7) | 1057(45) | 1.78 |
| 32 | 25.562(4) | 0.170(7) | 3.4819(5) | 1425(45) | 2.41 |
| 33 | 26.07(2) | 0.26(6) | 3.416(3) | 292(49) | 0.49 |
| 34 | 27.101(15) | 0.10(6) | 3.2876(18) | 242(90) | 0.41 |
| 35 | 27.39(2) | 0.16(4) | 3.254(2) | 648(103) | 1.09 |
| 36 | 28.436(10) | 0.07(3) | 3.1362(11) | 104(30) | 0.18 |
| 37 | 28.855(14) | 0.41(2) | 3.0917(15) | 1609(62) | 2.72 |
| 38 | 29.286(5) | 0.141(6) | 3.0471(5) | 1449(33) | 2.45 |
| 39 | 30.49(2) | 0.22(4) | 2.9292(19) | 514(49) | 0.87 |
| 40 | 30.776(7) | 0.089(10) | 2.9029(6) | 437(44) | 0.74 |
| 41 | 31.625(18) | 0.213(14) | 2.8269(16) | 738(34) | 1.25 |
| 42 | 32.80(2) | 0.21(3) | 2.7285(18) | 944(42) | 1.59 |
| 43 | 33.91(3) | 0.20(3) | 2.641(3) | 379(37) | 0.64 |
| 44 | 34.914(16) | 0.42(4) | 2.5678(12) | 380(34) | 0.64 |
| 45 | 36.041(6) | 0.218(17) | 2.4900(4) | 497(32) | 0.84 |
| 46 | 37.44(5) | 0.25(6) | 2.400(3) | 174(34) | 0.29 |
| 47 | 37.84(2) | 0.11(3) | 2.3754(14) | 106(25) | 0.18 |
| 48 | 38.946(18) | 0.083(17) | 2.3107(10) | 70(18) | 0.12 |
| 49 | 39.838(11) | 0.12(3) | 2.2610(6) | 201(22) | 0.34 |

### (3) Systematic characterization of free base crystal forms

### Comparison of free base crystal from properties (1)

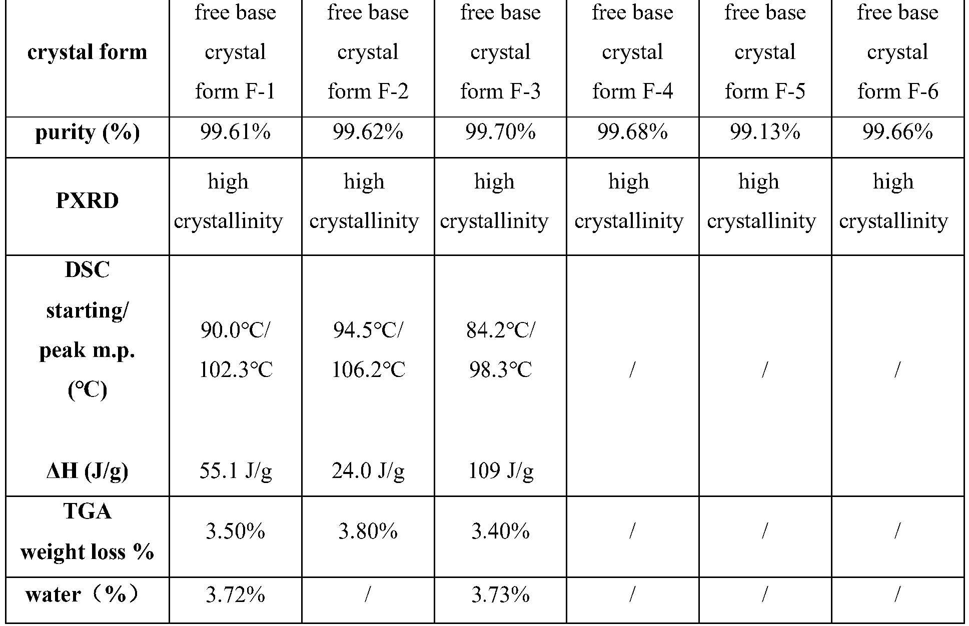

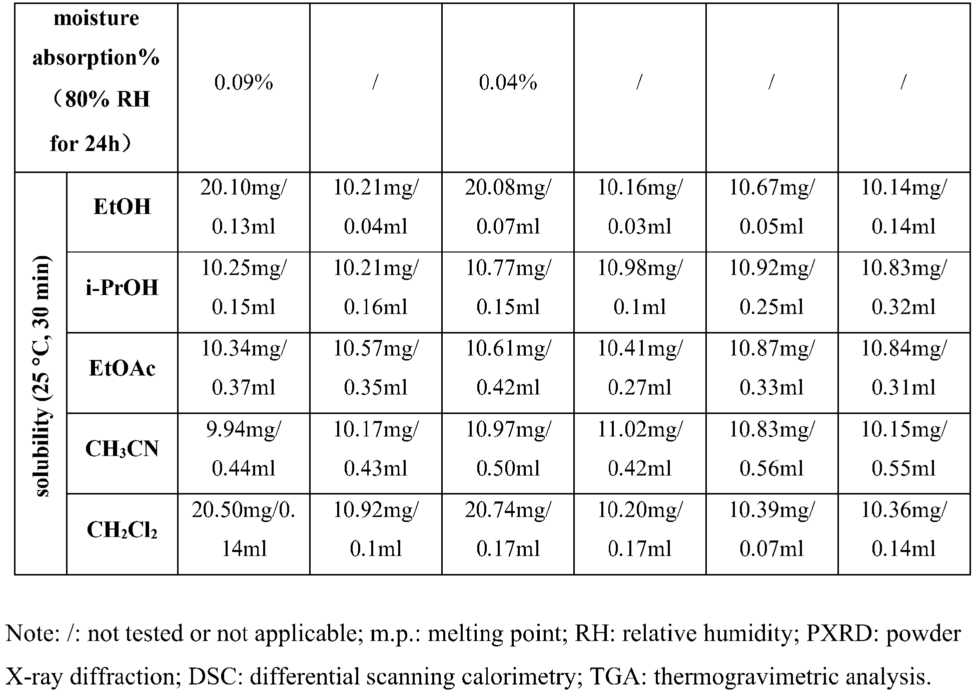

### Comparison of free base crystal form properties (2)

| **crystal form** | | free base crystal form F-1 | | free base crystal form F-3 | | free base crystal form F-4 | |
|---|---|---|---|---|---|---|---|
| | **at day** | 0 | 23 | 0 | 23 | 0 | 23 |
| | **purity** | 99.61% | 99.61% | 99.70% | 99.68% | 99.68% | 99.65% |
| **solid stability** | RRT= 0.29 | 0.06% | 0.06% | 0.07% | 0.08% | 0.06% | 0.06% |
| **(sealed centrifuge tube; kept at RT)** | RRT= 0.36 | 0.02% | 0.01% | 0.02% | 0.04% | 0.02% | 0.01% |
| | RRT= 0.62 | 0.03% | 0.03% | 0.03% | 0.03% | 0.01% | 0.03% |
| | RRT= 0.64 | 0.05% | 0.05% | 0.04% | 0.04% | 0.04% | 0.04% |
| | RRT= 0.78 | 0.06% | 0.08% | 0.02% | 0.03% | 0.02% | 0.03% |
| | | | | | | | +0.01% |
| | RRT= 0.89 | 0.04% | 0.05% | 0.03% | 0.03% | 0.07% | 0.05% |
| | RRT= 0.91 | 0.035% | 0.03% | 0.02% | 0.01% | | 0.02% |

| | | | | | | | |
|---|---|---|---|---|---|---|---|
| Note: RT: room temperature; RRT: relative retention time. | | | | | | | |

| **crystal form** | | free base crystal form F-5 | | free base crystal form F-6 | |
|---|---|---|---|---|---|
| | **at day** | 0 | 23 | 0 | 23 |
| | **purity** | 99.13% | 99.06% | 99.66% | 99.63% |
| **solid stability** | RRT= 0.29 | 0.01% | 0.02% | 0.04% | 0.03% |
| **(sealed centrifuge tube;** | RRT= 0.36 | 0.01% | 0.00% | 0.01% | 0.00% |
| | RRT= 0.62 | 0.53% | 0.53% | 0.03% | 0.06% |
| | RRT= | 0.05% | 0.07% | 0.05% | 0.05% |
| **kept at RT)** | 0.64 | | | | |
| | RRT= 0.78 | 0.12% | 0.08% | 0.05% | 0.05% |
| | | | +0.06% | | +0.01% |
| | RRT= 0.89 | 0.07% | 0.04% | 0.06% | 0.04% |
| | RRT= 0.91 | | 0.03% | | 0.02% |

| | | | | | |
|---|---|---|---|---|---|
| Note: RT: room temperature; RRT: relative retention time | | | | | |

### (4) Competition test for free base crystal forms

The three free base crystal forms (F-1, F-2, and F-3) were mixed and stirred in different solvents. The crystal forms did not change in water and isopropanol, but were converted into crystal form F-3 in other solvents (such as n-heptane, ethyl acetate, and a 1:1 mixture of n-heptane/ethyl acetate, 90% ethanol, and 90% isopropanol).

### Example 11. Solubility test of different crystal forms of (E)-4-(dimethylamino)-N-(4-((4-(4-fluoro-phenoxy)phenyl)amino)-7-ethoxyquinazolin-6-yl)but-2-enamide

Solubility study refers to the general rules of Part II of the 2020 edition of the Chinese Pharmacopoeia

| **crystal form** | | maleate crystal form C | maleate crystal form A | hydrochloride crystal form | sulfate crystal form |
|---|---|---|---|---|---|
| **solubility (25 °C, 30 min)** | i-PrOH | <0.1 mg/mL | >0.232 mg/mL | <0.1 mg/mL | <0.1 mg/mL |

### Example 12 Stability Test of (E)-4-(Dimethylamino)-N-(4-((4-(4-fluoro-phenoxy)phenyl)amino)-7-ethoxyquinazolin-6-yl)but-2-enamide Maleic Acid Form A

Solid Stability Study - Established with reference to the General Rules 0512 of Part IV of the 2020 edition of the Chinese Pharmacopoeia
1. Instrument: Agilent HPLC, Agilent-1100
2. Chromatography conditions
Column: YMC Triart C18, 4.6×250 mm, 5µm
Mobile phase A: 0.02 mol/L ammonium acetate buffer (1.54 g ammonium acetate dissolved in 1000 mL water; 1 mL triethylamine added; and the pH adjusted to 6.00 ± 0.05 with acetic acid)
Mobile phase B: methanol-acetonitrile (13:87)
Detection wavelength: 250 nm
Flow rate: 1.0 mL/min
Injection volume: 15 µL
Column temperature: 30 °C
Run time: 75 minutes
Elution gradient:

| **time (min)** | **mobile phase A(%)** | **mobile phase B(%)** |
|---|---|---|
| 0 | 55 | 45 |
| 25 | 55 | 45 |
| 40 | 20 | 80 |
| 65 | 20 | 80 |
| 66 | 55 | 45 |
| 75 | 55 | 45 |

3. Solution Preparation
Blank solution (diluent): methanol-acetonitrile-water (9:6:10)
Test sample solution: Weigh an appropriate amount of the test sample; dissolve it in the diluent prepared in 3; and dilute to a solution containing about 0.5 mg test sample per 1 mL.

4. Calculation
Purity of individual impurities and the main peak was calculated by area normalization method.
(1) In the crystal formation experiment, maleate, hydrochloride, and sulfate were able to obtain solids, among which the maleate obtained two different crystal forms, both of which had good crystallinity;
(2) In the stability experiment, after 21 days at room temperature, the maleate crystal form C showed obvious degradation, and its major impurity increased significantly. The maleate salt A had better stability.

### Example 13. Investigation of different crystal forms of different acid salts of (E)-4-(dimethylamino)-N-(4-((4-(4-fluoro-phenoxy)phenyl)amino)-7-ethoxyquinazolin-6-yl)but-2-enamide

### (1) Scale-up preparation

The (E)-4-(dimethylamino)-N-(4-((4-(4-fluoro-phenoxy)phenyl)amino)-7-ethoxyquinazolin-6-yl)but-2-enamide hydrochloride form S-1, sulfate form S-3, maleate forms A to C, fumarate form S-7, and p-toluenesulfonate form S-8 were scaled up, respectively.

### (2) Systematic characterization

### Comparison of properties of different salt crystal forms (1)

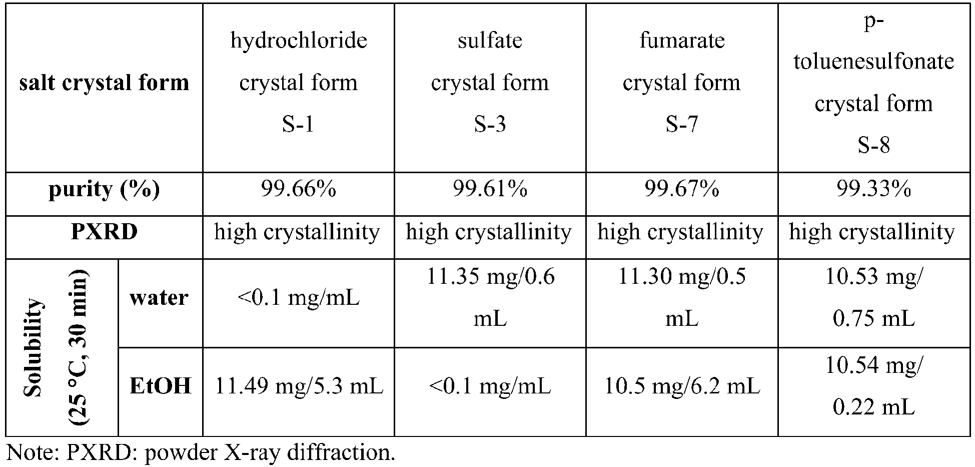

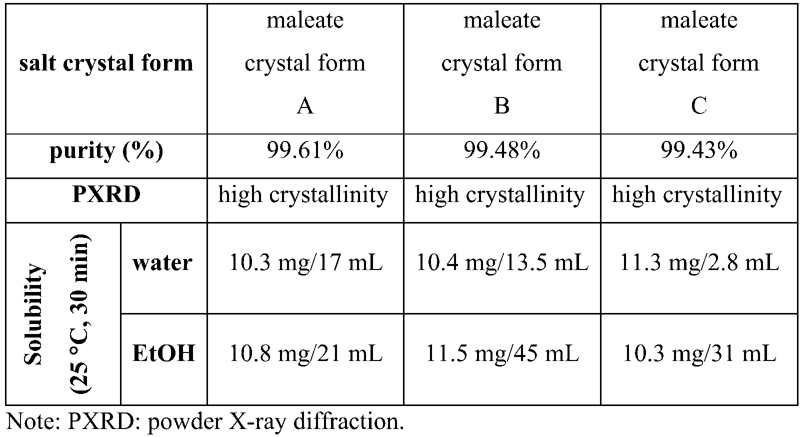

### Comparison of properties of different salt crystal forms (2)

| crystal form | | hydrochloride crystal form S-1 | | sulfate crystal form S-3 | | fumarate crystal form S-7 | | p-toluenesulfonate crystal form S-8 | |
|---|---|---|---|---|---|---|---|---|---|
| | at day | 0 | 24 | 0 | 24 | 0 | 24 | 0 | 24 |
| | purity | 99.66% | 99.66% | 99.61% | 99.54% | 99.67% | 99.51% | 99.33% | 99.77% |
| **solid stability** | RRT= 0.19 | ND | ND | ND | ND | ND | ND | ND | ND |
| **(sealed centrifuge tube; kept at RT)** | RRT= 0.29 | 0.05% | 0.05% | 0.06% | 0.06% | 0.04% | 0.30% | 0.04% | 0.06% |
| | RRT= 0.36 | 0.01% | ND | ND | ND | ND | ND | 0.01% | 0.02% |
| | RRT= 0.78 | 0.05% | 0.05% | 0.06% | 0.08% | 0.05% | 0.05% | 0.07% | 0.19% |
| | RRT= 0.89 | 0.06% | 0.07% | 0.07% | 0.07% | 0.07% | 0.07% | 0.07% | 0.07% |

| | | | | | | | | | |
|---|---|---|---|---|---|---|---|---|---|
| Note: RT: room temperature; RRT: relative retention time. | | | | | | | | | |

| **crystal form** | | maleate crystal form A | | maleate crystal form B | | maleate crystal form C | |
|---|---|---|---|---|---|---|---|
| | **at day** | 0 | 24 | 0 | 24 | 0 | 24 |
| | **purity** | 99.61% | 99.59% | 99.48% | 99.49% | 99.43% | 98.79% |
| **solid stability** | RRT= 0.19 | ND | ND | ND | ND | 0.08% | 0.16% |
| **(sealed centrifuge tube; kept at RT)** | RRT= 0.29 | 0.07% | 0.08% | 0.07% | 0.07% | 0.06% | 0.06% |
| | RRT= 0.36 | ND | ND | 0.06% | 0.06% | 0.01% | 0.03% |
| | RRT= 0.78 | 0.02% | 0.02% | 0.07% | 0.07% | 0.08% | 0.08% |
| | RRT= 0.89 | 0.06% | 0.07% | 0.08% | 0.08% | 0.07% | 0.07% |

| | | | | | | | |
|---|---|---|---|---|---|---|---|
| Note: RT: room temperature; RRT: relative retention time; ND: not detected. | | | | | | | |

### Results of competition between maleate crystal form A and maleate crystal form B as a stirred suspension in different solvents

| **solvent system** | **Amount of crystal form A** | **Amount of crystal form B** | **crystal form after the competition** |
|---|---|---|---|
| water | 100.8 mg | 100.8 mg | crystal form A |
| 90% EtOH | 101.6 mg | 101.8 mg | crystal form A |

Conclusion: Completely converted to crystal form A in water and 90% ethanol.

### Biological test Example 1: Enzymatic activity of (E)-4-(dimethylamino)-N-(4-((4-(4-fluoro-phenoxy)phenyl)amino)-7-ethoxyquinazolin-6-yl)but-2-enamide maleate hydrate crystal form A

### Homogeneous time-resolved fluorescence (HTRF) kinase assay

1) Use assay buffer to prepare 2× ATP & substrate solution and 2× kinase solution.
2) Transfer 50 nL of the compound to a 384-well assay plate using an Echo 655. After centrifugation, add 2.5 µL of 2× kinase solution to the 384-well assay plate, and incubate at 25 °C for 10 minutes.
3) Add 2.5 µL of 2× ATP & substrate solution to the well, and incubate for 40-50 minutes.
4) Use assay buffer to prepare 2× XL665 & antibody solution.
5) Add 5 µL of kinase detection reagent to the well, and incubate at 25 °C for 60 minutes.
6) Read the fluorescence signals at 620 nm (cryptate) and 665 nm (XL665) using a microtiter-plate reader.
7) IC₅₀ or IC₉₀ values were calculated by fitting the % inhibition values and the log values of the compound concentration to a nonlinear regression method (dose response - variable slope) using GraphPad 8.0.
8) $Y = Bottom + \left(Top-Bottom\right) / \left(1+{10}^{∧}\left(\left(LogIC50-X\right)*hillslope\right)\right) ;$ X: log value of the inhibitor concentration; Y: % inhibition

### Enzymatic activity data of (E)-4-(dimethylamino)-N-(4-((4-(4-fluoro-phenoxy)phenyl)amino)-7-ethoxyquinazolin-6-yl)but-2-enamide maleate hydrate crystal form A:

| enzymatic activity | IC₅₀ (nM) |
|---|---|
| HER2 | 1.299 |
| EGFR (D770_N771insNPG) | 5.993 |

| enzymatic activity (IC₉₀) | crystal form A | afatinib |
|---|---|---|
| HER2 | 8.027 nM | 37.14 nM |
| EGFR (D770_N771insNPG) | 171.6 nM | 6.463 nM |
| EGFR (T790M) | 45.42 nM | 2.497 nM |
| HER2 (V777-G778insCG) | 152.1 nM | |
| B-cell lymphocyte kinase (BLK) | 145.1 nM | |

### Biological test Example 2: Cell biology experiment of (E)-4-(dimethylamino)-N-(4-((4-(4-fluoro-phenoxy)phenyl)amino)-7-ethoxyquinazolin-6-yl)but-2-enamide maleate hydrate crystal form A

### 1. Cell seeding

1) Prepare a complete medium: Add fetal bovine serum (FBS) and appropriate additives according to the information sheet provided by the vendor. Mix gently.
2) Check the cell name, complete medium, and passage number marked on the culture flask.
3) Use a vacuum pump to remove and discard the culture medium.
4) Rinse the cell layer briefly with 0.25% (w/v) trypsin-0.038% (w/v) ethylenediaminetetraacetic acid (EDTA) solution to remove all traces of serum that contains trypsin inhibitors.
5) Add 3.0 mL of trypsin-EDTA solution to the culture flask, and observe the cells under an inverted microscope until the cell layer is dispersed.
6) Add 8.0 mL of the complete medium, and aspirate the cells by gently pipetting.
7) Transfer the cell suspension to a centrifuge tube, and centrifuge at 1,000 revolutions per minute (rpm) for 5 minutes.
8) Discard the supernatant using a vacuum pump.
9) Add an appropriate volume of complete medium, and suspend the cell pellet by gently pipetting.
10) Count the cells with Vi-cell XR Cell Viability Analyzer, and adjust the cells to an appropriate density.
11) Add 100 µL of cell suspension to each well of a 96-well opaque-walled, clear-bottom plate according to the planned plate layout, and place the plate in a CO₂ incubator overnight. For SW620 cells, place the plate in the incubator without CO₂.
12) Plate Layout:

### 2. Preparation of compound plates and addition of compounds

The information of the compounds is shown in the following table

### Compound preparation

| **Cmpd No.** | **Conc. (mM)** | **Source** | **Diluent** | **Initial conc. (mM)** | **Instruction** | **Final conc. (µM)** |
|---|---|---|---|---|---|---|
| staurosporine | 2 | Sellect | DMSO | 0.4 | 3× serial dilution, 9 parts of diluent | 2 |
| maleate hydrate crystal form A | 10 | Teligene Ltd | DMSO | 2 | 3× serial dilution, 9 parts of diluent | 10 |

| | | | | | | |
|---|---|---|---|---|---|---|
| Note: Cmpd: compound; Conc.: concentration. | | | | | | |

### 1) Preparation of testing sample plate:

Compounds were prepared at 10 mM in DMSO. According to information of the table above, a starting working solution (2 mM) and three-step, 10-fold (1:10) serial dilution were prepared in DMSO.

### 2) Preparation of staurosporine plate:

Prepare staurosporine in DMSO at 0.4 mM working concentration.

### 3) Addition of compounds:

Transfer 0.5 µL of diluted compound to a well containing 100 µL of culture medium. The total dilution factor is 200-fold.

The cells were incubated with the compounds at 5% CO₂ for 3 days at 37 °C. For SW620 cells, the cells were incubated with the compounds in a CO₂-free incubator.

### 3. Reagent preparation

1) Thaw CellTiter-Glo buffer and equilibrate to room temperature before use.
2) Equilibrate the lyophilized CellTiter-Glo substrate to room temperature before use.
3) Reconstitute the lyophilized enzyme/substrate mixture by transferring an appropriate volume of CellTiter-Glo buffer to the amber bottle containing CellTiter-Glo Substrate. This forms the CellTiter-Glo reagent.
4) Gently vortex, rotate, or invert the contents in the amber bottle to mix the solution and obtain a homogenous solution. The CellTiter-Glo Substrate dissolves easily within one minute.

### 4. Quantification of the assay

1) After the above corresponding treatments, observe cell morphology using an inverted microscope.
2) Allow the plate and its contents to equilibrate to room temperature for about 30 minutes.
3) Add 100 µL of CellTiter-Glo reagent to the assay plate using a Multidrop Combi instrument.
4) Mix the contents on an orbital shaker for 10 minutes to induce cell lysis.
5) Incubate the plate at room temperature for 10 minutes to stabilize the luminescent signal.
6) Seal the transparent bottom with white backing tape and record the luminescence using Envision. The settings are: luminescence, measurement time 0.1 milliseconds.

| **Cell line** | **Cell source** | **IC₅₀ (µM)** |
|---|---|---|
| BT-474 (HER2+) | ATCC | 0.1484 |
| HCC1954 (HER2+) | ATCC | 0.1325 |
| SK-BR-3 (HER2+) | ATCC | 0.0111 |

Under similar cell experimental conditions, the EC₅₀ of the compound of the present disclosure: (E)-4-(dimethylamino)-N-(4-((4-(4-fluoro-phenoxy)phenyl)amino)-7-ethoxyquinazolin-6-yl)but-2-enamide in the HCC1419 cell proliferation inhibition experiment was 0.016 µg/mL.

A compound with an analogous structure to the compound of the present disclosure: (E)-4-(dimethylamino)-N-(4-((4-(4-chloro-phenoxy)phenyl)amino)-7-methoxyquinazolin-6-yl)but-2-enamide has an IC₅₀ of 0.221 µM in the SK-BR-3 cell proliferation inhibition experiment.

### Biological test Example 3: In vivo efficacy study of (E)-4-(dimethylamino)-N-(4-((4-(4-fluoro-phenoxy)phenyl)amino)-7-ethoxyquinazolin-6-yl)but-2-enamide in mice

### Tumor cell lines

The BT-474 human breast cancer cell line (HTB-20^{™}) purchased from ATCC was maintained in vitro in Roswell Park Memorial Institute (RPMI) 1640 medium supplemented with 10% heat-inactivated fetal calf serum, 100 U/mL penicillin, 100 µg/mL streptomycin, 1 mM Napyr, 1× MEM-NEAA, 1× MEM vitamins, and 10 µg/mL human insulin at 37 °C in an atmosphere of 5% CO₂ in air. The Tumor cells are routinely subcultured every 5-7 days. The cells in an exponential phase are harvested, counted, and used for tumor inoculation.

### Tumor inoculation and treatment

Tumor cells were suspended in PBS + Matrigel (volume ratio 1:1) and inoculated subcutaneously at the right flank of CB17/SCID mice with 100 µL inoculated per mouse. Estradiol benzoate injection (Ningbo Second Hormone Factory) was administered subcutaneously one day before the inoculation and once a week thereafter, at 40 µg/mouse each time. When the average tumor volume reached about 100 mm³ , the mice were randomized to five groups of eight mice each based on the tumor volumes, and test articles were administered to the mice according to a predetermined regimen as shown in the experiment design table (p.o. QD×21, i.e., oral administration, once a day for 21 consecutive days).

### Tumor measurements and the endpoints

Tumor was measured twice a week, and tumor volume (TV) was calculated according to the formula: TV = 0.5 × long diameter × short diameter².

The T/C values were calculated from the tumor volume, where T is the average of the relative tumor volume (RTV) of each treatment group, and C is the average of the relative tumor volume (RTV) of the vehicle control group. RTV is the ratio of the post-dose tumor volume to the pre-dose tumor volume.

### a. Tumor growth inhibition rate (%)

$TGI \left(tumor growth inhibition rate\right) = \left(1-T/C\right) \times 100 %$$T / C = RTV of the treatment group / RTV of the control group$

The treatment is considered effective when the tumor growth inhibition rate is ≥60% and statistically significant (p<0.05).

### b. Tumor volume ratio T/C (%) of treatment group/control group

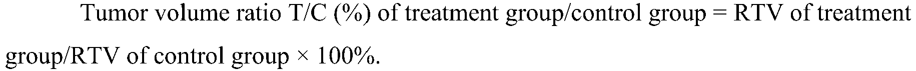

Tumor volume ratio T/C (%) of treatment group/control group = RTV of treatment group/RTV of control group × 100%.

Evaluation standard of curative effect: T/C (%)>40% is considered ineffective, T/C (%)≤40% and p<0.05 is considered effective.

Compounds tested: (E)-4-(dimethylamino)-N-(4-((4-(4-fluoro-phenoxy)phenyl)amino)-7-ethoxyquinazolin-6-yl)but-2-enamide maleate hydrate crystal form A, neratinib (ML900179);
Treatment with maleate hydrate crystal form A (ML900110, 10, 20, and 40 mg/kg, once daily for 21 consecutive days) and neratinib (20 mg/kg, once daily for 21 consecutive days) resulted in 100%, 100%, 100%, and 95% tumor growth inhibition (TGI), respectively. At the end of treatment, all mice treated with (10, 20, and 40 mg/kg) achieved complete tumor regression, while the majority (87.5%) of mice treated with neratinib (20 mg/kg) also achieved complete tumor regression. Maleate hydrate crystal form A exhibited superior antitumor efficacy at a dose of 10 mg/kg compared to neratinib at a dose of 20 mg/kg. Therefore, in an oral efficacy study in mice, the (E)-4-(dimethylamino)-N-(4-((4-(4-fluoro-phenoxy)phenyl)amino)-7-ethoxyquinazolin-6-yl)but-2-enamide maleate of the present disclosure exhibited significantly superior tumor inhibition compared to neratinib at the same dose. The results are shown in FIG. 29.

### Biology test Example 4: Pharmacokinetics of (E)-4-(dimethylamino)-N-(4-((4-(4-fluoro-phenoxy)phenyl)amino)-7-ethoxyquinazolin-6-yl)but-2-enamide in rats

Dosing regimen: Rats were intravenously (IV) and orally administered with the compound made in Example 1. Rats were fasted for at least 12 hours before the administration and had free access to water. Rats were fed 4 hours after the administration.

Intravenous administration: Collect 0.2 mL of blood from the retroorbital venous plexus before the administration (0 h) and at 5 min, 0.25, 0.5, 1.0, 2.0, 4.0, 6.0, 8.0, 10, and 24 h after the administration. Place the blood in an EDTA-K2 anticoagulant tube and centrifuge at 11,000 rpm for 5 min (4 °C). Separate the plasma within 30 min, and store the plasma in a refrigerator at -60°C or lower until testing.

Single oral administration: 0.2 mL of blood was collected from the retroorbital venous plexus before administration (0 h) and 0.25, 0.5, 1.0, 2.0, 4.0, 6.0, 8.0, 10, 24 and 48 h after administration, and placed in an EDTA-K2 anticoagulant tube. The sample was processed in the same way as above.

(E)-4-(dimethylamino)-N-(4-((4-(4-fluoro-phenoxy)phenyl)amino)-7-ethoxyquinazolin-6-yl)but-2-enamide showed a higher bioavailability of 79.5% in rats, which is a significant improvement over the bioavailability of 48% for (E)-4-(dimethylamino)-N-(4-((4-(4-fluoro-phenoxy)phenyl)amino)-7-methoxyquinazolin-6-yl)but-2-enamide (ML900199).

| | Example 1 Compound | ML900199 |
|---|---|---|
| Rat IV 1 mpk AUC0-t | 561 (h*ng/mL) | 240 (h*ng/mL) |
| Rat Oral 10 mpk AUC0-t | 4460 (h*ng/mL) | 1152 (h*ng/mL) |
| Cmax | 304 ng/mL | 73.5 ng/mL |
| F% | 79.50% | 48% |

| | | |
|---|---|---|
| Note: mpk is the drug mass in mg/animal weight in kg | | |

### Biological test Example 5: (E)-4-(dimethylamino)-N-(4-((4-(4-fluoro-phenoxy)phenyl)amino)-7-ethoxyquinazolin-6-yl)but-2-enamide blood-brain barrier assay

### 1. Medication and sample collection

Thirty SD rats were randomly divided into five groups, with six rats in each time point group (half were male, and half were female). Three mg/kg (E)-4-(dimethylamino)-N-(4-((4-(4-fluoro-phenoxy)phenyl)amino)-7-ethoxyquinazolin-6-yl)but-2-enamide maleate hydrate crystal form A was administered orally. The sampling time points were before the administration (0 h), and 1 h, 6 h, 10 h, and 24 h after the administration.

After CO₂ euthanasia, at least 0.5 mL of blood was collected by cardiac puncture, anticoagulated with EDTA-K₂, mixed, and placed on ice. Plasma was separated by centrifugation within 30 minutes (11,000 rpm, 5 minutes, 2-8 °C). Plasma samples were stored in an ultra-low temperature freezer until transferred to the testing entity.

After blood samples were collected from the animals, tissue samples were collected from the brain, heart, liver, spleen, lung, kidney, bladder, pancreas, testicles, ovaries, uterus, stomach wall, small intestine (jejunum), adrenal glands, skin, thymus, skeletal muscle, and fat (abdomen). Tissue samples were rinsed with saline to avoid cross-contamination, blotted dry with filter paper, and placed in labeled tubes (one tube per tissue). Samples were temporarily placed on ice before being stored in an ultra-low temperature freezer.

After thorough homogenization with a high-speed homogenizer to prepare tissue sample homogenates, the concentrations in plasma, whole blood, and tissue sample homogenates were determined by LC-MS/MS.

### 2. Quantification of tissue samples

LC-MS/MS was used to determine the levels ML900110 in whole blood, plasma and tissues of rats at different times after oral administration.

### 3. Results

The exposure ratio of the compound (E)-4-(dimethylamino)-N-(4-((4-(4-fluoro-phenoxy)phenyl)amino)-7-ethoxyquinazolin-6-yl)but-2-enamide of the present disclosure in the brain and plasma of male rats was 43.6%, and the exposure ratio of the compound (E)-4-(dimethylamino)-N-(4-((4-(4-fluoro-phenoxy)phenyl)amino)-7-ethoxyquinazolin-6-yl)but-2-enamide of the present disclosure in the brain and plasma of female rats was 79.7%, indicating that the compound of the present disclosure can pass through the blood-brain barrier.

### Biological test Example 6: Intracranial lesions of patients with brain metastases showed significant beneficial effects after treatment with (E)-4-(dimethylamino)-N-(4-((4-(4-fluoro-phenoxy)phenyl)amino)-7-ethoxyquinazolin-6-yl)but-2-enamide maleate hydrate crystal form A

| **Test Subject No.** | **Cancer Type** | **Dosage and Frequency** | **Intracranial Lesion Reduction%** |
|---|---|---|---|
| 03005 | breast cancer | 150 mg QD | -41.2% |
| 01021 | salivary gland cancer | 200 mg QD | -60% |

| | | | |
|---|---|---|---|
| Note: QD means once a day dosing | | | |

### Biological test Example 7: In human clinical trials, patients with HER2-positive tumors benefited from treatment with (E)-4-(dimethylamino)-N-(4-((4-(4-fluoro-phenoxy)phenyl)amino)-7-ethoxyquinazolin-6-yl)but-2-enamide maleate hydrate form A

### 1. Case selection criteria

### Inclusion criteria:

(1) 18 years-old ≤ age ≤ 65 years old, regardless of gender;
(2) patients with histologically or cytologically confirmed HER2-positive (overexpression, amplification, and/or mutation) advanced tumors;
(3) standard treatment has failed or standard treatment is not applicable at this stage;
(4) at least one evaluable tumor lesion according to RECIST version 1.1;
(5) Eastern Cooperative Oncology Group (ECOG) performance score is 0-1;
(6) expected survival time is more than 3 months;
(7) the level of organ function must meet the following requirements:

| **Hematologic System (no blood transfusions or treatment with hematopoietic stimulating factors within the past 14 days)** | |
|---|---|
| absolute neutrophil count (ANC) | ≥1.5×10⁹/L |
| platelet (PLT) | ≥90×10⁹/L |
| hemoglobin (Hb) | ≥90 g/L |

| **Liver Function** | |
|---|---|
| total bilirubin (TBIL) | ≤1.5×ULN |
| alanine aminotransferase (ALT) | ≤1.5×ULN |
| | patients with liver metastases ≤3×ULN |
| Aspartate aminotransferase (AST) | ≤1.5×ULN |
| | patients with liver metastases ≤3×ULN |

| **Kidney Function** | |
|---|---|
| creatinine clearance (Ccr) (calculation required only when creatinine > 1.5 × ULN) | ≥50 mL/min (calculated according to Cockcroft-Gault formula) |

| **Coagulation Function** | |
|---|---|
| activated partial thromboplastin time (APTT) | ≤1.5×ULN |
| international normalized ratio (INR) | ≤1.5×ULN |

| | |
|---|---|
| Note: ULN: upper limit of normal. | |

(8) Eligible patients (male and female) of reproductive potential must agree to use reliable contraceptive methods (hormonal or barrier methods or abstinence) with their partners during the trial and for at least 6 months after the last medication; female patients of childbearing age must have a negative blood pregnancy test within 7 days before enrollment;
(9) The subjects must give their informed consent to the study before the trial and voluntarily sign a written informed consent form.

### Exclusion criteria:

(1) Patients who have received chemotherapy within 3 weeks before the first dose of the experimental drugs; those who have received systemic anti-tumor treatment such as radiotherapy, targeted therapy, immunotherapy, endocrine therapy, etc. within 4 weeks before the first dose of the experimental drugs, excluding the following:
   - for nitrosourea or mitomycin C, received within 6 weeks before the first dose of the experimental drug;
   - for oral fluorouracils and small molecule targeted drugs, received within 2 weeks before the first dose of the experimental drug or within 5 half-lives of the drug (whichever is longer);
   - for traditional Chinese medicine with anti-tumor indications, received within 2 weeks before the first dose of the experimental drug;
   - for other unapproved clinical investigational drugs or treatments, received within 4 weeks before the first dose of the experimental drug;
(2) patients who have undergone major organ surgery (excluding puncture biopsy) or significant trauma within 4 weeks before the first dose of the experimental drug, or who need to undergo elective surgery during the trial;
(3) patients who have not recovered from the adverse reactions of previous anti-tumor treatment to CTCAE 5.0 grade ≤ 1 (excluding toxicities such as alopecia that the investigators determine to have no safety risks);
(4) patients who used strong CYP3A4 inhibitors or inducers within 7 days before the first dose of the experimental drug;
(5) patients who are unable to take the drug orally, or who have a condition that seriously affects gastrointestinal absorption (such as chronic diarrhea, intestinal obstruction, etc.) as determined by the investigator;
(6) patients who have history of immunodeficiency, including positive HIV antibody test;
(7) patients with clinical symptoms for unstable central nervous system metastases or meningeal metastases, or other evidence indicating that the patient's central nervous system metastases or meningeal metastases have not been controlled, and deemed unsuitable for enrollment by the investigator;
(8) patients having active hepatitis B (defined as HBsAg positive and HBV-DNA titer >1000 copies/mL or 200 IU/mL), with preventive antiviral treatment other than interferon allowed; hepatitis C virus infection (defined as anti-HCV antibody positive);
(9) patients with uncontrolled active infection requiring systemic anti-infective treatment or deemed unsuitable for enrollment by the investigator;
(10) patients having history of severe cardiovascular disease, including but not limited to:
   - severe cardiac rhythm or conduction abnormalities, such as ventricular arrhythmias requiring clinical intervention, II-III degree atrioventricular block, etc.;
   - acute coronary syndrome, congestive heart failure, or other grade 3 or higher cardiovascular events within 6 months before the first dose;
   - heart failure with New York Heart Association (NYHA) functional class ≥ II or left ventricular ejection fraction (LVEF) < 50%;
   - clinically uncontrolled hypertension;
   - QTc interval ≥450 ms for men and ≥470 ms for women, or the presence of any factors that increase the risk of QTc prolongation or arrhythmia, such as heart failure, hypokalemia, congenital long QT syndrome, or use of any concomitant medication known to prolong the QT interval (see Appendix 5 for details);
(11) patients whose third space effusion cannot be controlled clinically and who have been deemed unsuitable for enrollment by the investigator;
(12) patients having known alcohol or drug dependence;
(13) patients with mental disorders or poor compliance;
(14) pregnant or breastfeeding women;
(15) the investigator believes that the subject is not suitable for participation in this clinical study due to any clinical or laboratory abnormalities or other reasons.

### 3. Dosage: 50 mg-250 mg (E)-4-(dimethylamino)-N-(4-((4-(4-fluoro-phenoxy)phenyl)amino)-7-ethoxyquinazolin-6-yl)but-2-enamide maleate hydrate crystal form A once a day

### 4. Efficacy:

### Efficacy of (E)-4-(dimethylamino)-N-(4-((4-(4-fluoro-phenoxy)phenyl)amino)-7-ethoxyquinazolin-6-yl)but-2-enamide maleate hydrate crystal form A in subjects with HER2-positive breast cancer

| **No.** | **Subject No.** | **Dosage and Frequency** | **BOR (Percentage Reduction in target Lesions)** |
|---|---|---|---|
| 1 | 02014 | 150 mg QD | PR ( -65.2% ) |
| 2 | 03014 | 150 mg QD | PR ( -45.8% ) |
| 3 | 01013 | 200 mg QD | PR ( -46.5% ) |
| 4 | 01014 | 200 mg QD | PR ( -47.6% ) |
| 5 | 01023 | 200 mg QD | PR ( -53.3% ) |
| 6 | 02008 | 200 mg QD | PR ( -69.8% ) |
| 7 | 02010 | 200 mg QD | CR ( -100% ) |
| 8 | 02013 | 200 mg QD | PR ( -44.2% ) |
| 9 | 02004 | 200 mg QD | PR ( -32.7% ) |

| | | | |
|---|---|---|---|
| Note: BOR: Best Overall Response; QD: once a day; PR: Partial Response; CR: Complete Response. | | | |

### Efficacy of (E)-4-(dimethylamino)-N-(4-((4-(4-fluoro-phenoxy)phenyl)amino)-7-ethoxyquinazolin-6-yl)but-2-enamide maleate hydrate crystal form A in subjects with HER2-positive colorectal cancer

| **No.** | **Subject No.** | **Dosage and Frequency** | **BOR (Percentage Reduction in target Lesions)** |
|---|---|---|---|
| 1 | 01018 | 150 mg QD | PR ( -34.1% ) |
| 2 | 03012 | 200 mg QD | SD ( -21.8% ) |
| 3 | 01007 | 250 mg QD | SD ( -26.3% ) |

| | | | |
|---|---|---|---|
| Note: BOR: Best Overall Response; QD: once a day; PR: Partial Response; SD: Stable Disease. | | | |

### Efficacy of (E)-4-(dimethylamino)-N-(4-((4-(4-fluoro-phenoxy)phenyl)amino)-7-ethoxyquinazolin-6-yl)but-2-enamide maleate hydrate crystal form A in subjects with HER2-positive other tumor types

| **No.** | **Subject No.** | **Tumor** | **Dosage and Frequency** | **BOR (Percentage Reduction in target Lesions)** |
|---|---|---|---|---|
| 1 | 01003 | submandibular cancer | 150 mg QD | PR ( -59.3% ) |
| 2 | 01021 | salivary gland cancer | 200 mg QD | PR ( -34.5% ) |

In this application, unless otherwise specified, all weights related to biological activity are calculated based on the weight of the free base.

This application describes multiple embodiments. But the description is illustrative rather than restrictive, and there may be more embodiments and implementations within the scope of the embodiments described in this application.

## Claims

1. A trisubstituted quinazoline derivative, an acid addition salt thereof, or solvate or hydrate thereof, wherein the trisubstituted quinazoline derivative is (E)-4-(dimethylamino)-N-(4-((4-(4-fluoro-phenoxy)phenyl)amino)-7-ethoxyquinazolin-6-yl)but-2-enamide according to Formula (I):

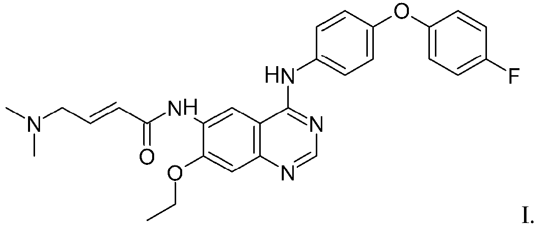

2. The trisubstituted quinazoline derivative, acid addition salt thereof, or solvate or hydrate thereof according to claim 1, wherein an acid in the acid addition salt is hydrochloric acid, sulfuric acid, nitric acid, phosphoric acid, acetic acid, propionic acid, glycolic acid, oxalic acid, maleic acid, fumaric acid, malonic acid, succinic acid, tartaric acid, citric acid, fumaric acid, mandelic acid, methanesulfonic acid, ethanesulfonic acid, benzenesulfonic acid, p-toluenesulfonic acid, trifluoroacetic acid, 1-hydroxy-2-naphthoic acid, or benzoic acid.

3. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide hydrochloride, or solvate or hydrate thereof.

4. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide sulfate, or solvate or hydrate thereof.

5. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide maleate, or solvate or hydrate thereof.

6. The trisubstituted quinazoline derivative, acid addition salt thereof, or solvate or hydrate thereof according to any one of claims 1-5, which is in a solid form comprising an amorphous form and/or a crystalline solid form.

7. The trisubstituted quinazoline derivative, acid addition salt thereof, or solvate or hydrate thereof according to claims 1, 2 or 5, which is a crystalline (E)-4-(dimethylamino)-N-(4-((4-(4-fluoro-phenoxy)phenyl)amino)-7-ethoxyquinazolin-6-yl)but-2-enamide maleate or solvate or hydrate thereof, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:1 or 1:2.

8. The trisubstituted quinazoline derivative, acid addition salt thereof, or solvate or hydrate thereof according to claim 5 or claim 7, which is (E)-4-(dimethylamino)-N-(4-((4-(4-fluoro-phenoxy)phenyl)amino)-7-ethoxyquinazolin-6-yl)but-2-enamide maleate or maleate hydrate, wherein the molar ratio of (E)-4-(dimethylamino)-N-(4-((4-(4-fluoro-phenoxy)phenyl)amino)-7-ethoxyquinazolin-6-yl)but-2-enamide to maleic acid is 1:2;
preferably, the (E)-4-(dimethylamino)-N-(4-((4-(4-fluoro-phenoxy)phenyl)amino)-7-ethoxyquinazolin-6-yl)but-2-enamide maleate or maleate hydrate is crystalline;
preferably, the (E)-4-(dimethylamino)-N-(4-((4-(4-fluoro-phenoxy)phenyl)amino)-7-ethoxyquinazolin-6-yl)but-2-enamide maleate hydrate is a monohydrate, optionally crystalline;
preferably, the (E)-4-(dimethylamino)-N-(4-((4-(4-fluoro-phenoxy)phenyl)amino)-7-ethoxyquinazolin-6-yl)but-2-enamide maleate is anhydrous, optionally crystalline.

9. The trisubstituted quinazoline derivative, acid addition salt thereof, or solvate or hydrate thereof according to claim 1, which is a crystal form A of (E)-4-(dimethylamino)-N-(4-((4-(4-fluoro-phenoxy)phenyl)amino)-7-ethoxyquinazolin-6-yl)but-2-enamide maleate hydrate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form A is 1:2; preferably, the crystal form A has a purity greater than 90%.

10. The trisubstituted quinazoline derivative, acid addition salt thereof, or solvate or hydrate thereof according to claim 9, wherein:
a PXRD pattern of the crystal form A comprises at least one of characteristic peaks at 2θ values of about 5.1°±0.2°, 5.7°±0.2°, 18.4°±0.2°, or 26.9°±0.2°; or
the PXRD pattern of the crystal form A comprises characteristic peaks at 2θ values of about 5.1°±0.2°, 5.7°±0.2°, 18.4°±0.2°, and 26.9°±0.2°.

11. The trisubstituted quinazoline derivative, acid addition salt thereof, or solvate or hydrate thereof according to claim 10, wherein:
the PXRD pattern of the crystal form A further comprises characteristic peaks at 2θ values of about 18.0°±0.2°, 20.4°±0.2°, 21.1°±0.2°, and 25.5°±0.2°; or
the PXRD pattern of the crystal form A comprises characteristic peaks at 2θ values of about 5.1°±0.2°, 5.7°±0.2°, 18.0°±0.2°, 18.4°±0.2°, 20.4°±0.2°, 21.1°±0.2°, 25.5°±0.2°, and 26.9°±0.2°2θ; or
the PXRD pattern of the crystal form A comprises the following characteristic peaks:
| 2θ | d-value | I/I₀ |
|---|---|---|
| 5.100 | 17.3131 | 64 |
| 5.680 | 15.5465 | 100 |
| 10.500 | 8.4182 | 22 |
| 11.240 | 7.8656 | 26 |
| 11.300 | 7.8240 | 28 |
| 16.700 | 5.3042 | 22 |
| 16.900 | 5.2419 | 29 |
| 18.020 | 4.9186 | 34 |
| 18.400 | 4.8178 | 47 |
| 18.480 | 4.7972 | 44 |
| 18.900 | 4.6915 | 22 |
| 19.000 | 4.6670 | 23 |
| 19.780 | 4.4847 | 22 |
| 20.420 | 4.3456 | 31 |
| 20.880 | 4.2509 | 29 |
| 21.080 | 4.2110 | 31 |
| 22.000 | 4.0369 | 23 |
| 25.180 | 3.5338 | 25 |
| 25.360 | 3.5092 | 33 |
| 25.480 | 3.4929 | 36 |
| 25.940 | 3.4320 | 23 |
| 26.040 | 3.4190 | 24 |
| 26.720 | 3.3336 | 48 |
| 26.780 | 3.3262 | 56 |
| 26.880 | 3.3141 | 61 |
| 27.260 | 3.2687 | 29 |
| 27.380 | 3.2547 | 28 |
| 27.540 | 3.2361 | 27 |

12. The trisubstituted quinazoline derivative, acid addition salt thereof, or solvate or hydrate thereof according to any one of claims 9-11, wherein a water content of the crystal form A is from 2.1 wt% to 10.0 wt%; or, the crystal form A is a monohydrate.

13. The trisubstituted quinazoline derivative, acid addition salt thereof, or solvate or hydrate thereof according to claim 1, which is a crystal form B of (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form B is 1:2; preferably, the crystal form B has a purity greater than 90%.

14. The trisubstituted quinazoline derivative, acid addition salt thereof, or solvate or hydrate thereof according to claim 13, wherein:
a PXRD pattern of the crystal form B comprises at least one of characteristic peaks at 2θ values of about 5.1°±0.2°, 9.8°±0.2°, 10.1°±0.2°, 19.4°±0.2°, or 20.3°±0.2°; or
the PXRD pattern of the crystal form B comprises characteristic peaks at 2θ values of about 5.1°±0.2°, 9.8°±0.2°, 10.1°±0.2°, 19.4°±0.2°, and 20.3°±0.2°.

15. The trisubstituted quinazoline derivative, acid addition salt thereof, or solvate or hydrate thereof according to claim 14, wherein:
the PXRD pattern of the crystal form B further comprises characteristic peaks at 2θ values of about 7.0°±0.2°, 18.4°±0.2°, 19.6°±0.2°, 22.3°±0.2°, and 25.1°±0.2°; or
the PXRD pattern of the crystal form B comprises characteristic peaks at 2θ values of about 5.1°±0.2°, 7.0°±0.2°, 9.8°±0.2°, 10.1°±0.2°, 18.4°±0.2°, 19.4°±0.2°, 19.6°±0.2°, 20.3°±0.2°, 22.3°±0.2°, and 25.1°±0.2°; or
the PXRD pattern of the crystal form B comprises the following characteristic peaks:
| 2θ | d-value | I/I₀ |
|---|---|---|
| 5.080 | 17.3812 | 100 |
| 7.040 | 12.5459 | 14 |
| 9.800 | 9.0179 | 28 |
| 10.120 | 8.7335 | 46 |
| 14.080 | 6.2848 | 11 |
| 16.520 | 5.3616 | 10 |
| 17.700 | 5.0068 | 12 |
| 17.800 | 4.9789 | 11 |
| 18.080 | 4.9024 | 11 |
| 18.400 | 4.8178 | 15 |
| 19.380 | 4.5764 | 27 |
| 19.620 | 4.5209 | 20 |
| 20.280 | 4.3753 | 35 |
| 20.720 | 4.2833 | 11 |
| 22.120 | 4.0153 | 14 |
| 22.280 | 3.9868 | 16 |
| 22.480 | 3.9518 | 15 |
| 22.560 | 3.9380 | 16 |
| 23.100 | 3.8471 | 15 |
| 23.420 | 3.7953 | 15 |
| 25.120 | 3.5421 | 17 |
| 25.740 | 3.4582 | 12 |
| 26.640 | 3.3434 | 12 |
| 29.600 | 3.0154 | 13 |
| 29.680 | 3.0075 | 12 |

16. The trisubstituted quinazoline derivative, acid addition salt thereof, or solvate or hydrate thereof according to any one of claims 13-15, wherein the crystal form B is an anhydrate.

17. The trisubstituted quinazoline derivative, acid addition salt thereof, or solvate or hydrate thereof according to claim 1, which is a crystal form C of (E)-4-(dimethylamino)-N-(4-((4-(4-fluoro-phenoxy)phenyl)amino)-7-ethoxyquinazolin-6-yl)but-2-enamide maleate, wherein the molar ratio of (E)-4-(dimethylamino)-N-(4-((4-(4-fluoro-phenoxy)phenyl)amino)-7-ethoxyquinazolin-6-yl)but-2-enamide to maleic acid in the crystal form C is 1:1; preferably, the crystal form C has a purity greater than 90%.

18. The trisubstituted quinazoline derivative, acid addition salt thereof, or solvate or hydrate thereof according to claim 17, wherein:
a PXRD pattern of the crystal form C comprises at least one of characteristic peaks at 2θ values of about 5.4°±0.2°, 5.8°±0.2°, 18.2°±0.2°, 18.7°±0.2°, 19.0°±0.2°, or 26.8°±0.2°; or
the PXRD pattern of the crystal form C comprises characteristic peaks at 2θ values of about 5.4°±0.2°, 5.8°±0.2°, 18.2°±0.2°, 18.7°±0.2°, 19.0°±0.2°, and 26.8°±0.2°.

19. The trisubstituted quinazoline derivative, acid addition salt thereof, or solvate or hydrate thereof according to claim 18, wherein:
the PXRD pattern of the crystal form C further comprises characteristic peaks at 2θ values of about 6.1°±0.2°, 22.3°±0.2°, and 25.6°±0.2°; or
the PXRD pattern of the crystal form C comprises characteristic peaks at 2θ values of about 5.4°±0.2°, 5.8°±0.2°, 6.1°±0.2°, 18.2°±0.2°, 18.7°±0.2°, 19.0°±0.2°, 22.3°±0.2°, 25.6°±0.2°, and 26.8°±0.2°; or
the PXRD pattern of the crystal form C further comprises characteristic peaks at 2θ values of about 5.4°±0.2°, 5.8°±0.2°, 6.1°±0.2°, 18.2°±0.2°, 18.7°±0.2°, 19.0°±0.2°, 19.8°±0.2°, 21.5°±0.2°, 22.3°±0.2°, 22.8°±0.2°, 23.7°±0.2°, 25.6°±0.2°, and 26.8°±0.2°; or
the PXRD pattern of the crystal form C comprises the following characteristic peaks:
| 2θ | d-value | I/I₀ |
|---|---|---|
| 5.380 | 16.4127 | 81 |
| 5.760 | 15.3307 | 100 |
| 6.100 | 14.4770 | 74 |
| 18.200 | 4.8703 | 84 |
| 18.700 | 4.7412 | 100 |
| 19.020 | 4.6622 | 80 |
| 19.800 | 4.4802 | 63 |
| 21.460 | 4.1373 | 64 |
| 22.260 | 3.9904 | 70 |
| 22.840 | 3.8903 | 62 |
| 23.740 | 3.7448 | 64 |
| 25.620 | 3.4741 | 77 |
| 26.820 | 3.3214 | 81 |

20. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide hydrochloride, wherein the hydrochloride is crystalline; preferably, a crystal form of the hydrochloride has a purity greater than 90%.

21. The trisubstituted quinazoline derivative, acid addition salt thereof, or solvate or hydrate thereof according to claim 20, wherein:
the crystal form of the hydrochloride is a crystal form S-1 or a crystal form S-2, a PXRD pattern of the crystal form S-1 comprises at least one of characteristic peaks at 2θ values of about 6.1°±0.2°, 15.5°±0.2°, 18.1°±0.2°, 19.7°±0.2°, or 24.1°±0.2°; or
the PXRD pattern of the crystal form S-1 comprises characteristic peaks at 2θ values of about 6.1°±0.2°, 15.5°±0.2°, 18.1°±0.2°, 19.7°±0.2°, and 24.1°±0.2°.

22. The trisubstituted quinazoline derivative, acid addition salt thereof, or solvate or hydrate thereof according to claim 21, wherein:
the PXRD pattern of the crystal form S-1 further comprises characteristic peaks at 2θ values of about 14.9°±0.2°, 22.6°±0.2°, 23.3°±0.2°, 25.6°±0.2°, and 26.0°±0.2°; or
the PXRD pattern of the crystal form S-1 comprises characteristic peaks at 2θ values of about 6.1°±0.2°, 14.9°±0.2°, 15.5°±0.2°, 18.1°±0.2°, 19.7°±0.2°, 22.6°±0.2°, 23.3°±0.2°, 24.1°±0.2°, 25.6°±0.2°, and 26.0°±0.2°; or
the PXRD pattern of the crystal form S-1 comprises the following characteristic peaks:
| 2θ | d-value | I/I₀ |
|---|---|---|
| 6.120 | 14.4297 | 100 |
| 14.920 | 5.9328 | 52 |
| 15.500 | 5.7121 | 60 |
| 18.140 | 4.8863 | 78 |
| 19.620 | 4.5209 | 69 |
| 19.740 | 4.4937 | 78 |
| 20.360 | 4.3582 | 48 |
| 20.480 | 4.3330 | 48 |
| 22.580 | 3.9345 | 59 |
| 23.300 | 3.8145 | 56 |
| 24.120 | 3.6867 | 67 |
| 24.980 | 3.5617 | 48 |
| 25.560 | 3.4822 | 54 |
| 26.020 | 3.4216 | 51 |
| 28.560 | 3.1228 | 46 |
| 28.700 | 3.1079 | 47 |

23. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide sulfate, wherein the sulfate is crystalline; preferably, a crystal form of the sulfate has a purity greater than 90%.

24. The trisubstituted quinazoline derivative, acid addition salt thereof, or solvate or hydrate thereof according to claim 23, wherein:
the crystal form of the sulfate is crystal form S-3, crystal form S-4, or crystal form S-5, a PXRD pattern of the crystal form S-3 comprises at least one of characteristic peaks at 2θ values of about 5.2°±0.2°, 16.0°±0.2°, 25.4°±0.2°, or 26.1°±0.2°; or
the PXRD pattern of the crystal form S3 comprises characteristic peaks at 2θ values of about 5.2°±0.2°, 16.0°±0.2°, 25.4°±0.2°, and 26.1°±0.2°.

25. The trisubstituted quinazoline derivative, acid addition salt thereof, or solvate or hydrate thereof according to claim 24, wherein:
the PXRD pattern of the crystal form S-3 further comprises characteristic peaks at 2θ values of about 17.7°±0.2°, 22.5°±0.2°, 24.5°±0.2°, 25.1°±0.2°, and 26.6°±0.2°; or
the PXRD pattern of the crystal form S-3 comprises characteristic peaks at 2θ values of about 5.2°±0.2°, 16.0°±0.2°, 17.7°±0.2°, 22.5°±0.2°, 24.5°±0.2°, 25.1°±0.2°, 25.4°±0.2°, 26.1°±0.2°, and 26.6°±0.2°; or
the PXRD pattern of the crystal form S-3 comprises the following characteristic peaks:
| 2θ | d-value | I/I₀ |
|---|---|---|
| 5.200 | 16.9804 | 100 |
| 16.040 | 5.5210 | 29 |
| 17.700 | 5.0068 | 24 |
| 22.540 | 3.9414 | 24 |
| 24.540 | 3.6245 | 23 |
| 25.120 | 3.5421 | 25 |
| 25.400 | 3.5037 | 30 |
| 26.080 | 3.4139 | 30 |
| 26.160 | 3.4036 | 29 |
| 26.600 | 3.3483 | 22 |

26. The trisubstituted quinazoline derivative, acid addition salt thereof, or solvate or hydrate thereof according to claim 1 or claim 2, which is (E)-4-(dimethylamino)-N-(4-((4-(4-fluoro-phenoxy)phenyl)amino)-7-ethoxyquinazolin-6-yl)but-2-enamide tartrate, wherein the tartrate is crystalline; preferably, a crystal form of the tartrate has a purity greater than 90%.

27. The trisubstituted quinazoline derivative, acid addition salt thereof, or solvate or hydrate thereof according to claim 26, wherein:
the crystal form of the tartrate is a crystal form S-6, and a PXRD pattern of the crystal form S-6 comprises at least one of characteristic peaks at 2θ values of about 20.2°±0.2°, 20.6°±0.2°, 23.5°±0.2°, or 27.4°±0.2°; or
the PXRD pattern of the crystal form S-6 comprises characteristic peaks at 2θ values of about 20.2°±0.2°, 20.6°±0.2°, 23.5°±0.2°, and 27.4°±0.2°.

28. The trisubstituted quinazoline derivative, acid addition salt thereof, or solvate or hydrate thereof according to claim 27, wherein:
the PXRD pattern of the crystal form S-6 further comprises characteristic peaks at 2θ values of about 18.7°±0.2°, 24.1°±0.2°, 24.9°±0.2°, and 36.5°±0.2°; or
the PXRD pattern of the crystal form S-6 comprises characteristic peaks at 2θ values of about 18.7°±0.2°, 20.2°±0.2°, 20.6°±0.2°, 23.5°±0.2°, 24.1°±0.2°, 24.9°±0.2°, 27.4°±0.2°, and 36.5°±0.2°; or
the PXRD pattern of the crystal form S-6 comprises the following characteristic peaks:
| 20 | d-value | I/I₀ |
|---|---|---|
| 4.038(5) | 21.86(3) | 0.32 |
| 4.84(4) | 18.23(16) | 0.66 |
| 6.186(5) | 14.277(12) | 0.50 |
| 6.997(9) | 12.624(15) | 0.16 |
| 10.237(18) | 8.634(15) | 0.27 |
| 11.776(17) | 7.509(11) | 0.08 |
| 12.43(2) | 7.117(12) | 0.22 |
| 14.202(11) | 6.231(5) | 0.25 |
| 16.78(5) | 5.280(14) | 0.52 |
| 17.805(19) | 4.978(5) | 0.42 |
| 18.710(18) | 4.739(4) | 0.98 |
| 19.390(15) | 4.574(4) | 0.29 |
| 20.208(18) | 4.391(4) | 1.13 |
| 20.59(3) | 4.311(7) | 100 |
| 21.77(7) | 4.079(14) | 0.35 |
| 22.59(3) | 3.933(5) | 0.28 |
| 23.54(3) | 3.776(5) | 1.38 |
| 24.112(10) | 3.6879(16) | 1.09 |
| 24.869(16) | 3.577(2) | 0.86 |
| 27.359(12) | 3.2572(14) | 2.19 |
| 36.51(10) | 2.459(7) | 0.85 |

29. The trisubstituted quinazoline derivative, acid addition salt thereof, or solvate or hydrate thereof according to claim 1 or claim 2, which is (E)-4-(dimethylamino)-N-(4-((4-(4-fluoro-phenoxy)phenyl)amino)-7-ethoxyquinazolin-6-yl)but-2-enamide fumarate, wherein the fumarate is crystalline; preferably, a crystal form of the fumarate has a purity greater than 90%.

30. The trisubstituted quinazoline derivative, acid addition salt thereof, or solvate or hydrate thereof according to claim 29, wherein:
the crystal form of the fumarate is crystal form S-7, and a PXRD pattern of the crystal form S-7 comprises at least one of characteristic peaks at 2θ values of about 6.9°±0.2°, 17.9°±0.2°, 22.7°±0.2°, 24.2°±0.2°, 25.4°±0.2°, and 25.7°±0.2°; or
the PXRD pattern of the crystal form S-7 comprises characteristic peaks at 2θ values of about 6.9°±0.2°, 17.9°±0.2°, 22.7°±0.2°, 24.2°±0.2°, 25.4°±0.2°, and 25.7°±0.2°.

31. The trisubstituted quinazoline derivative, acid addition salt thereof, or solvate or hydrate thereof according to claim 30, wherein:
the PXRD pattern of the crystal form S-7 further comprises characteristic peaks at 2θ values of about 8.8°±0.2°, 24.9°±0.2°, and 28.1°±0.2°; or
the PXRD pattern of the crystal form S-7 comprises characteristic peaks at 2θ values of about 6.9°±0.2°, 8.8°±0.2°, 17.9°±0.2°, 22.7°±0.2°, 24.2°±0.2°, 24.9°±0.2°, 25.4°±0.2°, 25.7°±0.2°, and 28.1°±0.2°; or
the PXRD pattern of the crystal form S-7 comprises the following characteristic peaks:
| 2θ | d-value | I/I₀ |
|---|---|---|
| 3.701(3) | 23.853(19) | 1.42 |
| 4.361(2) | 20.244(10) | 3.61 |
| 6.8914(15) | 12.816(3) | 9.02 |
| 7.504(3) | 11.771(4) | 1.54 |
| 7.931(2) | 11.138(3) | 1.08 |
| 8.820(2) | 10.017(2) | 5.37 |
| 10.203(18) | 8.662(16) | 0.13 |
| 10.902(11) | 8.109(8) | 1.29 |
| 11.804(8) | 7.491(5) | 2.79 |
| 12.3(2) | 7.17(13) | 0.80 |
| 13.52(4) | 6.54(2) | 1.01 |
| 13.812(7) | 6.406(3) | 1.66 |
| 15.280(9) | 5.794(3) | 0.99 |
| 16.086(10) | 5.505(3) | 1.13 |
| 16.421(11) | 5.394(4) | 2.48 |
| 16.971(16) | 5.220(5) | 1.12 |
| 17.870(6) | 4.9597(16) | 8.08 |
| 18.859(17) | 4.702(4) | 1.66 |
| 19.548(16) | 4.537(4) | 1.35 |
| 20.282(14) | 4.375(3) | 1.04 |
| 21.740(9) | 4.0846(16) | 4.57 |
| 22.708(9) | 3.9128(16) | 8.95 |
| 24.155(5) | 3.6815(8) | 12.14 |
| 24.885(4) | 3.5752(6) | 7.73 |
| 25.43(5) | 3.499(6) | 100.00 |
| 25.650(7) | 3.4702(9) | 19.37 |
| 28.078(12) | 3.1754(13) | 6.43 |
| 29.15(3) | 3.061(3) | 2.33 |
| 31.79(5) | 2.812(4) | 0.29 |
| 32.87(4) | 2.723(3) | 0.70 |
| 34.1(9) | 2.62(6) | 0.87 |
| 34.77(4) | 2.578(3) | 0.53 |
| 38.33(9) | 2.346(5) | 0.54 |

32. The trisubstituted quinazoline derivative, acid addition salt thereof, or solvate or hydrate thereof according to claim 1 or claim 2, which is (E)-4-(dimethylamino)-N-(4-((4-(4-fluoro-phenoxy)phenyl)amino)-7-ethoxyquinazolin-6-yl)but-2-enamide p-toluenesulfonate, wherein the p-toluenesulfonate is crystalline; preferably, a crystal form of the p-toluenesulfonate has a purity greater than 90%.

33. The trisubstituted quinazoline derivative, acid addition salt thereof, or solvate or hydrate thereof according to claim 32, wherein:
the crystal form of the p-toluenesulfonate is crystal form S-8, and the PXRD pattern of the crystal form S-8 comprises at least one of characteristic peaks at 2θ values of about 5.7°±0.2°, 18.6°±0.2°, 20.2°±0.2°, 23.4°±0.2°, and 25.5°±0.2°; or
the PXRD pattern of the crystal form S-8 comprises characteristic peaks at 2θ values of about 5.7°±0.2°, 18.6°±0.2°, 20.2°±0.2°, 23.4°±0.2°, and 25.5°±0.2°.

34. The trisubstituted quinazoline derivative, acid addition salt thereof, or solvate or hydrate thereof according to claim 33, wherein:
the PXRD pattern of the crystal form S-8 further comprises characteristic peaks at 2θ values of about 8.9°±0.2°, 10.1°±0.2°, 24.4°±0.2°, 25.0°±0.2°, and 31.7°±0.2°; or
the PXRD pattern of the crystal form S-8 comprises characteristic peaks at 2θ values of about 5.7°±0.2°, 8.9°±0.2°, 10.1°±0.2°, 18.6°±0.2°, 20.2°±0.2°, 23.4°±0.2°, 24.4°±0.2°, 25.0°±0.2°, 25.5°±0.2°, and 31.7°±0.2°; or
the PXRD pattern of the crystal form S-8 comprises the following characteristic peaks:
| 2θ | d-value | I/I₀ |
|---|---|---|
| 5.7102(15) | 15.465(4) | 31.61 |
| 8.948(5) | 9.875(5) | 13.46 |
| 10.127(7) | 8.728(6) | 18.62 |
| 11.534(11) | 7.666(7) | 2.57 |
| 12.411(13) | 7.126(8) | 2.02 |
| 13.254(6) | 6.675(3) | 6.45 |
| 13.702(6) | 6.457(3) | 7.56 |
| 14.642(9) | 6.045(4) | 5.09 |
| 15.521(14) | 5.705(5) | 6.43 |
| 16.237(12) | 5.455(4) | 7.04 |
| 16.96(3) | 5.223(9) | 0.83 |
| 18.588(4) | 4.7695(10) | 36.66 |
| 19.590(9) | 4.528(2) | 5.82 |
| 20.231(4) | 4.3859(8) | 30.93 |
| 21.465(5) | 4.1363(10) | 5.68 |
| 21.949(11) | 4.046(2) | 3.72 |
| 23.373(8) | 3.8029(12) | 45.79 |
| 24.369(4) | 3.6496(6) | 14.02 |
| 25.014(5) | 3.5569(7) | 20.99 |
| 25.47(6) | 3.494(8) | 100.00 |
| 26.532(9) | 3.3569(11) | 5.01 |
| 27.905(11) | 3.1947(12) | 8.76 |
| 29.18(2) | 3.058(2) | 1.99 |
| 31.65(5) | 2.825(4) | 11.84 |
| 32.308(14) | 2.7687(12) | 4.09 |
| 34.45(2) | 2.6012(17) | 7.63 |
| 36.92(4) | 2.432(3) | 0.98 . |

35. The trisubstituted quinazoline derivative, acid addition salt thereof, or solvate or hydrate thereof according to claim 1, which is (E)-4-(dimethylamino)-N-(4-((4-(4-fluoro-phenoxy)phenyl)amino)-7-ethoxyquinazolin-6-yl)but-2-enamide (free base), wherein the free base is a crystalline solid or an amorphous solid; optionally, in the form of a hydrate; optionally, a crystal form has a purity greater than 90%; optionally, the (E)-4-(dimethylamino)-N-(4-((4-(4-fluoro-phenoxy)phenyl)amino)-7-ethoxyquinazolin-6-yl)but-2-enamide (free base) is in the form of any one of crystal forms F1 to F6.

36. A method for preparing the trisubstituted quinazoline derivative, acid addition salt thereof, or solvate or hydrate thereof according to any one of claims 1-35, comprising: mixing and reacting (E)-4-(dimethylamino)-N-(4-((4-(4-fluoro-phenoxy)phenyl)amino)-7-ethoxyquinazolin-6-yl)but-2-enamide (free base) with an acid in an organic solvent or a mixture of an organic solvent and water, and then separating the solids from the liquid, thereby obtaining an acid addition salt of (E)-4-(dimethylamino)-N-(4-((4-(4-fluoro-phenoxy)phenyl)amino)-7-ethoxyquinazolin-6-yl)but-2-enamide, or a solvate or hydrate thereof, comprising a crystal form and/or amorphous form thereof.

37. The method for preparing the trisubstituted quinazoline derivative, acid addition salt thereof, or solvate or hydrate thereof according to claim 36, further comprising the following steps:
(1) reacting 7-fluoro-N⁴-(4-(4-fluorophenoxy)phenyl)-6-nitroquinazolin-4-amine with sodium ethoxide, thereby obtaining 7-ethoxy-N⁴-(4-(4-fluorophenoxy)phenyl)-6-nitroquinazolin-4-amine;
(2) subjecting 7-ethoxy-N⁴-(4-(4-fluorophenoxy)phenyl)-6-nitroquinazolin-4-amine to a reduction reaction, thereby obtaining 7-ethoxy-N⁴-(4-(4-fluorophenoxy)phenyl)quinazoline-4,6-diamine;
(3) reacting 7-ethoxy-N⁴-(4-(4-fluorophenoxy)phenyl)quinazoline-4,6-diamine with diethylphosphonoacetic acid, thereby obtaining diethyl (2-((7-ethoxy-4-((4-(4-fluorophenoxy)phenyl)amino)quinazolin-6-yl)amino)-2-oxoethyl)phosphonate; and
(4) reacting diethyl (2-((7-ethoxy-4-((4-(4-fluorophenoxy)phenyl)amino)quinazolin-6-yl)amino)-2-oxoethyl)phosphonate with 2-dimethylaminoacetaldehyde, thereby obtaining (E)-4-(dimethylamino)-N-(4-((4-(4-fluoro-phenoxy)phenyl)amino)-7-ethoxyquinazolin-6-yl)but-2-enamide.

38. A pharmaceutical composition comprising the trisubstituted quinazoline derivative, acid addition salt thereof, or solvate or hydrate thereof according to any one of claims 1-35; optionally, wherein the pharmaceutical composition is in the form of a unit dose, and wherein the unit dose is from 25 mg to 700 mg, or from 35 mg to 500 mg, or from 40 mg to 400 mg, or from 50 mg to 350 mg, or from 50 mg to 250 mg.

39. Use of the trisubstituted quinazoline derivative, acid addition salt thereof, or solvate or hydrate thereof according to any one of claims 1-35, or the pharmaceutical composition according to claim 38 in the preparation of a medicament for preventing, inhibiting or treating a cancer; particularly; in the preparation of a medicament for preventing, inhibiting or treating a brain metastasis.

40. The trisubstituted quinazoline derivative, acid addition salt thereof, or solvate or hydrate thereof according to any one of claims 1-35, or the pharmaceutical composition according to claim 38, for use in preventing, inhibiting or treating a cancer; in particular, for use in preventing, inhibiting or treating a brain metastasis.

41. A method for preventing, inhibiting or treating a cancer, comprising administering to a subject in need thereof a therapeutically effective amount of a trisubstituted quinazoline derivative, an acid addition salt thereof, or solvate or hydrate thereof according to any one of claims 1-35, or the pharmaceutical composition according to claim 38.

42. The use according to claim 39, or the trisubstituted quinazoline derivative, acid addition salt thereof or their solvate or hydrate or pharmaceutical composition for use according to claim 40, or the method according to claim 41, wherein:
the cancer is HER2-positive cancer;
the HER2-positive cancer comprises HER2 protein overexpression, HER2 gene amplification, or HER2 gene mutation, or a combination thereof;
particularly, the HER2-positive cancer is one or more selected from the group of breast cancer, ovarian cancer, epithelial cancer, colorectal cancer, renal cancer, bladder cancer, laryngeal cancer, esophageal cancer, salivary gland cancer, submandibular cancer, gastric cancer and lung cancer, especially breast cancer, ovarian cancer, gastric cancer and/or colorectal cancer; more particularly, cancer with brain metastasis comprising breast cancer with brain metastasis, ovarian cancer with brain metastasis, gastric cancer with brain metastasis, and/or colorectal cancer with brain metastasis.

43. The use according to claim 39 or claim 42, or the trisubstituted quinazoline derivative, acid addition salt thereof or solvate or hydrate thereof or the pharmaceutical composition for use according to claim 40 or 42, or the method according to claim 41 or 42, wherein the trisubstituted quinazoline derivative, acid addition salt thereof or solvate or hydrate thereof or the pharmaceutical composition is administered in a unit dose of 25 mg to 700 mg, or 35 mg to 500 mg, or 40 mg to 400 mg, or 50 mg to 350 mg, or 50 mg to 250 mg; the number of administrations is 1 to 4 times a day.

44. The use according to claim 39 or 42, or the trisubstituted quinazoline derivative, acid addition salt thereof, or solvate or hydrate thereof or the pharmaceutical composition for use according to claim 40 or claim 42, or the method according to claim 41 or claim 42, wherein the trisubstituted quinazoline derivative, acid addition salt thereof or solvate or hydrate thereof is administered in a therapeutically effective amount, wherein the therapeutically effective amount is 0.01-500 mg/kg, preferably 0.01-50 mg/kg; more preferably 0.1-10 mg/kg; and wherein the number of administrations is 1 to 4 times a day.
